# EUROPEAN PATENT APPLICATION

(11) **EP 4 506 357 A1**
(43) Date of publication of application: **12.02.2025**
(21) Application number: 23784285.1
(22) Date of filing: 04.04.2023
(51) Int. Cl.: C07K 7/56, C12N 15/52, C12N 1/15, C12P 21/04, A61K 38/12, A61K 45/06, A61P 31/10, C12R 1/66

(54) **ECHINOCANDIN COMPOUND, AND PREPARATION METHOD THEREFOR AND USE THEREOF**

(30) Priority: 07.04.2022 CN 202210364079
(71) Applicant: Zhejiang University, Hangzhou, Zhejiang 310058 (CN)
(72) Inventor: XU, Jinzhong, Hangzhou, Zhejiang 310058 (CN); YU, Xionghui, Hangzhou, Zhejiang 310058 (CN); WANG, Pinmei, Hangzhou, Zhejiang 310058 (CN)
(74) Representative: Hoffmann Eitle
(86) International application number: PCT/CN2023/086269
(87) International publication number: WO 2023/193722

(57) **Abstract**

The present invention relates to an echinocandin compound, and a preparation method therefor and the use thereof. Specifically, disclosed in the present invention are an echinocandin B deoxidation analogue and a biosynthesis method therefor. The echinocandin B deoxidation analogue has a good antifungal activity.

## Description

### Technical Field

The present invention belongs to the field of synthetic biology and pharmaceutical science, and specifically relates to a class of echinocandin B deoxidation analogues and a preparation method therefor and a use thereof.

### Background Art

Echinocandins compounds are a class of natural cyclic lipopeptide compounds discovered in the 1970s. Their chemical structure contains a cyclic hexapeptide scaffold and fatty acid side chain structural fragments (connected to the hexapeptide scaffold via amide bonds). Echinocandins have antifungal activity, and their mechanism of action is to prevent the synthesis of fungal cell walls through non-competitive inhibition *of β*-(1,3)-D-glucan synthase. The typical representative is echinocandin B. To date, new antifungal drugs, such as anidulafungin and rezafungin, etc, have been developed through semi-synthesis based on echinocandin B.

Studies have shown that deoxidation analogues of echinocandin B have similar antifungal activity with echinocandin B. Currently, the preparation methods of echinocandin B deoxidation analogues are divided into chemical synthesis methods and biosynthesis methods. Chinese patent CN1298410A discloses the conversion of mulondulin into its C4-homotyrosine monodeoxy analogue using Raney nickel hydrogenolysis under neutral conditions; US patent application 222157 uses trifluoroacetic acid, NaCNBH₃ or NaBH(OAc)₃ to reduce pure echinocandin B into a monodeoxy analog. The above methods both belong to chemical methods, which require the use of pure products for conversion, have low conversion efficiency and high cost, and the reagents used are harmful to the human body. Chinese patent application CN101993477A discloses a method for preparing deoxidation analogues of echinocandin B, which includes mixing a fermentation broth of echinocandin B with an acidic solution to prepare the deoxidation analogues. This method obtains a variety of echinocandin B deoxidation analogue components, but has low conversion efficiency and high purification cost. There have also been reports on the preparation of echinocandin B deoxidation analogues by fermentation culture of customized mutants with oxygenase genes (such as *ecdG* or *ecd*H) knocked out. However, the echinocandin B deoxidation analogues obtained by this method have very complex components, many byproducts, low conversion efficiency, and high purification costs, which are not conducive to industrialization.

Therefore, there is an urgent need to find a method for preparing a certain echinocandin B deoxidation analogue with high conversion efficiency and specificity, and to discover and prepare echinocandin B deoxidation analogues with good antifungal activity through this method.

### Summary of the Invention

In order to solve the above problems, the present inventors have found a biosynthetic method after extensive research, which can specifically synthesize desired echinocandin B deoxidation analogues. The Method has high conversion efficiency, with relatively simple reaction product compositions and few impurities, which is conducive to industrialization. The present inventors also found that novel echinocandin B deoxidation analogues prepared by the method have good antifungal activity. Moreover, by further chemical structure modification of these novel echinocandin B deoxidation analogues, the obtained compound derivatives can also exhibit good antifungal activity.

Therefore, in one aspect, the present invention provides an echinocandin compound represented by general formula (I), 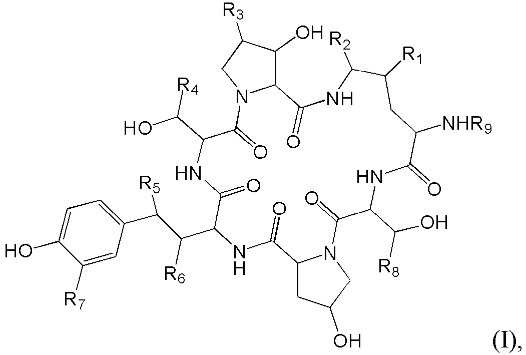 or an ester, a stereoisomer, a prodrug, a solvate and a pharmaceutically acceptable salt thereof,
wherein,
R₁, R₅, and R₆ are identical with or different from each other, and are each independently selected from H or OH;
R₂ is selected from the group consisting of H, OH, -X₁-(alkylene-X₂)ₘ -alkylene-X₃, -X₁-(alkylene-X₂)n -CH[CH₂(X₄-alkylene)ₚ-X₅ ]₂, wherein X₁, X₂, X₄ are selected from -O-, -NH-, and -S-; X₃, X₅ are selected from ORₐ, NRₐR_{b}, NRₐR_{b}R_{c}, wherein Rₐ, R_{b}, R_{c} are each independently selected from H, alkyl, arylalkyl, heteroarylalkyl, heterocyclylalkyl, and cycloalkylalkyl, said aryl, heteroaryl, heterocyclyl, and cycloalkyl are optionally substituted by a group selected from: hydroxyl, alkoxyl, amino, alkyl, alkenyl, alkynyl, halogen, cyano, nitro, thiol, -S-alkyl, -S(O)-alkyl, -SO₂-alkyl, NR_{c}R_{d}, COORₑ, and CONR_{c}R_{d}, wherein R_{c}, R_{d}, and Rₑ are each independently selected from H and alkyl; or any two of Rₐ, R_{b}, and R_{c} are connected to form a heterocycle; or any one of Rₐ, R_{b}, and R_{c} is connected to a carbon atom of the alkylene group to which NRₐR_{b}R_{c} is connected to form a heterocycle; m, n, and p are integers of 0-5;
R₃ is selected from H, alkyl;
R₄ and R₈ are identical with or different from each other, and are each independently selected from H, alkyl, alkenyl, alkynyl, aryl, heteroaryl, cycloalkyl, and heterocyclyl, said alkyl, alkenyl, alkynyl, aryl, heteroaryl, cycloalkyl, and heterocyclyl are optionally substituted by halogen, cyano, nitro, thiol, -S-alkyl, -S(O)-alkyl, -SO₂-alkyl, NR_{c}R_{d}, COORₑ, and CONR_{c}'R_{d}', wherein R_{c}, R_{d}, Rₑ, R_{c}', and R_{d}' are each independently selected from H and alkyl;
R₇ is selected from H, OH, halogen, -OSO₃H, or -alkylene-N_{f}R_{g}, wherein R_{f} and R_{g} are each independently selected from H, alkyl;
R₉ is H or fatty acyl;
provided that:
   at least one of R₁, R₂, R₅, and R₆ is H;
   R_{c}' and R_{d}' are not both H;
   when R₁, R₂, and R₅ are H, and R₆ is OH, R₃ is not methyl;
   when R₁ and R₆ are OH, R₂ and R₅ are H, R₃ is not methyl;
   when R₁, R₂, and R₆ are OH, and R₅ is H, R₃ is not methyl;
   when R₁, R₅, and R₆ are OH, and R₂ is H, R₃ is not methyl.

The compound or an ester, a stereoisomer, a prodrug, a solvate and a pharmaceutically acceptable salt thereof, according to the preceding, wherein
R₂ is selected from the group consisting of H, OH, -O-(C₁₋₃ alkylene-O)ₘ-C₁₋₃alkylene-X₃, -NH-(C₁₋₃alkylene-O)ₘ-C₁₋₃alkylene-X₃, -O-(C₁₋₃alkylene-NH)ₘ-C₁₋₃alkylene-X₃, -NH-(C₁₋₃alkylene-NH)ₘ-C₁₋₃alkylene-X₃, -O-(C₁₋₃alkylene-O)ₙ-CH[CH₂(O-C₁₋₃alkylene)ₚ-X₅]₂, -NH-(C₁₋₃alkylene-O)ₙ-CH[CH₂(O-C₁₋₃alkylene)ₚ-X₅]₂, -O-(C₁₋₃alkylene-O)ₙ -CH[CH₂(O-C₁₋₃alkylene)ₚ-X₅]₂, -O-(C₁₋₃alkylene-NH)ₙ-CH[CH₂(NH-C₁₋₃alkylene)ₚ-X₅]₂, and -NH-(C₁₋₃alkylene-NH)ₙ-CH[CH₂(NH-C₁₋₃alkylene)ₚ-X₅]₂; X₃ and X₅ are selected from ORₐ, NRₐR_{b}, NRₐR_{b}R_{c}, wherein, Ra, Rb, Rc are each independently selected from H, alkyl, arylalkyl, heteroarylalkyl, heterocyclylalkyl, and cycloalkylalkyl, said aryl, heteroaryl, heterocyclyl, cycloalkyl are optionally substituted by a group selected from: hydroxyl, alkoxyl, amino, alkyl, alkenyl, alkynyl, halogen, cyano, nitro, thiol, -S-alkyl, -S(O)-alkyl, -SO₂-alkyl, NR_{c}R_{d}, COORₑ, and CONR_{c}R_{d}, wherein R_{c}, R_{d}, and Rₑ are each independently selected from H and alkyl; or any two of Rₐ, R_{b}, and R_{c} are connected to form a heterocycle; or any one of Rₐ, R_{b}, and R_{c} is connected to a carbon atom of the alkylene group to which NRₐR_{b}R_{c} is connected to form a heterocycle; m, n, and p are integers of 0-5;

The compound or an ester, a stereoisomer, a prodrug, a solvate and a pharmaceutically acceptable salt thereof, according to the preceding, wherein
R₂ is selected from the group consisting of H, OH, -O-(C₁₋₃alkylene-O)ₘ-C₁₋₃alkylene-X₃, -NH-(C₁₋₃alkylene-O)ₘ-C₁₋₃alkylene-X₃, -O-(C₁₋₃alkylene-NH)ₘ-C₁₋₃alkylene-X₃, -NH-(C₁₋₃alkylene-NH)ₘ-C₁₋₃alkylene-X₃, -O-(C₁₋₃alkylene-O)ₙ-CH[CH₂(O-C₁₋₃alkylene)ₚ-X₅]₂, -NH-(C₁₋₃alkylene-O)ₙ-CH[CH₂(O-C₁₋₃alkylene)ₚ-X₅]₂, -O-(C₁₋₃alkylene-O)ₙ -CH[CH₂(O-C₁₋₃alkylene)ₚ-X₅]₂, -O-(C₁₋₃alkylene-NH)ₙ-CH[CH₂(NH-C₁₋₃alkylene)ₚ-X₅]₂, and -NH-(C₁₋₃alkylene-NH)ₙ-CH[CH₂(NH-C₁₋₃alkylene)ₚ-X₅]₂; X3, X5 are selected from ORₐ, NRₐR_{b}, NRₐR_{b}R_{c}, wherein Rₐ, R_{b}, R_{c} are each independently selected from H, C₁₋₆ alkyl, C₇₋₂₀ arylalkyl, C₇₋₂₀ heteroarylalkyl, C₇₋₂₀ heterocyclylalkyl, and C₇₋₂₀ cycloalkylalkyl, said aryl, heteroaryl, heterocyclyl, cycloalkyl are optionally substituted by a group selected from: hydroxyl, C₁₋₆ alkoxyl, amino, C₁₋₆ alkyl, C₁₋₆ alkenyl, C₁₋₆ alkynyl, halogen, cyano, nitro, thiol, -S-C₁₋₆alkyl, -S(O)-C₁₋₆alkyl, -SO₂-C₁₋₆alkyl, NR_{c}R_{d}, COORₑ, and CONR_{c}R_{d}, wherein R_{c}, R_{d}, and Rₑ are each independently selected from H, C₁₋₆ alkyl; or any two of Rₐ, R_{b} and R_{c} are connected to form a heterocycle; or any one of Rₐ, R_{b} and R_{c} is connected to a carbon atom of the alkylene group to which NRₐR_{b}R_{c} is connected to form a heterocycle; m, n and p are integers of 0-5.

The compound or an ester, a stereoisomer, a prodrug, a solvate and a pharmaceutically acceptable salt thereof, according to the preceding, wherein
R₂ is selected from H, OH,

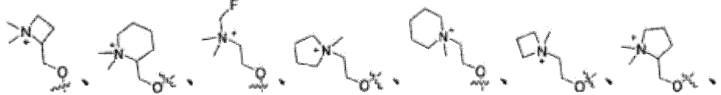

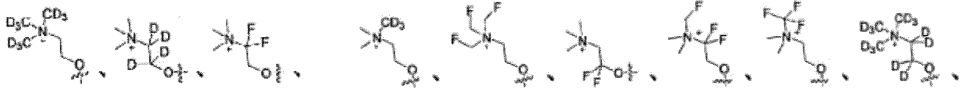

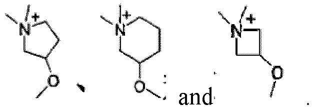

The compound or an ester, a stereoisomer, a prodrug, a solvate and a pharmaceutically acceptable salt thereof, according to the preceding, wherein
R₄ and R₈ are identical with or different from each other and are each independently selected from the group consisting of H, C₁₋₆ alkyl, C₁₋₆ alkenyl, C₁₋₆ alkynyl, C₆₋₁₄ aryl, C₆₋₁₄ heteroaryl, C₆₋₁₄ cycloalkyl, C₆₋₁₄ heterocyclyl, wherein the C₁₋₆ alkyl, C₁₋₆ alkenyl, C₁₋₆ alkynyl, C₆₋₁₄ aryl, C₆₋₁₄ heteroaryl, C₆₋₁₄ cycloalkyl, C₆₋₁₄ heterocyclyl are optionally substituted by halogen, cyano, nitro, thiol, -S-C₁₋₆alkyl, -S(O)-C₁₋₆alkyl, -SO₂ -C₁₋₆alkyl, NR_{c}R_{d}, COORₑ, and CONR_{c}'R_{d}', wherein R_{c}, R_{d}, Rₑ, R_{c}', and R_{d}' are each independently selected from H, C₁₋₆ alkyl.

The compound or an ester, a stereoisomer, a prodrug, a solvate and a pharmaceutically acceptable salt thereof, according to the preceding, wherein R₄ and R₈ are identical with or different from each other, and are each independently selected from H, C₁₋₆ alkyl, said C₁₋₆ alkyl is optionally substituted by halogen, cyano, nitro, thiol, -S-C₁₋₆alkyl, -S(O)-C₁₋₆alkyl, -SO₂-C₁₋₆alkyl, NR_{c}R_{d}, COORₑ, and CONR_{c}'R_{d}', wherein R_{c}, R_{d}, Rₑ, R_{c}', and R_{d}' are each independently selected from H, C ₁₋₆ alkyl.

The compound or an ester, a stereoisomer, a prodrug, a solvate and a pharmaceutically acceptable salt thereof, according to the preceding, wherein
R₉ is C₆₋₅₀ fatty acyl, which optionally contains an unsaturated alkenyl, cycloalkyl, aryl, heteroaryl, or heterocyclic.

The compound or an ester, a stereoisomer, a prodrug, a solvate and a pharmaceutically acceptable salt thereof, according to the preceding, wherein
R₉ is

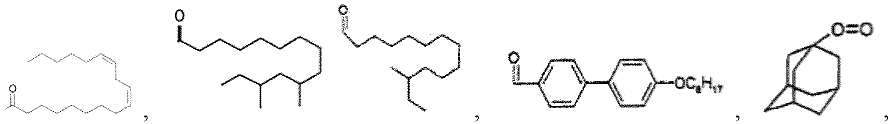

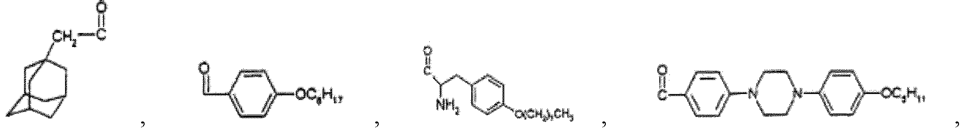

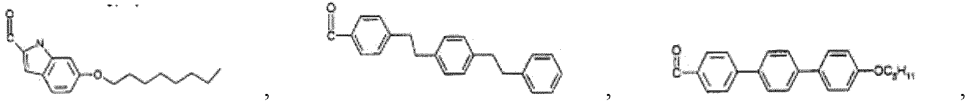

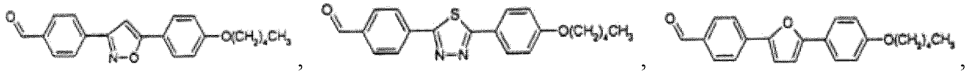

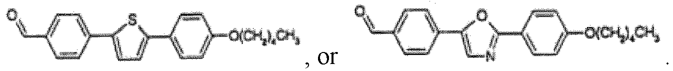

The compound or an ester, a stereoisomer, a prodrug, a solvate and a pharmaceutically acceptable salt thereof, according to the preceding,
provided that:
at least two of R₁, R₂, R₅, and R₆ are H;
R_{c}' and R_{d}' are not both H;
when R₁, R₂, and R₅ are H, and R₆ is OH, R₃ is not methyl;
when R₁ and R₆ are OH, R₂ and R₅ are H, R₃ is not methyl;
when R₁, R₂, and R₆ are OH, and R₅ is H, R₃ is not methyl;
when R₁, R₅, and R₆ are OH, and R₂ is H, R₃ is not methyl.

The compound or an ester, a stereoisomer, a prodrug, a solvate and a pharmaceutically acceptable salt thereof, according to the preceding,
provided that:
at least three of R₁, R₂, R₅, and R₆ are H;
R_{c}' and R_{d}' are not both H;
when R₁, R₂, and R₅ are H, and R₆ is OH, R₃ is not methyl;
when R₁ and R₆ are OH, R₂ and R₅ are H, R₃ is not methyl;
when R₁, R₂, and R₆ are OH, and R₅ is H, R₃ is not methyl;
when R₁, R₅, and R₆ are OH, and R₂ is H, R₃ is not methyl.

The compound or an ester, a stereoisomer, a prodrug, a solvate and a pharmaceutically acceptable salt thereof, according to the preceding, wherein
R₁, R₅, and R₆ are H;
R₂ is selected from H, -X₁-(alkylene-X₂)ₘ-alkylene-X₃, or -X₁-(alkylene-X₂)n -CH[CH₂(X₄-alkylene)ₚ-X₅ ]₂, wherein X₁, X₂, X₄ are selected from -O-, -NH-, and -S-; X3, X5 are selected from ORa, NRaRb, NRaRbRc, wherein Ra, Rb, Rc are each independently selected from H, alkyl, arylalkyl, heteroarylalkyl, heterocyclylalkyl, and cycloalkylalkyl, said aryl, heteroaryl, heterocyclyl, cycloalkyl are optionally substituted by a group selected from: hydroxyl, alkoxyl, amino, alkyl, alkenyl, alkynyl, halogen, cyano, nitro, thiol, -S-alkyl, -S(O)-alkyl, -SO₂-alkyl, NR_{c}R_{d}, COORₑ, and CONRₑRd, wherein Rc, Rd, and Re are each independently selected from H and alkyl; or any two of Ra, Rb, and Rc are connected to form a heterocycle; or any one of Ra, Rb, and Rc is connected to a carbon atom of the alkylene to which NRaRbRc is connected to form a heterocycle; m, n, and p are integers of 0-5.

The compound or an ester, a stereoisomer, a prodrug, a solvate and a pharmaceutically acceptable salt thereof, according to the preceding wherein
R₁, R₂, R₅, and R₆ are H.

The compound or an ester, a stereoisomer, a prodrug, a solvate and a pharmaceutically acceptable salt thereof, according to the preceding, wherein
R₁, R₂, R₅, and R₆ are H;
R₃, R₄, and R₈ are each independently H or methyl;
R₇ is H, OH, or -OSO₃H;
R₉ is

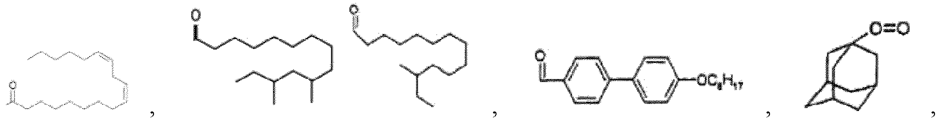

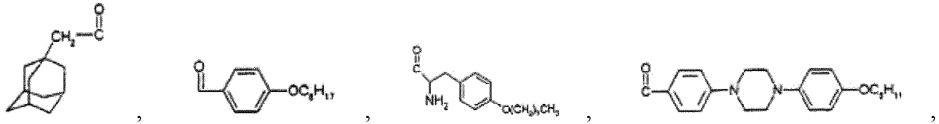

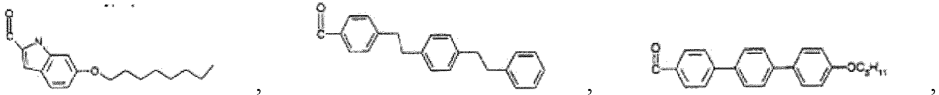

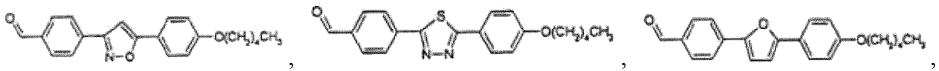

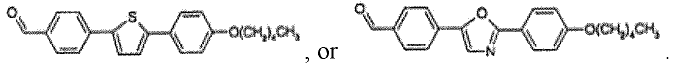

The compound according to the preceding, which is: 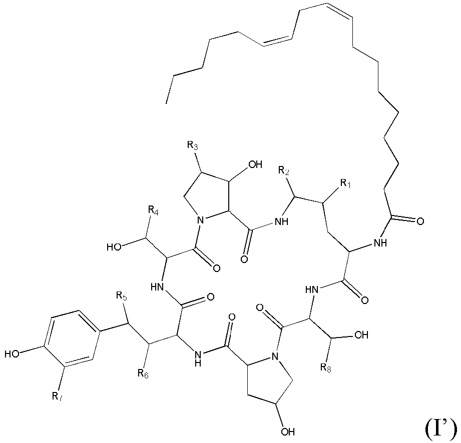 or an ester, a stereoisomer, a prodrug, a solvate and a pharmaceutically acceptable salt thereof,
wherein R₁, R₂, R₃, R₄, R₅, R₆, R₇ and R₈ are as defined herein.

The compound according to the preceding, which is: 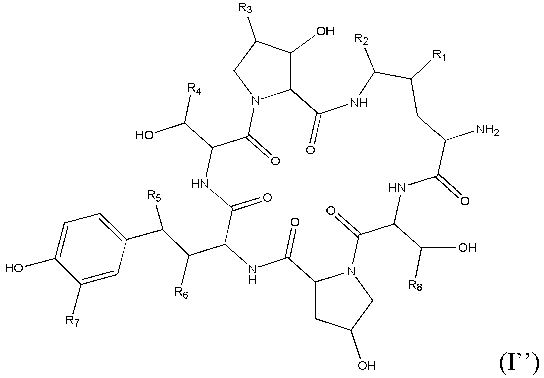 or an ester, a stereoisomer, a prodrug, a solvate and a pharmaceutically acceptable salt thereof,
wherein R₁, R₂, R₃, R₄, R₅, R₆, R₇ and R₈ are as defined herein.

The compound according to the preceding, which is: 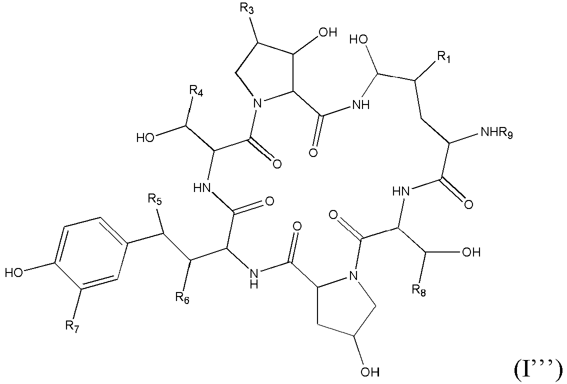 or an ester, a stereoisomer, a prodrug, a solvate and a pharmaceutically acceptable salt thereof,
wherein R₁, R₃, R₄, R₅, R₆, R₇, R₈ and R₉ are as defined herein.

The compound according to the preceding, which is: 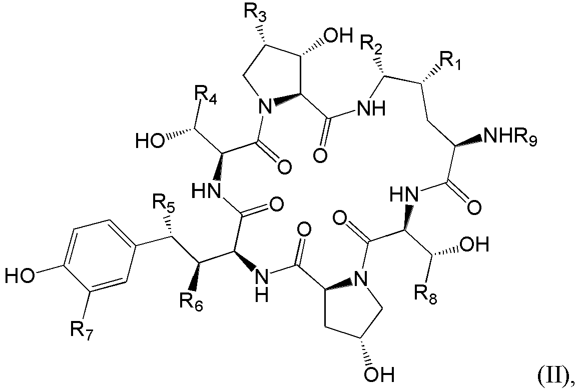 or an ester, a stereoisomer, a prodrug, a solvate and a pharmaceutically acceptable salt thereof,
wherein R₁, R₂, R₃, R₄, R₅, R₆, R₇, R₈, and R9 are as defined herein.

The compound according to the preceding, which is: 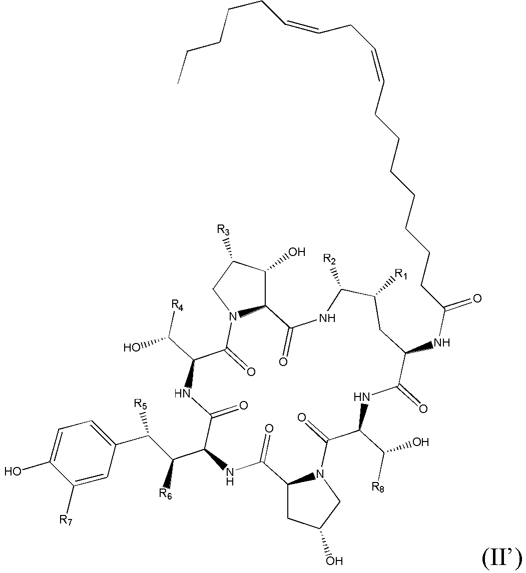 or an ester, a stereoisomer, a prodrug, a solvate and a pharmaceutically acceptable salt thereof,
wherein R₁, R₂, R₃, R₄, R₅, R₆, R₇ and R₈ are as defined herein.

The compound according to the preceding, which is: 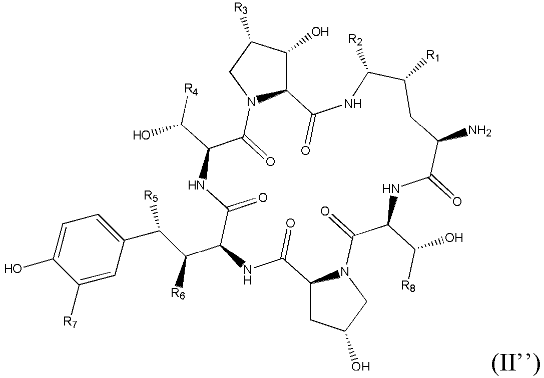 or an ester, a stereoisomer, a prodrug, a solvate and a pharmaceutically acceptable salt thereof,
wherein R₁, R₂, R₃, R₄, R₅, R₆, R₇ and R₈ are as defined herein.

The compound according to the preceding, which is: 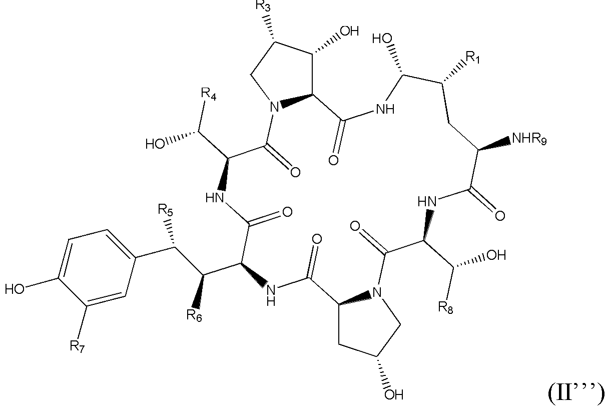 or an ester, a stereoisomer, a prodrug, a solvate and a pharmaceutically acceptable salt thereof,
wherein R₁, R₃, R₄, R₅, R₆, R₇, R₈ and R₉ are as defined herein.

The compound according to the preceding, which is: 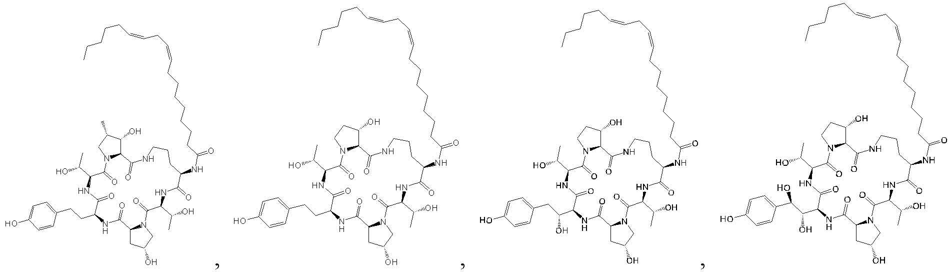 or an ester, a stereoisomer, a prodrug, a solvate and a pharmaceutically acceptable salt thereof.

Another aspect of the present invention relates to a pharmaceutical composition, comprising the compound, or an ester, a stereoisomer, a prodrug, a solvate and a pharmaceutically acceptable salt thereof, according to the preceding.

The pharmaceutical composition according to the preceding, further comprising an additional antifungal agent.

Yet another aspect of the present invention relates to an echinocandin biosynthetic gene cluster comprising two or more of the genes *ecdA, ecd*I*, ecd*K and htyE.

The echinocandin biosynthesis gene cluster according to the preceding, which comprises genes *ecd*A*, ecd*I and *htyE.*

The echinocandin biosynthesis gene cluster according to the preceding, which comprises genes *ecd*A*, ecd*I*, ecd*K and *htyE.*

The echinocandin biosynthesis gene cluster according to the preceding, which consists of genes *ecd*A*, ecd*I and *htyE.*

The echinocandin biosynthesis gene cluster according to the preceding, which consists of genes *ecd*A*, ecd*I*, ecd*K and *htyE.*

The echinocandin biosynthesis gene cluster according to the preceding, which further comprises one or more of the genes *ecd*G*, ecd*H or *htyF.*

The echinocandin biosynthesis gene cluster according to the preceding, which consists of genes *ecd*A*, ecd*I*, ecd*G and *htyE.*

The echinocandin biosynthesis gene cluster according to the preceding, which consists of genes *ecd*A*, ecd*I*, ecd*G*, hty*E*,* and *htyF.*

Another aspect of the present invention relates to a genetically engineered transformed strain comprising the echinocandin biosynthetic gene cluster according to any one of the preceding.

The genetically engineered transformant strain according to the preceding, which is a genetically engineered transformant strain deposited in the China Center for Type Culture Collection (Wuhan University, Wuhan, China) on November 2, 2021, with a deposit accession number of CCTCC M 20211360, named as *Aspergillus nidulans* LO8030-5.1; or a genetically engineered transformant strain deposited in the China Center for Type Culture Collection (Wuhan University, Wuhan, China) on February 28, 2022, with a deposit accession number of CCTCC M 2022168, named as *Aspergillus nidulans* LO8030-4.2.1; or a genetically engineered transformant strain deposited in the China Center for Type Culture Collection (Wuhan University, Wuhan, China) on February 20, 2023, with a deposit accession number of CCTCC M 2023155, named as *Aspergillus nidulans* LO8030-4.2.1.5; or a genetically engineered transformant strain deposited in the China Center for Type Culture Collection (Wuhan University, Wuhan, China) on February 20, 2023, with the deposit accession number CCTCC M 2023156, named as *Aspergillus nidulans* LO8030-4.2.1.9.

Another aspect of the present invention relates to a method for preparing the preceding compound, which includes: fermenting and culturing any of the preceding genetically engineered transformant strains to express the target product, and extracting and separating the fermentation broth to obtain the compound.

According to the preceding preparation method, the genetically engineered *Aspergillus nidulans* LO8030-5.1 or LO8030-4.2.1 or LO8030-4.2.1.5 or LO8030-4.2.1.9 is fermented in XSMM fermentation medium for 5-14 days; fungal liquid is separated from the fungal cells, extracted with methanol or ethyl acetate respectively, concentrated under vacuum respectively and combined into a total extract; the total extract is separated by silica gel column chromatography and high performance liquid column (HPLC) chromatography to obtain pure product.

According to the preceding preparation method, the eluent of the silica gel column chromatography is dichloromethane/methanol (gradient elution).

According to the preceding preparation method, the eluent of the HPLC is methanol/water, and the elution ratio (volume ratio) is 85:15.

On the other aspect, when the compound of the invention has been obtained by a biosynthetic method and the structure of the compound of the invention is known to those skilled in the art, the compound of the invention can also be prepared by a conventional organic synthesis method.

For example, amino acids 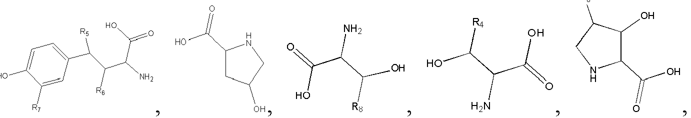 and 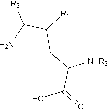 may be used as reaction starting materials, and the compound of the present invention is prepared by conventional polypeptide synthesis methods (including liquid phase synthesis and solid phase synthesis methods). During the reaction, if necessary, the active groups such as amino group and carboxyl group etc in the above amino acids that do not participate in the current reaction can be temporarily protected by conventional protecting groups, and the protecting groups can be removed after the current reaction is completed to proceed with the next reaction. Specific protecting groups may be referred to "Green's protective groups in organic synthesis 5th edition".

In one embodiment, the method for preparing the echinocandin compound represented by general formula (I) provided by the present invention further includes:
Step 1: the compound represented by formula (I') is delinoleoylated under the catalysis of echinocandin B deacylase to obtain the compound represented by formula (I"); 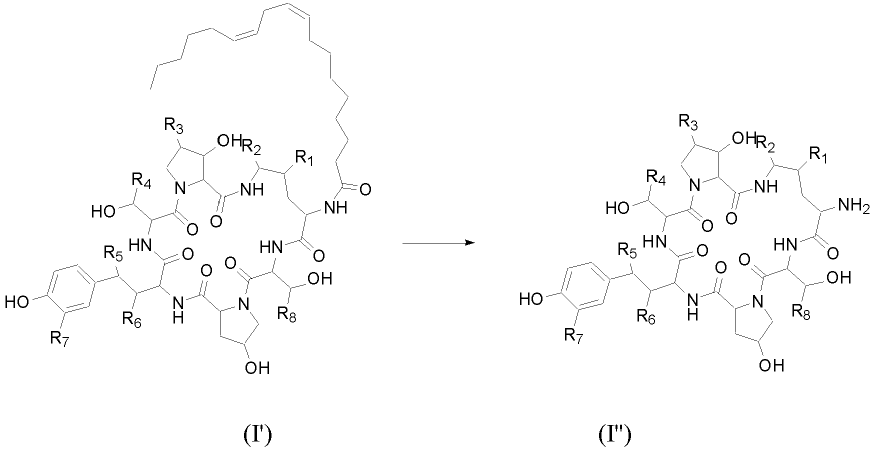
Step 2: the compound represented by formula (I") is reacted with R₉ -X under alkaline conditions to obtain the compound represented by formula (I); 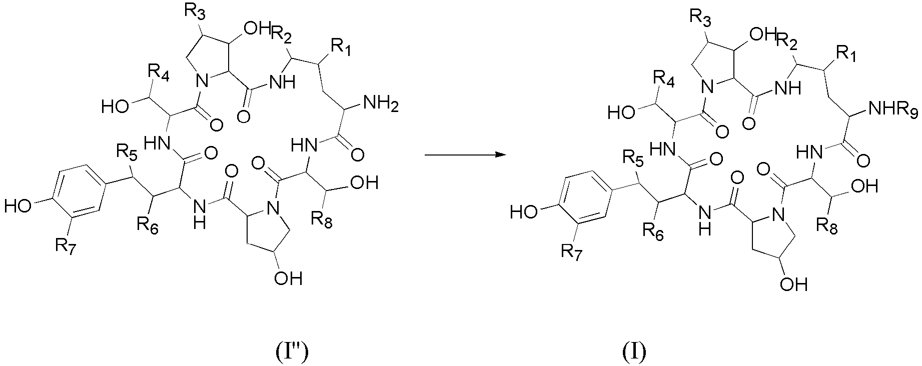 wherein, R₁, R₂, R₃, R₄, R₅, R₆, R₇, and R₈ are as defined herein, R₉ is fatty acyl, and X is a leaving group, including but not limited to halogen, active ester, and acid anhydride.

In a specific embodiment, the preparation method provided by the present invention includes:
Step 1: a linoleoyl group is removed from echinocandin E (compound 2) or echinocandin F (compound 1) under the catalysis of echinocandin B deacylase to obtain compound 2' or compound 1', respectively: 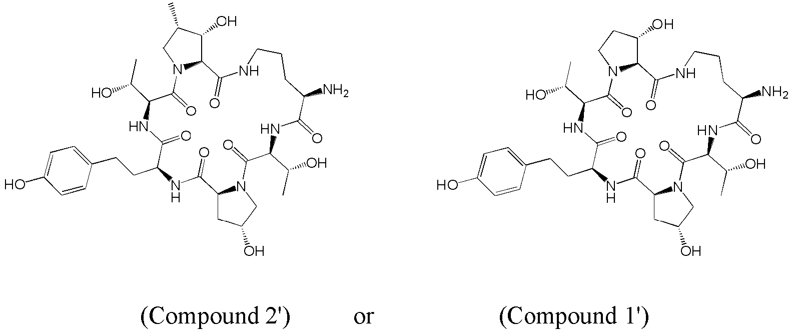
Step 2: Compound 2' or compound 1' is reacted with R₉-X under alkaline conditions to obtain the compound represented by the following formula.

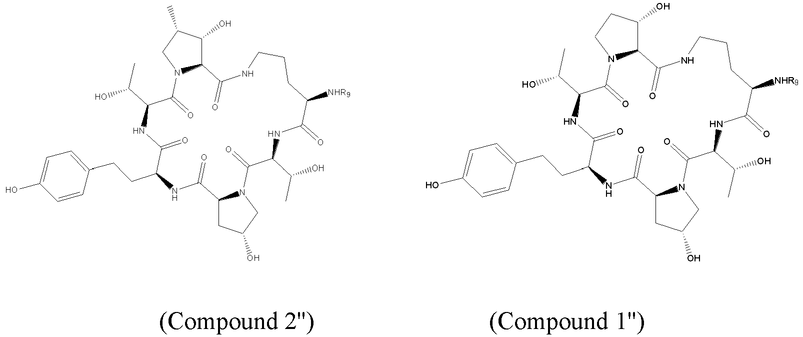

In a further embodiment, the method for preparing the echinocandin compound represented by general formula (I) provided by the present invention further includes:
the compound represented by formula (I"') is reacted with a halide or an active ester of R₂H, or in the presence of a condensing agent to obtain the compound represented by formula (I). 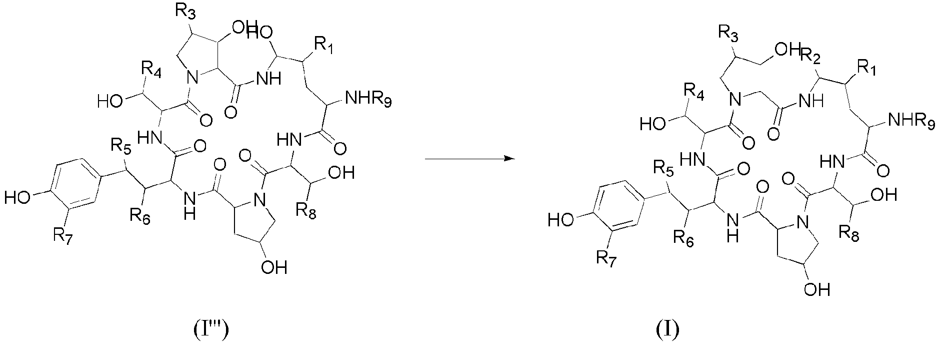
wherein, R₁, R₃, R₄, R₅, R₆, R₇, R₈, and R₉ are as defined herein; R₂ is -X₁-(alkylene-X₂)ₘ-alkylene-X₃, or -X₁-(alkylene-X₂)n -CH[CH₂(X₄-alkylene)ₚ-X₅ ]₂, wherein X₁, X₂, X₄ are selected from -O-, -NH-, and -S-; X3, X5 are selected from ORa, NRaRb, NRaRbRc, wherein Ra, Rb, Rc are each independently selected from H, alkyl, arylalkyl, heteroarylalkyl, heterocyclylalkyl, and cycloalkylalkyl, said aryl, heteroaryl, heterocyclyl, cycloalkyl are optionally substituted by a group selected from: hydroxyl, alkoxyl, amino, alkyl, alkenyl, alkynyl, halogen, cyano, nitro, thiol, -S-alkyl, -S(O)-alkyl, -SO₂-alkyl, NR_{c}R_{d}, COORₑ, and CONR_{c}R_{d}, wherein R_{c}, R_{d}, and Rₑ are each independently selected from H and alkyl; or any two of Rₐ, R_{b}, and R_{c} are connected to form a heterocycle; or any one of Rₐ, R_{b}, and R_{c} is connected to a carbon atom of the alkylene to which NRₐR_{b}R_{c} is connected to form a heterocycle; m, n, and p are integers of 0-5.

Another aspect of the present invention provides a method for treating or preventing fungal infection, comprising administering a therapeutically effective amount of the compound of the present invention to a patient in need thereof.

Another aspect of the present invention relates to use of the aforementioned compound in the preparation of a medicament for treating or preventing fungal infection-related diseases.

According to the aforementioned method or use, the fungus is selected from any one or more of *Aspergillus* (such as *Aspergillus flavus, Aspergillus fumigatus, Aspergillus glaucus, Aspergillus nidulans, Aspergillus niger* and *Aspergillus terreus), Blastomyces dermatitidis, Candida* (such as *Candida albicans, Candida glabrata, Candida tropicalis, Candida parapsilosis, Candida krusei* and *Candida guilliermondii*)*, Coccidioides immitis, Cryptococcus* (such as *Cryptococcus neoformans, Cryptococcus albicans* and *Cryptococcus laurentii*)*, Histoplasma capsulatum var.capsulatum, Histoplasma capsulatum* duboisii, *Paracoccidioides brasiliensis, Sporothrix schenckii, Absidia corymbifera, Rhizomucor pusillus* and *Rhizopus arrhizus.*

According to the proceding method or use, the fungal infection-associated diseases are selected from one or more of scalp tinea, tinea corporis, tinea pedis, onychomycosis, perionychomycosis, tinea versicolor, thrush, vaginal candidiasis, respiratory tract candidiasis, biliary tract candidiasis, esophageal candidiasis, urethral candidiasis, systemic candidiasis, mucosal and skin candidiasis, aspergillosis, mucormycosis, paracoccidioidomycosis, North American blastomycosis, histoplasmosis, coccidioidomycosis, sporotrichosis, fungal sinusitis and chronic sinusitis.

The beneficial effects of the present invention are:
The present inventors have designed a series of biosynthetic gene clusters for echinocandin B deoxidation analogues, and specifically produced the desired echinocandin B deoxidation analogues by culturing and expressing the same in a heterologous host. Compared with the prior art, this method has simple product components, high conversion efficiency, few reaction impurities, easy separation and purification, which is conducive to industrialization.

Compared with the wild-type echinocandin B producing strain, a secondary metabolic profile of the fermentation product of the genetically engineered strain is simple and the yield is higher. The yield of the echinocandin B deoxidation analogue produced by the *Aspergillus nidulans* genetically engineered transformant strain of the present invention is significantly higher than the echinocandin B yield of the wild type echinocandin B producing strain.

The echinocandin compounds prepared by the invention exhibit good antifungal activity and have the potential to be developed into antifungal drugs.

Studies have shown that the detachment of hydroxyl groups on the ornithine residue and homotyrosine residue of echinocandin B can lead to the instable structure of echinocandin B. However, the echinocandin B deoxidation analogues of the present invention, especially the homotyrosine residue and ornithine residue in the structures of echinocandin E and echinocandin F, have no hydroxylation modification, but have higher chemical stability than the echinocandin B cyclic hexapeptide structure, which is more conducive to subsequent chemical derivatization.

### Brief Description of the Drawings

Fig. 1 illustrates a schematic diagram of the construction of a plasmid by connecting multiple fragments using yeast homologous recombination. "F 1-F3" are multiple segments of a target gene or multiple target genes; the plasmid backbone includes "Ori" E. coli origin of replication, "Amp^{R} " E. coli ampicillin resistance screening marker, "2µ" yeast origin of replication, and "URA" yeast uracil auxotrophic screening marker.
Fig. 2 illustrates a schematic diagram of the transformation of the Ecd/Hty gene cluster in *Aspergillus nidulans* LO8030.
Fig. 3 illustrates a schematic diagram of the map of a plasmid pYX1 and the first round of transformation to construct a gene expression cassette.
Fig. 4 illustrates a schematic diagram of the map of a plasmid pYX5 and a second round of transformation to construct a gene expression cassette.
Fig. 5 illustrates a schematic diagram of the map of a plasmid pYX9 and a third round of transformation to construct a gene expression cassette.
Fig. 6 illustrates a schematic diagram of the map of a control plasmid pYX14 and the transformation to construct a gene expression cassette.
Fig. 7 illustrates a schematic diagram of the map of a plasmid pYX10 and a fourth round of transformation.
Fig. 8 illustrates a schematic diagram of the map of a control plasmid pYX12 and the transformation.
Fig. 9 illustrates the results of sequencing and comparation of biosynthetic genes inserted into *Aspergillus nidulans* genetically engineered transformant LO8030-5.1 (a: *ecd*A; b: *ecd*I; c: *ecd*K; *d: htyE).*
Fig. 10 illustrates transcriptional analysis of biosynthetic genes in *Aspergillus nidulans* genetically engineered transformant LO8030-5.1.
Fig. 11 illustrates HPLC and LC-MS analysis of metabolites of transformant strains (LO8030-4.1, LO8030-4C.1, LO8030-5C.1, LO8030-5.1). (A) HPLC tracking (222 nm) of metabolites from different transformant strains. (B) MS data of compounds 1 and 2. "Exp" represents the experimental *m*/*z* value; "The" represents the predicted *m*/*z* value for the tetradeoxy compound.
Fig. 12 illustrates a schematic diagram of the biosynthesis of the echinocandin B deoxidation analogues of the present invention.
Fig. 13 illustrates a schematic diagram of the map of a plasmid pJQ10 and the transformation.
Fig. 14A illustrates a peak area-concentration standard curve of compound 2 (echinocandin E); Fig. 14B illustrates a peak area-concentration standard curve of compound 1 (echinocandin F).

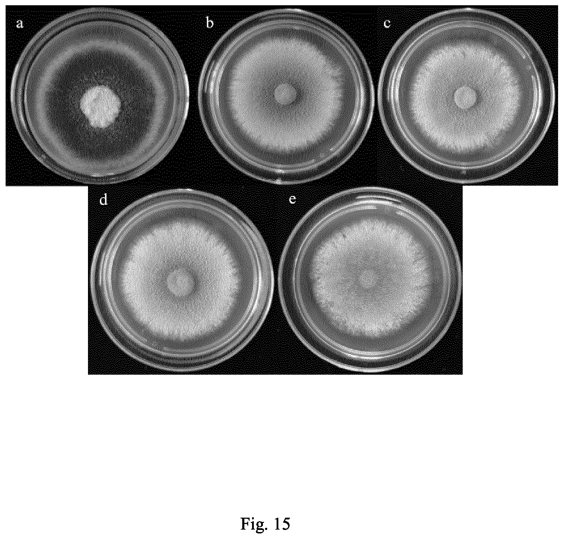

Fig. 15 illustrates the growth of *Aspergillus nidulans* LO8030 and its genetically engineered transformant strains (a. LO8030, b. LO8030-1.3, c. LO8030-2.2, d. LO8030-4.1, e. LO8030-5.1) on GMM supplemented with appropriate additives.

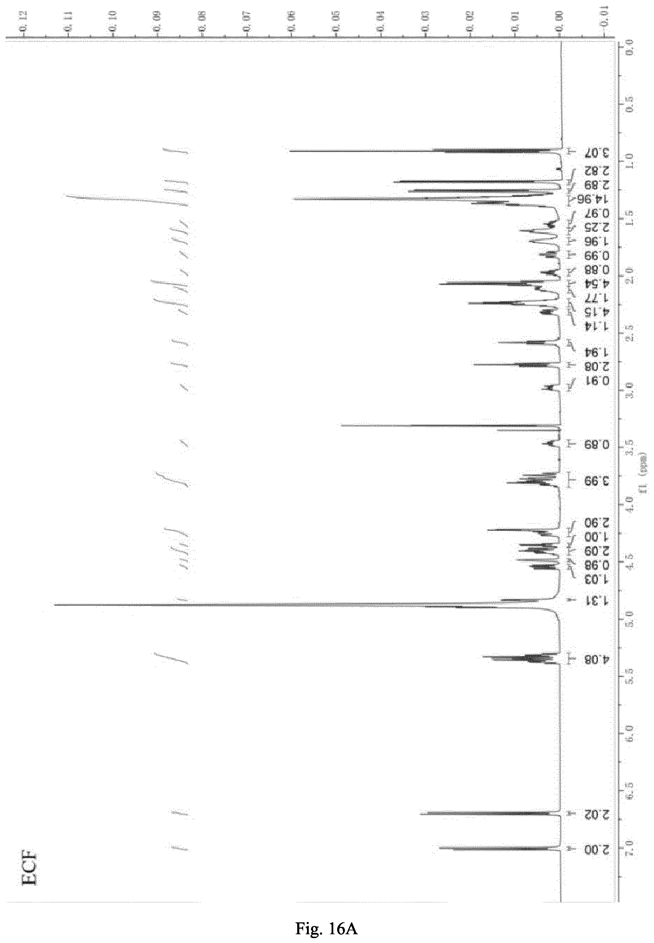

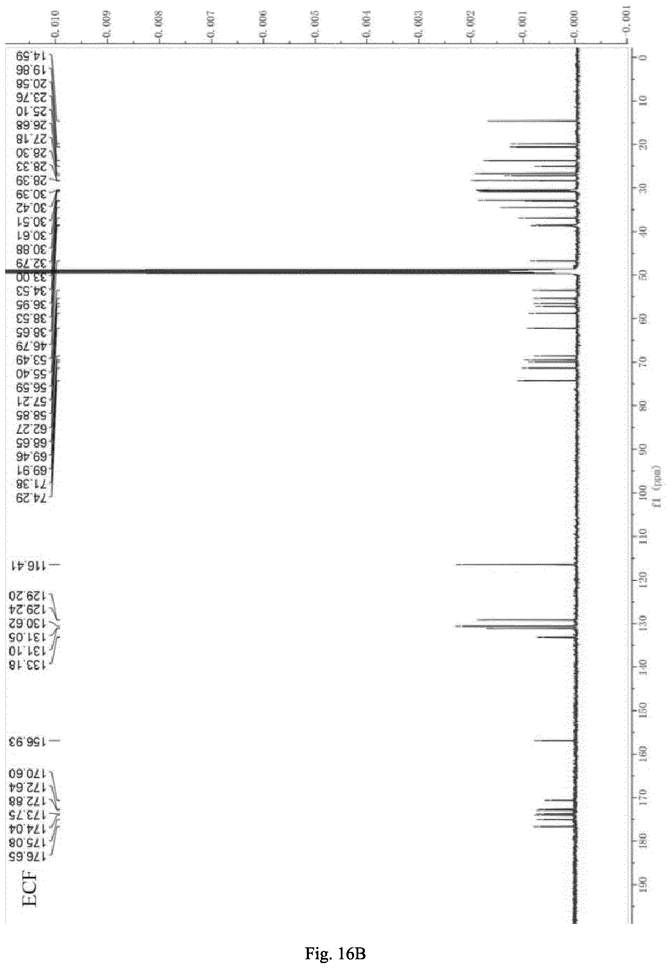

Fig. 16A illustrates the ¹H NMR spectrum of Compound 1 (CD₃OD, 600 MHz); Fig. 16B illustrate the ¹³C NMR spectrum of Compound 1 (CD₃OD, 150 MHz).

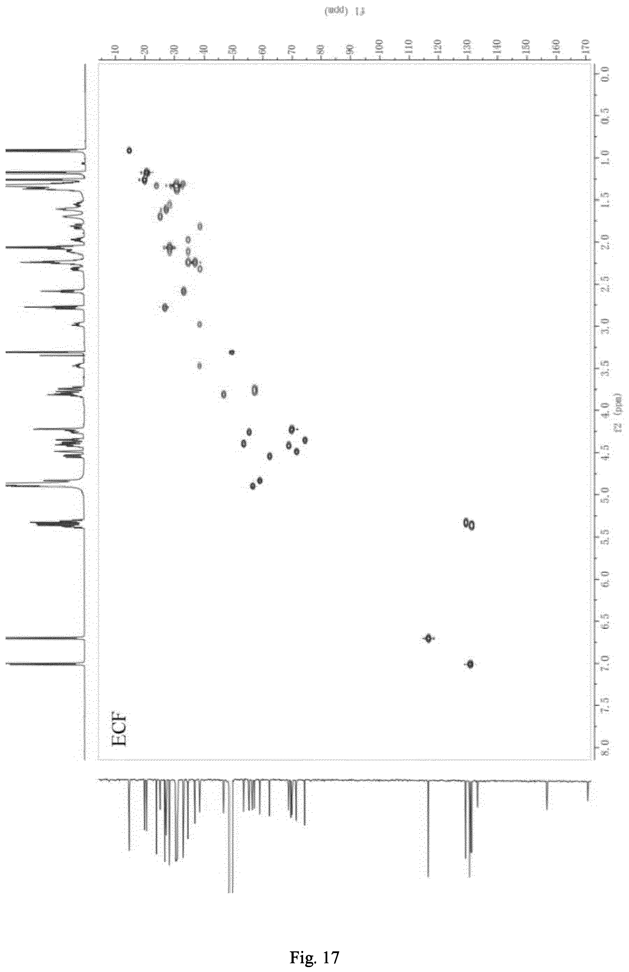

Fig. 17 illustrates the ¹H-¹³C HSQC spectrum of Compound 1 (CD₃OD, 600 MHz).

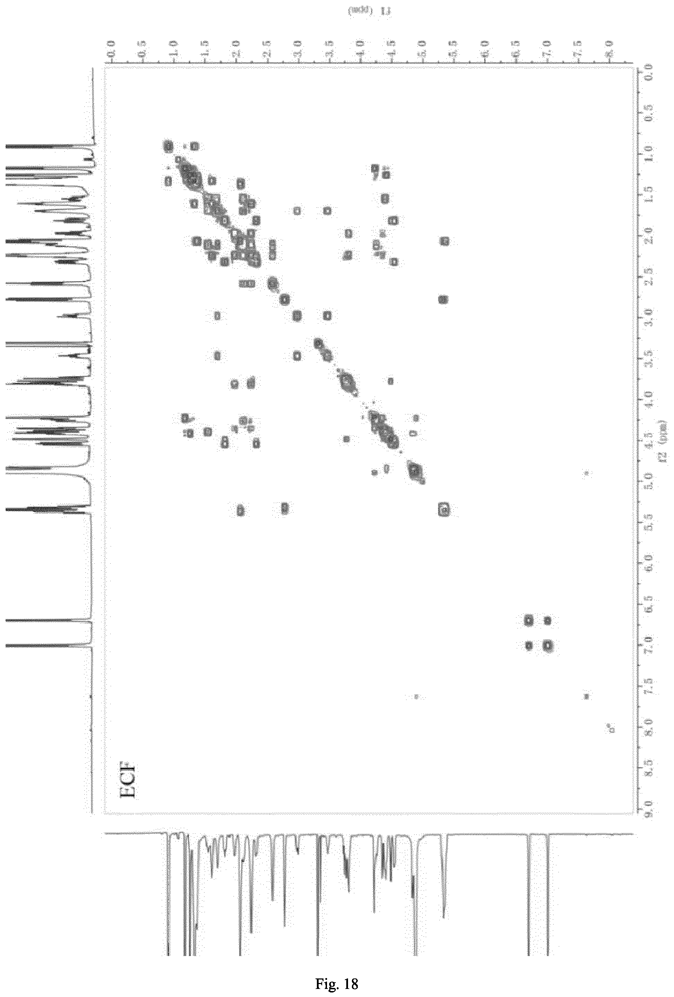

Fig. 18 illustrates the ¹H-¹H COSY spectrum of Compound 1 (CD₃OD, 600 MHz).

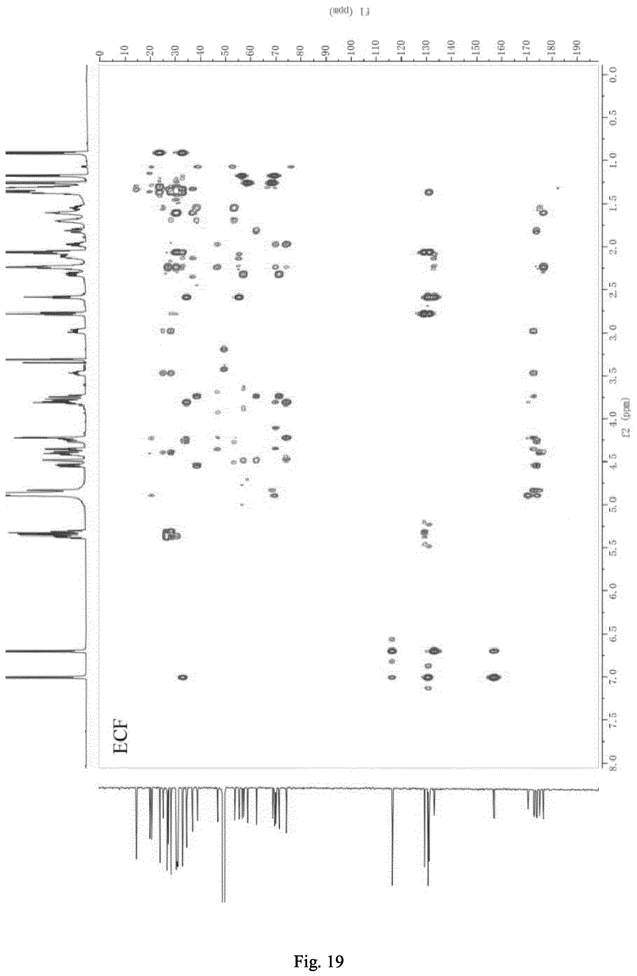

Fig. 19 illustrates the ¹H-¹³C HMBC spectrum of Compound 1 (CD₃OD, 600 MHz).

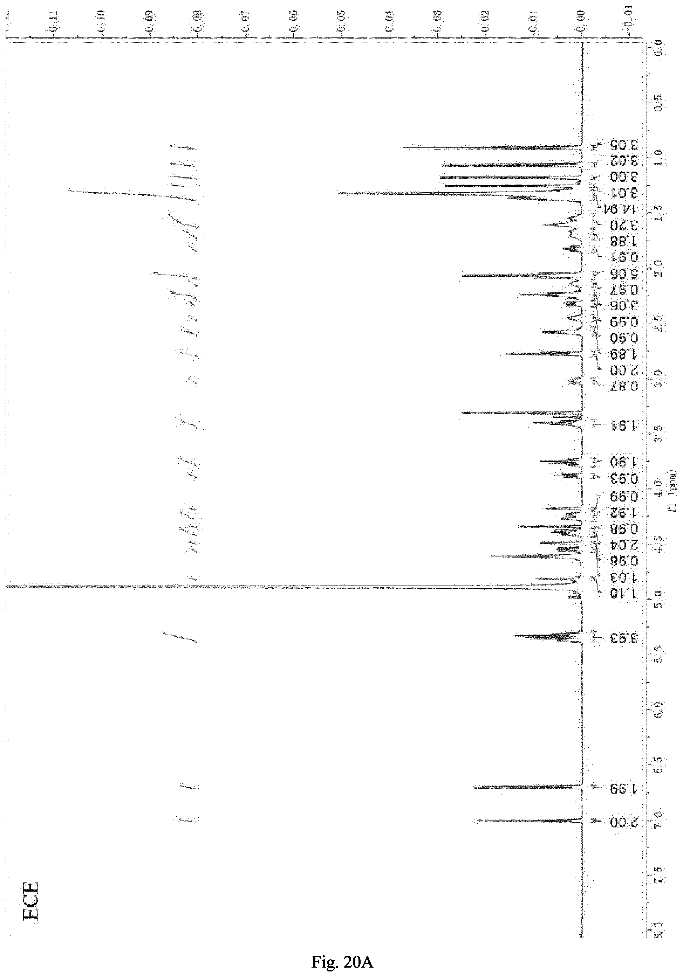

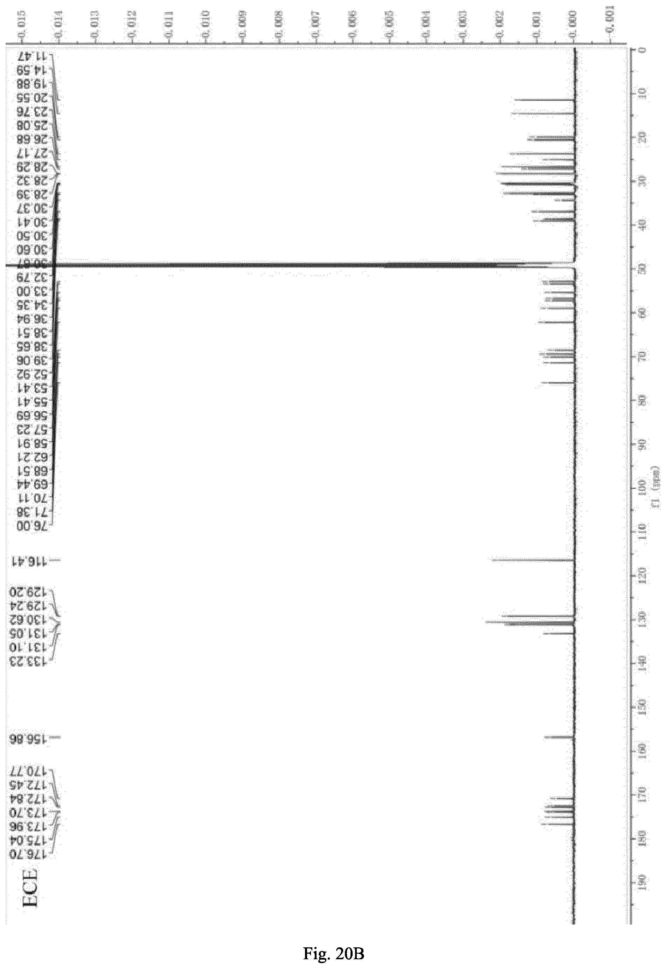

Fig. 20A illustrates the ¹H NMR spectrum of Compound 2 (CD₃OD, 600 MHz); Fig. 20B illustrate the ¹³C NMR spectrum of Compound 2 (CD₃OD, 150 MHz).

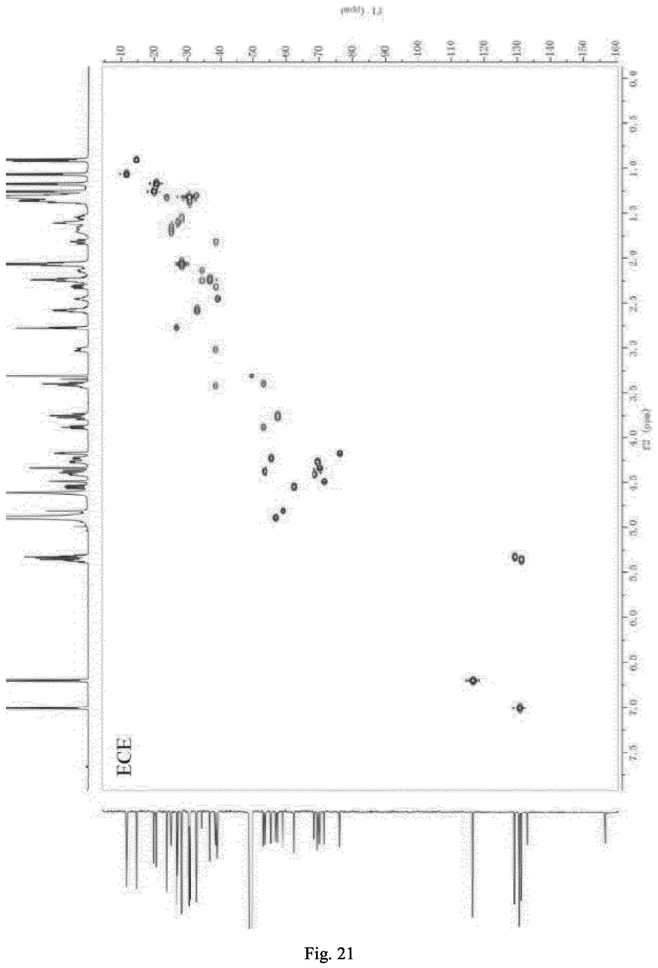

Fig. 21 illustrates the ¹H-¹³C HSQC spectrum of Compound 2 (CD₃OD, 600 MHz).

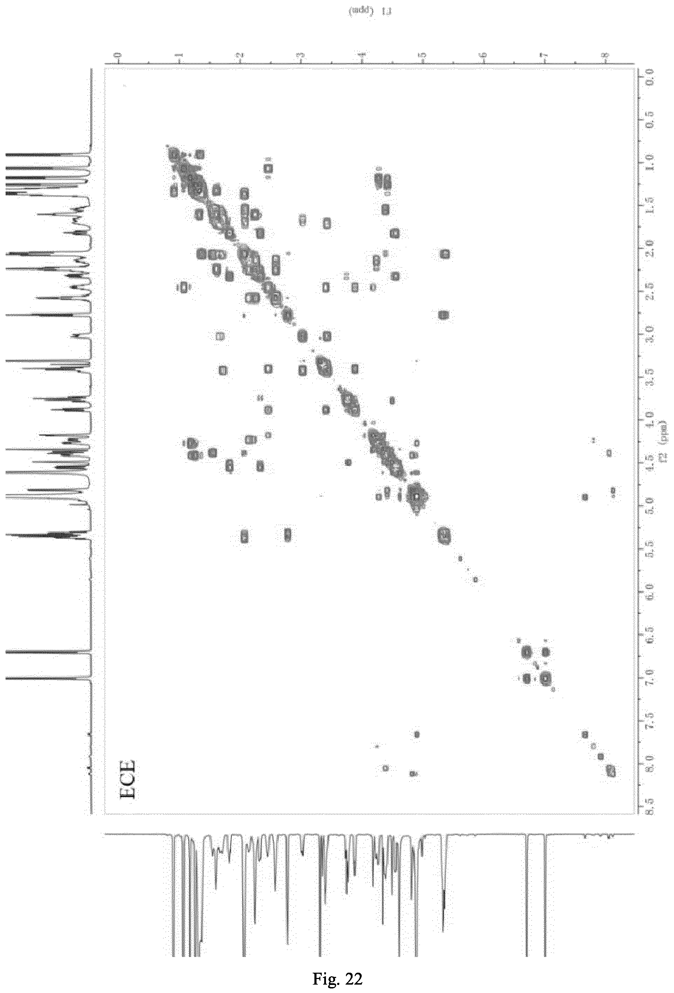

Fig. 22 illustrates the ¹H-¹H COSY spectrum of Compound 2 (CD₃OD, 600 MHz).

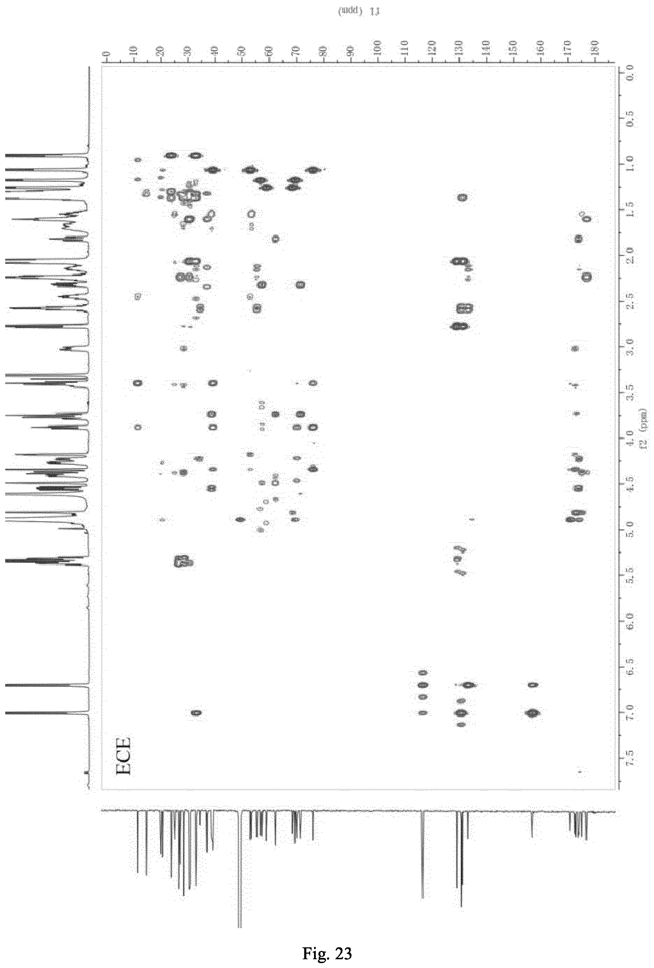

Fig. 23 illustrates the ¹H-¹³C HMBC spectrum of Compound 2 (CD₃OD, 600 MHz).

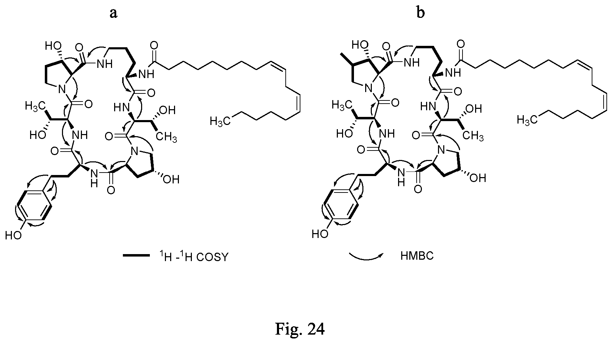

Fig. 24 illustrates ¹H-¹H COSY and critical HMBC correlation peaks of Compound 1 and Compound 2 (a: Compound 1; b: Compound 2).
Fig. 25 illustrates a schematic diagram of the map of a plasmid pJQ5 and the transformation.
Fig. 26 illustrates a schematic diagram of the map of a plasmid pJQ9 and the transformation.

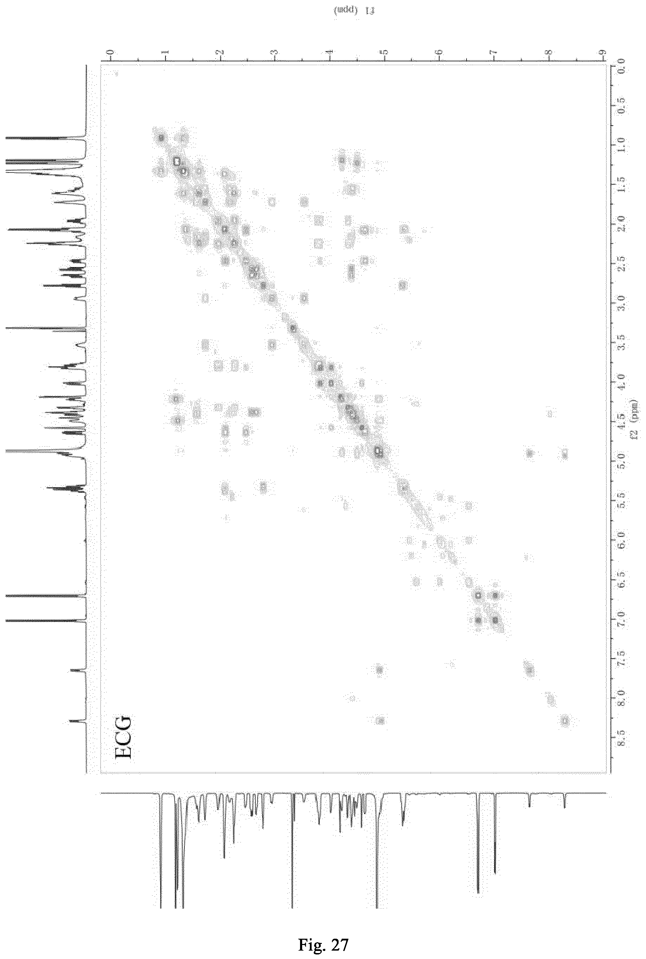

Fig. 27 illustrates the ¹H-¹H COSY spectrum of Compound 3 (echinocandin G) (CD₃OD, 600 MHz).
Fig. 28 illustrates the ¹H-¹³C HMBC spectrum of Compound 3 (CD₃OD, 600 MHz).
Fig. 29 illustrates the ¹H-¹H COSY spectrum of Compound 4 (echinocandin H) (CD₃OD, 600 MHz).

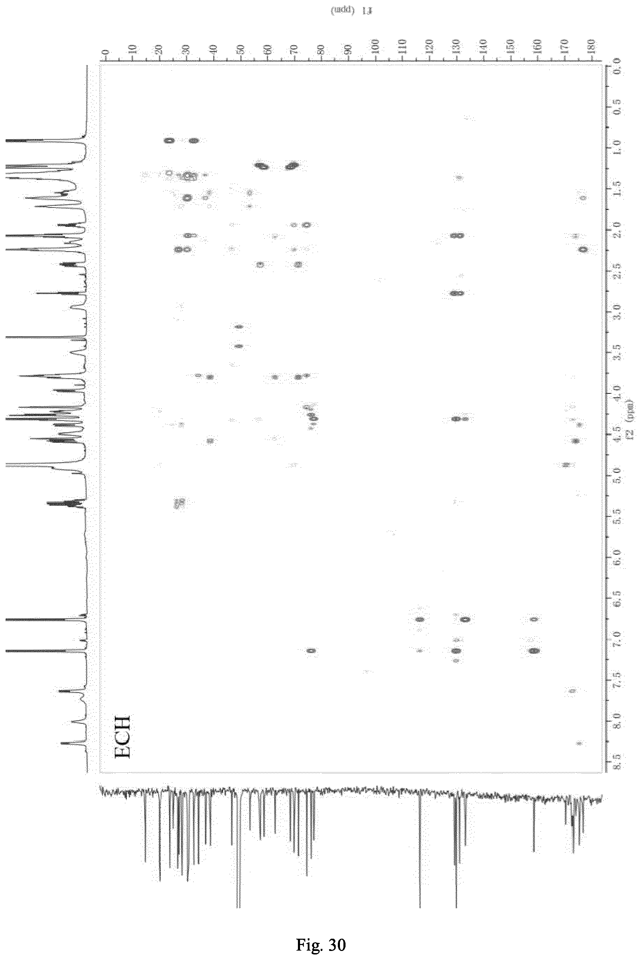

Fig. 30 illustrates the ¹H-¹³C HMBC spectrum of Compound 4 (CD₃OD, 600 MHz).

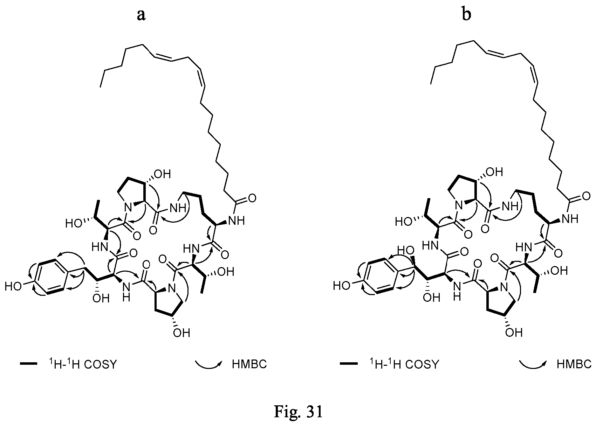

Fig. 31 illustrates ¹H-¹H COSY and critical HMBC correlation peaks of Compound 3 and Compound 4 (a: Compound 3; b: Compound 4).

### Detailed Description

### Definition

Unless otherwise defined herein, scientific and technical terms used in connection with the present disclosure shall have the meanings commonly understood by one of ordinary skill in the art. The meaning and scope of terms should be clear, however, in the event of any potential ambiguity, the definitions provided hereinafter take precedence over any dictionary or external definition.

As used herein, the term "ester" refers to an ester formed by an esterification reaction between OH of the compound represented by formula (I) and an organic acid, an inorganic acid, etc. The ester may undergo hydrolysis under acidic or alkaline conditions to generate the corresponding acid or alcohol.

As used herein, the term "stereoisomer(s)" refers to compounds that have identical chemical constitution and connectivity but have different orientations of their atoms in space that are not interchangeable by single bond rotation. "Stereoisomer(s)" includes "diastereomer(s)" and "enantiomer(s)". "Diastereomer(s)" refers to stereoisomers that have two or more chiral centers and of which molecules are not mirror images of one another. Diastereomers have different physical properties, such as melting points, boiling points, spectral characteristics, and reactivity. Diastereomeric mixtures may be separated under high resolution analytical procedures such as crystallization, electrophoresis and chromatography. "Enantiomers" refers to two stereoisomers of a compound that are non-superimposable mirror images of one another.

As used herein, the term "pharmaceutically acceptable salt" refers to a pharmaceutically acceptable organic or inorganic salt of a compound of the present invention. Exemplary salts include, but are not limited to, sulfates, citrates, acetates, oxalates, chlorides, bromides, iodides, nitrates, bisulfates, phosphates, acidic phosphates, isonicotinates, lactates, salicylates, acidic citrates, tartrates, oleates, tannates, pantothenates, bitartrates, ascorbates, succinates, maleates, gentisinates, fumarates, gluconates, glucuronates, saccharates, formates, benzoates, glutamates, methanesulfonates (mesylate), ethanesulfonates, benzenesulfonates, p-toluenesulfonates, and pamoates; or ammonium salts (e.g., primary, secondary, tertiary, quaternary ammonium salts), metal salts (e.g., sodium, potassium, calcium, magnesium, manganese, iron, zinc, copper, lithium, aluminum salt).

As used herein, the term "pharmaceutically acceptable" means that the substance or composition must be chemically and/or toxicologically compatible with the other ingredients comprising the formulation and/or the mammals to be treated therewith.

As used herein, the term "treatment" refers to both therapeutic treatment and prophylactic or preventative or preventative measures, wherein the object is to prevent or slow down (lessen) the undesired pathological change or condition. For purposes of this invention, beneficial or desired clinical results include, but are not limited to, alleviation of symptoms, reduction in disease severity, delay or slowing of disease progression, improvement or palliation of the disease state, and remission (whether partial or total), whether detectable or undetectable.

As used herein, the term "therapeutically effective amount" refers to an amount of a compound of the invention that can: (i) treat or prevent a disease or condition described herein, (ii) ameliorate or eliminate one or more diseases or conditions described herein, or (iii) prevent or delay the onset of one or more symptoms of a disease or condition described herein.

As used herein, the term "alkyl" refers to a linear or branched saturated hydrocarbon group having 1 to 20 carbon atoms. Preferably, the alkyl is an alkyl having 1 to 12 carbon atoms. More preferably, the alkyl is an alkyl having 1 to 6 carbon atoms. Most preferably, the alkyl is an alkyl having 1 to 4 carbon atoms. Examples of alkyl include, but are not limited to, methyl, ethyl, 1-propyl (n-propyl), 2-propyl (isopropyl), 1-butyl (n-butyl), 2-methyl-1-propyl (isobutyl), 2-butyl (sec-butyl), 2-methyl-2-propyl (tert-butyl), 1-pentyl (n-pentyl), 2-pentyl, 3-pentyl, 2-methyl-2-butyl, 3-methyl-2-butyl, 3-methyl-1-butyl, 2-methyl-1-butyl, 1-hexyl, 2-hexyl, 3-hexyl, 2-methyl-2-pentyl, 3-methyl-2-pentyl, 4-methyl-2-pentyl, 3-methyl-3-pentyl, 2-methyl-3-pentyl, 2,3-dimethyl-2-butyl, 3,3-dimethyl-2-butyl, 1-heptyl, 1-octyl, 1-nonyl, 1-decyl, etc.

As used herein, the term "alkylene" refers to a divalent alkyl as defined above. As used herein, the alkylene has 1 to 10 carbon atoms (i.e., C₁₋₁₀ alkylene), 1 to 8 carbon atoms (i.e., C₁₋₈ alkylene), 1 to 6 carbon atoms (i.e., C₁₋₆ alkylene), or 1 to 4 carbon atoms (i.e., C₁₋₄ alkylene).

As used herein, the term "alkenyl" refers to a hydrocarbon containing at least one carbon-carbon double bond and having 2 to 20 carbon atoms (i.e., C₂₋₂₀ alkenyl), 2 to 8 carbon atoms (i.e., C₂₋₈ alkenyl), 2 to 6 carbon atoms (i.e., C₂₋₆ alkenyl), or 2 to 4 carbon atoms (i.e., C₂₋₄ alkenyl). Examples of the alkenyl include, for example, ethenyl, propenyl, butadienyl (including 1,2-butadienyl and 1,3-butadienyl).

As used herein, the term "alkynyl" refers to a hydrocarbon group containing at least one carbon-carbon triple bond and having 2 to 20 carbon atoms (i.e., C₂₋₂₀ alkynyl), 2 to 8 carbon atoms (i.e., C₂₋₈ alkynyl), 2 to 6 carbon atoms (i.e., C₂₋₆ alkynyl), or 2 to 4 carbon atoms (i.e., C₂₋₄ alkynyl). The term "alkynyl" also includes those groups having one triple bond and one double bond.

As used herein, the term "halogen" refers to fluorine, chlorine, bromine, and iodine.

As used herein, the term "cycloalkyl" refers to a monovalent saturated carbocyclic group. Preferably, the cycloalkyl is a 3 to 8 membered monocyclic group. More preferably, the cycloalkyl is a 3 to 6 membered monocyclic group. Examples of cycloalkyl include, but are not limited to, cyclopropyl, cyclobutyl, cyclopentyl, cyclohexyl, etc.

The term "aryl" as used herein refers to a single all-carbon aromatic ring or a multiple condensed all-carbon ring system wherein at least one ring is aromatic, and wherein the aryl has 6 to 20 carbon atoms, 6 to 14 carbon atoms, 6 to 12 carbon atoms, or 6 to 10 carbon atoms. Aryl includes phenyl. Aryl also includes multiple condensed ring systems (e.g., ring systems comprising 2, 3 or 4 rings) having about 9 to 12 carbon atoms in which at least one ring is aromatic and wherein the other ring(s) may be aromatic or not aromatic (i.e., carbocycle). Such multiple condensed ring system is optionally substituted with one or more (e.g., 1, 2 or 3) oxo groups on any carbocycle portion of the multiple condensed ring system. The rings of the multiple condensed ring system can be connected to each other via condensed, spiro and bridged bonds when allowed by valency requirements. It is to be understood that the point of attachment of a multiple condensed ring system, as defined above, can be at any position of the ring system including the aromatic portion or the carbocycle portion of the ring. Exemplary aryl includes, but are not limited to, phenyl, indenyl, naphthyl, 1, 2, 3, 4-tetrahydronaphthyl, anthryl, and the like.

As used herein, the term "heteroaryl" refers to a 5-to 6-membered aromatic ring having at least one atom other than carbon in the ring, wherein the atom is selected from oxygen, nitrogen and sulfur; "heteroaryl" also includes multiple condensed ring systems having 8-16 atoms, which have at least one such aromatic ring, and multiple condensed ring systems are further described below. Thus, "heteroaryl" includes single aromatic rings having about 1 to 6 carbon atoms and about 1-4 heteroatoms selected from the group consisting of oxygen, nitrogen and sulfur. The sulfur and nitrogen atoms may also be present in an oxidized form if the ring is aromatic. Exemplary heteroaryl ring systems include but are not limited to pyridyl, pyrimidinyl, oxazolyl or furyl. "Heteroaryl" also includes multiple condensed ring systems (e.g., ring systems comprising 2 or 3 rings) wherein a heteroaryl, as defined above, is condensed with one or more rings selected from heteroaryls (to form for example a naphthyridinyl such as 1,8-naphthyridinyl), heterocycles, (to form for example a 1, 2, 3, 4-tetrahydronaphthyridinyl such as 1,2,3,4-tetrahydro-1,8-naphthyridinyl), carbocycles (to form for example 5,6,7,8-tetrahydroquinolyl) and aryls (to form for example indazolyl) to form the multiple condensed ring system. Thus, a heteroaryl (a single aromatic ring or multiple condensed ring system) has about 1-15 carbon atoms and about 1-6 heteroatoms within the heteroaryl ring. Multiple condensed ring systems may be optionally substituted with one or more (e.g., 1, 2, 3 or 4) oxo groups on the carbocycle or heterocycle portions of the condensed ring. The rings of the multiple condensed ring system can be connected to each other via condensed, spiro and bridged bonds when allowed by valency requirements. It is to be understood that the individual rings of the multiple condensed ring system may be connected in any order relative to one another. It is also to be understood that the point of attachment of a multiple condensed ring system (as defined above for a heteroaryl) can be at any position of the multiple condensed ring system including a heteroaryl, heterocycle, aryl or carbocycle portion of the multiple condensed ring system. It is also to be understood that the point of attachment for a heteroaryl or heteroaryl multiple condensed ring system can be at any suitable atom of the heteroaryl or heteroaryl multiple condensed ring system including a carbon atom and a heteroatom (e.g., a nitrogen). Exemplary heteroaryls include but are not limited to pyridyl, pyrrolyl, pyrazinyl, pyrimidinyl, pyridazinyl, pyrazolyl, thienyl, indolyl, imidazolyl, oxazolyl, isoxazolyl, thiazolyl, furyl, oxadiazolyl, thiadiazolyl, quinolyl, isoquinolyl, benzothiazolyl, benzoxazolyl, indazolyl, quinoxalyl, quinazolyl, 5,6,7,8-tetrahydroisoquinolinyl benzofuranyl, benzimidazolyl, thianaphthenyl, pyrrolo[2,3-b]pyridinyl, quinazolinyl-4(3H)-one, triazolyl, 4,5,6,7-tetrahydro-1H-indazolyl, and 3b,4,4a,5-tetrahydro-1H-cyclopropa[3,4]cyclopenta[1,2-c]pyrazolyl.

The term "heterocyclyl" or "heterocycle" as used herein refers to a single saturated or partially unsaturated 4 to 8 membered ring that has at least one atom other than carbon in the ring, wherein the atom is selected from oxygen, nitrogen and sulfur; the term also includes multiple condensed ring systems that have at least one such saturated or partially unsaturated ring, which multiple condensed ring systems have 7 to 12 atoms and are further described below. Thus, the term includes single saturated or partially unsaturated rings (e.g., 3, 4, 5, 6, 7 or 8 membered rings) having about 1 to 7 carbon atoms and about 1 to 4 heteroatoms selected from oxygen, nitrogen and sulfur in the ring. The ring may be substituted with one or more (e.g., 1, 2 or 3) oxo groups and the sulfur and nitrogen atoms may also be present in their oxidized forms. The rings of the multiple condensed ring system can be connected to each other via condensed, spiro and bridged bonds when allowed by valency requirements. It should be understood that the individual rings of the multiple condensed ring system may be connected in any order relative to one another. It should also be understood that the point of attachment of the multiple condensed ring system (as defined above for heterocyclyl or heterocycle) may be at any position of the multiple condensed ring system. It should also be understood that the point of attachment of the heterocycle or heterocyclic multiple condensed ring system may be at any suitable atom of the heterocyclyl, including carbon and nitrogen atoms. Exemplary heterocyclyls or heterocycles include, but are not limited to aziridinyl, azetidinyl, pyrrolidinyl, piperidinyl, homopiperidinyl, morpholinyl, thiomorpholinyl, piperazinyl, tetrahydrofuranyl, dihydrooxazolyl, tetrahydropyranyl, tetrahydrothiopyranyl, 1,2,3,4-tetrahydroquinolyl, benzoxazinyl, dihydrooxazolyl, chromanyl, 1,2-dihydropyridinyl, 2,3-dihydrobenzofuranyl, 1,3-benzodioxolyl, 1,4-benzodioxanyl, spiro[cyclopropane-1,1'-isoindolinyl]-3'-one, isoindolinyl-1-one, 2-oxa-6-azaspiro[3.3]heptanyl, imidazolidin-2-one-N-methylpiperidine, imidazolidine, pyrazolidine, butyrolactam, valerolactam, imidazolidinone, hydantoin, dioxolane, phthalimide, 1,4-dioxane, thiomorpholine, thiomorpholine-S-oxide, thiomorpholine-S,S-oxide, pyranyl, 3-pyrrolinyl, thiopyranyl, pyronyl, tetrahydrothiophenyl, quinuclidinyl, tropanyl, 2-azaspiro[3.3]heptanyl, (1R,5S)-3-azabicyclo[3.2.1]octanyl, (1s,4s)-2-azabicyclo[2.2.2]octanyl, (1R,4R)-2-oxa-5-azabicyclo[2.2.2]octanyl, and pyrrolidin-2-one.

As used herein, the term "optionally substituted" means that a given structure or group is not substituted, or that a given structure or group is substituted with one or more specific substituents. Unless otherwise stated, optional substitution may occur at any position of the substituted group.

As used herein, the term "solvate" refers to an association complex or complex of one or more solvent molecules and a compound of the present invention. Examples of solvents that form solvates include, but are not limited to, water, isopropanol, ethanol, methanol, DMSO, ethyl acetate, acetic acid, and ethanolamine. The term "hydrate" refers to a complex wherein the solvent molecule is water.

As used herein the term "prodrug" refers to those compounds that readily undergo chemical changes under physiological conditions to provide the compounds of the present invention. Additionally, prodrugs can be converted to the compounds of the present invention by chemical or biochemical methods in an *ex vivo* environment. For example, prodrugs can be slowly converted to the compounds of the present invention when placed in a transdermal patch reservoir with a suitable enzyme or chemical reagent.

Any compound or structure given herein, is also intended to represent unlabeled forms as well as isotopically labeled forms of the compounds. These forms of the compounds may also be referred to as "isotopically enriched analogues". Isotopically labeled compounds have structures depicted by the formulas given herein except that one or more atoms are replaced by an atom having a selected atomic mass or mass number. Examples of isotopes that can be incorporated into compounds of the disclosure include isotopes of hydrogen, carbon, nitrogen, oxygen, phosphorous, fluorine chlorine, and iodine, such as, ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ³⁶Cl, ¹²³I, and ¹²⁵I, respectively. Various isotopically labeled compounds of the present disclosure, for example those into which radioactive isotopes such as ³H, ¹³C, and ¹⁴C are incorporated. Such compounds are synthesized by means well known in the art, for example by employing starting materials in which one or more hydrogens have been replaced with deuterium.

The term "genome" or "genomic DNA" as used herein is referring to the genetic information of a host organism. The genomic DNA comprises the entire genetic material of a cell or an organism, including the DNA of the nucleus (chromosomal DNA), extrachromosomal DNA, and organellar DNA (e.g. of mitochondria). Preferably, the terms "genome" or "genomic DNA" is referring to the chromosomal DNA of the nucleus.

As used herein, in case the term "recombination" is used to specify an organism or cell, e.g. a microorganism, it is used to express that the organism or cell comprises at least one "transgene", "transgenic" or "recombination" polynucleotide, which is usually specified later on.

As used herein, A polynucleotide "exogenous to" an individual organism is a polynucleotide which is introduced into the organism by any means other than by sexual hybridization.

As used herein, the term "promoter" refers to a polynucleotide region that initiates transcription of a coding sequence. Promoters are located near the transcription start sites of genes, on the same strand and upstream on the DNA (towards the 5' region of the sense strand). Some promoters are constitutive as they are active in all circumstances in the cell, while others are regulated becoming active in response to specific stimuli, e.g., an inducible promoter. The term "promoter activity" and its grammatical equivalents as used herein refer to the extent of expression of nucleotide sequence that is operably linked to the promoter whose activity is being measured. Promoter activity can be measured directly by determining the amount of the produced RNA transcript, for example by Northern blot analysis or be measured indirectly by determining the amount of product coded for by the linked nucleic acid sequence, such as a reporter nucleic acid sequence linked to the promoter.

The term "expression cassette" means those constructs in which the polynucleotide sequence to encode the amino acid sequence to be expressed is effectively linked to at least one genetic control element which enables or regulates the expression (i.e. transcription and / or translation) of the same. The expression may be, for example, stable or transient, constitutive or inducible.

As used herein, the term "plasmid" refers to an extrachromosomal element that often carries genes that are not part of the core metabolic mechanism of the cell and is usually in the form of a circular double-stranded DNA molecule. These elements may be autonomously replicating sequence, genome-integrating sequence, phage or nucleotide sequence of any origin, linear, circular or supercoiled single-stranded or double-stranded DNA or RNA. Typically, the plasmid contains an origin of replication functional in host cells (e.g., *E. coli*) and a selectable marker for detecting host cells containing the plasmid.

As used herein, "gene *ecd*A*"* includes gene sequences having about 83%, 84%, 85%, 86%, 87%, 88%, 89%, 90%, 91%, 92%, 93%, 94%, 95%, 96%, 97%, 98%, or 99% sequence identity with the gene sequence having accession number AFT91378.1; preferably, "gene *ecd*A*"* is a gene sequence having about 83%, 84%, 85%, 86%, 87%, 88%, 89%, 90%, 91%, 92%, 93%, 94%, 95%, 96%, 97%, 98%, or 99% sequence identity with the gene sequence having accession number AFT91378.1; and more preferably, "gene *ecd*A*"* is a gene sequence having accesion number AFT91378.1.

As used herein, "gene *ecd*I" includes gene sequences having about 83%, 84%, 85%, 86%, 87%, 88%, 89%, 90%, 91%, 92%, 93%, 94%, 95%, 96%, 97%, 98%, or 99% sequence identity with the gene sequence having accession number AFT91380.1; preferably, "gene *ecd*I" is a gene sequence having about 83%, 84%, 85%, 86%, 87%, 88%, 89%, 90%, 91%, 92%, 93%, 94%, 95%, 96%, 97%, 98%, or 99% sequence identity with the gene sequence having accession number AFT91380.1; and more preferably, "gene *ecd*I" is a gene sequence having accession number AFT91380.1.

As used herein, "gene *ecd*K*"* includes gene sequences having about 83%, 84%, 85%, 86%, 87%, 88%, 89%, 90%, 91%, 92%, 93%, 94%, 95%, 96%, 97%, 98%, or 99% sequence identity with the gene sequence having accession number AFT91382.1; preferably, "gene *ecd*K*"* is a gene sequence having about 83%, 84%, 85%, 86%, 87%, 88%, 89%, 90%, 91%, 92%, 93%, 94%, 95%, 96%, 97%, 98%, or 99% sequence identity with the gene sequence having accession number AFT91382.1; and more preferably, "gene *ecd*K*"* is a gene sequence having accession number AFT91382.1.

As used herein, "gene *hty*E*"* includes gene sequences having about 83%, 84%, 85%, 86%, 87%, 88%, 89%, 90%, 91%, 92%, 93%, 94%, 95%, 96%, 97%, 98%, or 99% sequence identity with the gene sequence having accession number AFT91391.1; and preferably, "gene *htyE* " is a gene sequence having about 83%, 84%, 85%, 86%, 87%, 88%, 89%, 90%, 91%, 92%, 93%, 94%, 95%, 96%, 97%, 98%, or 99% sequence identity with the gene sequence having accession number AFT91391.1. More preferably, "gene *hty*E*"* is the gene sequence having accession number AFT91391.1.

As used herein, "gene *hty*F*"* includes gene sequences having about 83%, 84%, 85%, 86%, 87%, 88%, 89%, 90%, 91%, 92%, 93%, 94%, 95%, 96%, 97%, 98%, or 99% sequence identity with the gene sequence having accession number AFT91399.1; and preferably, "gene *htyF* " is a gene sequence having about 83%, 84%, 85%, 86%, 87%, 88%, 89%, 90%, 91%, 92%, 93%, 94%, 95%, 96%, 97%, 98%, or 99% sequence identity with the gene sequence having accession number AFT91399.1. More preferably, the "gene *hty*F*"* is the gene sequence having accession number AFT91399.1.

As used herein, "gene *ecd*G*"* includes gene sequences having about 83%, 84%, 85%, 86%, 87%, 88%, 89%, 90%, 91%, 92%, 93%, 94%, 95%, 96%, 97%, 98%, or 99% sequence identity with the gene sequence having accession number AFT91379.1; and preferably, "gene *ecd*G*"* is a gene sequence having about 83%, 84%, 85%, 86%, 87%, 88%, 89%, 90%, 91%, 92%, 93%, 94%, 95%, 96%, 97%, 98%, or 99% sequence identity with the gene sequence having accession number AFT91379.1. More preferably, "gene *ecd*G*"* is the gene sequence having accession number AFT91379.1.

As used herein, "gene *ecd*H*"* includes gene sequences having about 83%, 84%, 85%, 86%, 87%, 88%, 89%, 90%, 91%, 92%, 93%, 94%, 95%, 96%, 97%, 98%, or 99% sequence identity with the gene sequence having accession number AFT91389.1; and preferably, "gene *ecd*H*"* is a gene sequence having about 83%, 84%, 85%, 86%, 87%, 88%, 89%, 90%, 91%, 92%, 93%, 94%, 95%, 96%, 97%, 98%, or 99% sequence identity with the gene sequence having accession number AFT91389.1. More preferably, "gene *ecd*H*"* is the gene sequence having accession number AFT91389.1.

As used herein, the term "transformation" refers to the introduction of one or more exogenous polynucleotides into a host cell by using physical or chemical methods.

As used herein, the term "expression" is the process by which the information encoded within a gene is revealed. If the gene encodes a protein, then expression involves both transcription of the DNA into mRNA, the processing of mRNA (if necessary) into a mature mRNA product, and translation of the mature mRNA into protein.

As used herein, the term "host cell" refers to any cell type that is susceptible to transformation, transfection, transduction, and the like, with a nucleic acid construct or plasmid comprising a polynucleotide of the present invention. The term "host cell" encompasses any progeny of a parent cell that is not identical to the parent cell due to mutations that occur during replication. Preferably, the host cell is a microorganism. More preferably, the microorganism is a bacterium, a yeast, a fungus, such as a cell of the class Ascomycetes. The bacteria that can be used as the host cell of the present invention are preferably selected from *Escherichia coli* and *Bacillus subtilis.* Preferably the fungus is selected from the genus *Aspergillus* (such as *Aspergillus japonicus, Aspergillus niger, Aspergillus oryzae, Aspergillus nidulans, Aspergillus fumigatus, Aspergillus aculeatus, Aspergillus caesiellus, Aspergillus candidus, Aspergillus carneus, Aspergillus clavatus, Aspergillus deflectus, Aspergillus fischerianus, Aspergillus flavus, Aspergillus glaucus, Aspergillus ochraceus, Aspergillus parasiticus, Aspergillus penicilloides, Aspergillus restrictus, Aspergillus sojae, Aspergillus tamari, Aspergillus terreus, Aspergillus ustus, Aspergillus versicolor),* and the genus *Penicillium* (such as *Penicillium aurantiogriseum, Penicillium bilaiae, Penicillium camemberti, Penicillium candidum, Penicillium chrysogenum, Penicillium claviforme, Penicillium commune, Penicillium crustosum, Penicillium digitatum, Penicillium expansum, Penicillium funiculosum, Penicillium glabrum, Penicillium glaucum, Penicillium italicum, Penicillium lacussarmientei, Penicillium marneffei, Penicillium purpurogenum, Penicillium roqueforti, Penicillium stoloniferum, Penicillium ulaiense, Penicillium verrucosum, Penicillium viridicatum*)*.* Preferably the yeast is selected from the genera *Saccharomyces, Schizosaccharomyces, Candida* and *Pichia.*

### Pharmaceutical compositions and administration

In one embodiment, the present invention provides a pharmaceutical composition having antifungal infection activity, including a compound represented by general formula (I) or an ester, stereoisomer, prodrug, solvate or pharmaceutically acceptable salt thereof, and a pharmaceutically acceptable carrier.

Pharmaceutically acceptable carriers can be solid or liquid. Among them, the solid carrier may be one or more materials used as excipients, diluents, sweeteners, solubilizers, lubricants, binders, tablet disintegrants, stabilizers, preservatives, or encapsulating materials. The liquid carrier may be solvents or liquid dispersion media. Suitable solid carriers include, but are not limited to, for example, cellulose, glucose, lactose, mannitol, magnesium stearate, magnesium carbonate, saccharin sodium, sucrose, dextrin, talc, starch, pectin, gelatin, tragacanth, arabic gum, sodium alginate, parabens, methylcellulose, sodium carboxymethyl cellulose, a low-melting point wax, cocoa butter, and the like. Suitable liquid carriers include, but are not limited to, water, ethanol, polylol (such as glycerol, propanediol, liquid polyethylene glycol, etc.), a vegetable oil(such as peanut oil, cottonseed oil, safflower oil, sesame oil, olive oil, corn oil, and soybean oil), glyceride, agar, water free of pyrogen, isotonic saline, Ringer's solution and a mixture thereof.

The pharmaceutical composition according to the present invention may be prepared by a known method, including conventional blending, granulating, tableting, coating, dissolving, or lyophilization processes.

In one embodiment, the pharmaceutical composition of the present invention may be administered to a patient or subject in need of treatment by any suitable route of administration, including oral administration, parenteral administration (including subcutaneous, intramuscular, intravenous, and intradermal administration), inhalation spray administration, topical administration, rectal administration, nasal administration, cervicobuccal administration, vaginal administration or administration via an implantable reservoir.

The orally administered composition in the present invention includes a solid dosage form such as pill, tablet, caplet, capsule (including immediate release, timed release and sustained release formulations), granule, and powder; or a liquid dosage form such as solution, syrup, elixir, emulsion, and suspension. Sterile solutions or ocular drug delivery devices are intended for ocular administration. Sterile solutions, emulsions and suspensions are intended for parenteral administration.

### Indications and treatments

The compound of the present invention or its ester, stereoisomer or pharmaceutically acceptable salt and the pharmaceutical composition comprising the compound of the present invention or its ester, stereoisomer or pharmaceutically acceptable salt can be used for treating and/or preventing diseases caused by fungal infection. Therefore, the present invention also relates to a method for treating and/or preventing diseases caused by fungal infection, comprising administering a therapeutically effective amount of a compound represented by formula (I) or its ester, stereoisomer, prodrug, solvate or pharmaceutically acceptable salt of the present invention to a patient in need.

Diseases caused by fungal infections include, but are not limited to, one or more of scalp tinea, tinea corporis, tinea pedis, onychomycosis, perionychomycosis, tinea versicolor, thrush, vaginal candidiasis, respiratory tract candidiasis, biliary tract candidiasis, esophageal candidiasis, urethral candidiasis, systemic candidiasis, mucosal and skin candidiasis, aspergillosis, mucormycosis, paracoccidioidomycosis, North American blastomycosis, histoplasmosis, coccidioidomycosis, sporotrichosis, fungal sinusitis and chronic sinusitis.

The dosage depends on various factors including the age, weight and condition of a patient and the route of administration. The exact dosage to be administered is left to the discretion of the attending physician. The actual dose levels and time frame of the active ingredient of the pharmaceutical composition of the present invention may be varied to obtain an amount of the active ingredient that, for a particular patient, composition and route of administration, may induce the desired therapeutic response without posing toxicity to the patient. Typically, the pharmaceutical composition of the present invention is administered at a dose sufficient to reduce or eliminate symptoms associated with fungal infection.

The preferred dose of the agent is the maximum dose that a patient can tolerate and that does not produce serious or unacceptable side effects. Exemplary dose ranges include 0.01 to 250 mg/day, 0.01 to 100 mg/day, 1 to 100 mg/day, 10 to 100 mg/day, 1 to 10 mg/day, and 0.01 to 10 mg/day.

The preferred dose of the agent is the maximum dose that a patient can tolerate and that does not produce serious or unacceptable side effects. In examples, the medicament is administered at a dose of about 0.01 to about 100 mg/kg bw/day, about 0.1 to about 10 mg/kg bw/day, or about 1.0 to about 10 mg/kg bw/day.

In one embodiment, a therapeutically effective dose results in a serum medicament concentration from about 0.1 ng/mL to about 50-100 mg/mL. Typically, these pharmaceutical compositions should be administrated at a dose of about 0.001 to about 2000 mg/kg bw/day. For example, the range of the dose for systemic administration to a human patient may be 1-10 mg/kg, 20-80 mg/kg, 5-50 mg/kg, 75-150 mg/kg, 100-500 mg/kg, 250-750 mg/kg, 500-1000 mg/kg, 1-10 mg/kg, 5-50 mg/kg, 25-75 mg/kg, 50-100 mg/kg, 100-250 mg/kg, 50-100 mg/kg, 250-500 mg/kg, 500-750 mg/kg, 750-1000 mg/kg, 1000-1500 mg/kg, 10 1500-2000 mg/kg, 5 mg/kg, 20 mg/kg, 50 mg/kg, 100 mg/kg, 500 mg/kg, 1000 mg/kg, 1500 mg/kg, or 2000 mg/kg. Pharmaceutical unit dosage forms are prepared to provide about 1 to about 5000 mg (e.g., about 100 to about 2500 mg) of the compound or combination of essential ingredients per unit dosage form. Preferred unit dose formulations are those containing a daily dose or unit, a daily sub-dose, or an appropriate fraction thereof, as discussed herein, of a given ingredient.

The present invention is further illustrated by the following specific examples, which are not intended to limit the present invention. Many modifications and adjustments may be made by those skilled in the art in light of the above teachings without departing from the spirit and scope of the present invention.

### Examples

### Strains:

The strains used in the present invention are listed in Table 1. Among them, by knocking out eight most highly expressed biosynthetic gene clusters (BGCs) genes, the triple auxotrophic chassis strain *Aspergillus nidulans* LO8030 (*pyrG-, riboB-, pyroA-*) has a clean metabolic background and low demand for precursors *(see* Chiang YM et al., (2013) An efficient system for heterologous expression of secondary metabolite genes in Aspergillus nidulans, Journal of the American Chemical Society 135, 7720-7731). Considering the large number of genes that need to be transferred and the huge gene fragments encoding core biosynthetic enzymes, one of the challenges of heterologous expression of fungal BGCs is to assemble and transfer the BGC genes into heterologous chassis strains.

### Culture medium:

### Solid Glucose Minimum Medium (GMM)

Formulation: each liter of culture medium contains 10 g glucose, 6 g NaNO₃, 0.52 g KCl, 0.52 g MgSO₄·7H₂O, 1.52 g KH₂PO₄, 0.022 g ZnSO₄·7H₂O, 0.011 g H₃BO₃, 0.005 g MnCl₂·4H₂O, 0.0016 g FeSO₄·7H₂O, 0.0016 g CoCl₂·5H₂O, 0.0016 g CuSO₄·5H₂O, 0.0011 g (NH₄)₆Mo₇O₂₄·4H₂O, 0.05 g Na₄EDTA, 16 g agar powder, and NaOH (adjusting pH to 6.5).

Preparation process:
1L 20×Nitrate Salts was prepared, containing 120 g NaNO₃, 10.4 g KCl, 10.4 g MgSO₄·7H₂O, and 30.4 g KH₂PO₄;
100 mL of Trace Elements was prepared, containing 2.2 g ZnSO₄·7H₂O, 1.1 g H₃BO₃, 0.5 g MnCl₂·4H₂O, 0.16 g FeSO₄·7H₂O, 0.16 g CoCl₂·5H₂O, 0.16 g CuSO₄·5H₂O, 0.11 g (NH₄)₆Mo₇O_{24·}4H₂O, and 5 g Na₄EDTA;
Then, for each liter of GMM liquid medium, a solid glucose minimum medium was obtained by taking 50 mL of 20× Nitrate Salts, 1 mL of Trace Elements, 10.0 g of glucose, and 16.0 g of agar powder, and by adding 800 mL of ddH₂O, adjusting the pH to 6.5 with NaOH, making up to 1 liter, with autoclave at 121°C for 20 minutes, further cooling to 60°C, dispensing into plates, and cooling and solidify at room temperature.

Liquid glucose minimum medium:
Formulation: each liter of culture medium contains 10 g glucose, 6 g NaNO₃, 0.52 g KCl, 0.52 g MgSO₄·7H₂O, 1.52 g KH₂PO₄, 0.022 g ZnSO₄·7H₂O, 0.011 g H₃BO₃, 0.005 g MnCl₂·4H₂O, 0.0016 g FeSO₄·7H₂O, 0.0016 g CoCl₂·5H₂O, 0.0016 g CuSO₄·5H₂O, 0.0011 g (NH₄)₆Mo₇O₂₄·4H₂O, 0.05 g Na₄EDTA, and NaOH (adjusting pH to 6.5).

Preparation process:
1L 20×Nitrate Salts was prepared, containing 120 g NaNO₃, 10.4 g KCl, 10.4 g MgSO₄·7H₂O, and 30.4 g KH₂PO₄;
100 mL of Trace Elements was prepared, containing 2.2 g ZnSO₄·7H₂O, 1.1 g H₃BO₃, 0.5 g MnCl₂·4H₂O, 0.16 g FeSO₄·7H₂O, 0.16 g CoCl₂·5H₂O, 0.16 g CuSO₄·5H₂O, 0.11 g (NH₄)₆Mo₇O₂₄·4H₂O, and 5 g Na₄EDTA;
Then, for each liter of GMM liquid medium, a liquid glucose minimum medium was obtained by taking 50 mL of 20× Nitrate Salts, 1 mL of Trace Elements, 10.0 g of glucose, and by adding 800 mL of ddH₂O, adjusting the pH to 6.5 with NaOH, making up to 1 liter, with autoclave at 121°C for 20 minutes, further cooling to room temperature.

### Culture conditions:

*Emericella rugulosa* NRRL 11440 and *Aspergillus nidulans* LO8030 strains were stored as glycerol stocks at -80°C and activated in solid glucose minimum medium (GMM) at 37°C *(see* Shimizu et al. (2001) Genetic involvement of a cAMP-dependent protein kinase in a G protein signaling pathway regulating morphological and chemical transitions in Aspergillus nidulans, Genetics 157, 591-600), supplementing for nutritional deficiencies when necessary. For the *pyr* G auxotrophic type, 0.56 g/L uracil and 1.26 g/L uridine were supplemented in the GMM; for the *pyro* A auxotrophic type, 1 mg/L pyridoxine hydrochloride was supplemented; for the *ribo* B auxotrophic type, 2.5 mg/L riboflavin was supplemented. *Saccharomyces cerevisiae* BJ5464 was used for yeast homologous recombination plasmid construction *(see* Yin WB, et al., (2013). Discovery of cryptic polyketide metabolites from dermatophytes using heterologous expression in Aspergillus nidulans, ACS Synth Biol 2, 629-634.), and *Escherichia coli* DH5α was used for plasmid amplification.

**Table 1 Strains used in the present invention**

| Strains | Notes | Purpose | Source |
|---|---|---|---|
| *Emericella rugulosa* NRRL 11440 | Wild type | obtain *Ecd*/*Hty* BGC gene | ATCC, USA |
| *Escherichia coli* DH5α | | Plasmid cloning and storage | laboratory |
| *Saccharomyces cerevisiae* BJ5464 | MATalpha, *ura* 3-52, *trp* 1, leu2-Δ1, *his* 3-Δ200, pep4::HIS 3, *prb* 1-Δ1.6R, *can* 1, GAL | Plasmid construction | Wen-Bing Yin et al.*¹* |
| *Aspergillus nidulans* LO8030 ^{a} | *pyr* G89, *pyro* A4*, ribo* B2*, nkuA::argB,* sterigmatocystin cluster (AN7804-AN7825)Δ, emericellamide cluster (AN2545-AN2549)Δ, asperfuranone cluster (AN1039-AN1029)Δ, monodictyphenone cluster (AN10023-AN10021)Δ, terrequinone cluster (AN8512-AN8520) Δ, austinol cluster (AN8379-AN8384, AN9246-AN9259) Δ, F9775 cluster (AN7906-AN7915), asperthecin cluster (AN6000-AN6002) Δ. | Chassis strain | Yi-Ming Chiang et al.*²* |
| LO8030-1.1/1.3/1.6 ^{b} | *pyr* G89, *pyro* A4*, ΔyA::* (*xylp-ecdA* 1 - 2 - *AfriboB)* | transformant strains of the first round | This study |
| LO8030-2.2 /2.4/2.5 ^{b} | *pyro A4, ribo B2, ΔyA::* (*xylp-ecdA* 1 - 2 *-ecdA 3 -AppyrG -gpdAp-ecdI*) | transformant strains of the second round | This study |
| LO8030-4.1/4.2/4.3 ^{b} | *pyr* G89, *pyro* A4*, ΔyA::* (*xylp-ecdA-gpdAp-ecdI-AfriboB*) | transformant strains of the third round | This study |
| LO8030-5.1/5.2/5.3/5.4/5.5/5.6^{b} | *pyro A4, ΔyA::* (*xylp-ecdA-gpdAp-ecdI-AfriboB),* pYX10 | transformant strains of the fourth round | This study |
| LO8030-4.2.1^{b} | *pyro A4, ΔyA::* (*xylp-ecdA-gpdAp-ecdI-AfriboB),* pJQ10 | transformant strains of the fourth round | This study |
| LO8030-4C.1 | *pyr* G89, *pyro* A4, *ΔyA::* (*xylp-gpdAp-AfriboB*) | Control of transformant strains of the third round, without biosynthetic genes | This study |
| LO8030-5C.1 | *pyro A4, ΔyA:: (xylp-gpdAp-AfriboB),* pYX12 | Control of transformant strains of the fourth round, without biosynthetic genes | This study |
| LO8030-4.2.1.5 | *pyro A4, ΔyA::(xylp-ecdA-gpdAp-ecdI-AfriboB),* pJQ10, pJQ5 | transformant strains of the fifth round | This study |
| LO8030-4.2.19 | *pyro A4, ΔyA:* :(*xylp-ecdA-gpdAp-ecdI-AfriboB),* pJQ10, pJQ9 | transformant strains of the fifth round | This study |

| | | | |
|---|---|---|---|
| ^{a} Description of the *Aspergillus nidulans* LO8030 transformant strains, with omitting the genotype portion identical to LO8030. ^{b} 1.1/1.3/1.6 represent three parallel transformant strains generated in the first round of *Aspergillus* transformation, and the three transformants have the same genotype; 2.2/2.4/2.5, 4.1/4.2/4.3 and 5.1/5.2/5.3/5.4/5.5/5.6 represent different parallel transformant strains generated in the second, third and fourth rounds of transformation, respectively. ¹ Yin, WB, Chooi, YH, Smith, AR, Cacho, RA, Hu, Y, White, TC, and Tang, Y. (2013) Discovery of cryptic polyketide metabolites from dermatophytes using heterologous expression in Aspergillus nidulans, ACS Synth Biol 2, 629-634. ² Chiang, YM, Ahuja, M., Oakley, CE, Entwistle, R., Asokan, A., Zutz, C., Wang, CC, and Oakley, BR (2016) Development of Genetic Dereplication Strains in Aspergillus nidulans Results in the Discovery of Aspercryptin, Angewandte Chemie (International ed. in English) 55, 1662-1665. | | | |

### Example 1. Plasmid construction

To extract genomic DNA (gDNA), *Emericella rugulosa* NRRL 11440 and *Aspergillus nidulans* LO8030 were cultivated in liquid GMM at 37°C for 24 h and supplemented for auxotrophs as necessary. gDNA was extracted from *Emericella rugulosa* NRRL 11440 according to the standard protocol *(see* Sambrook, J., and Russell, DW (2001) Molecular cloning: a laboratory manual, 3rd ed., Cold Spring Harbor Laboratory Press, Cold Spring Harbor, NY). Then, the gDNA of *Emericella rugulosa* NRRL 11440 was used as a template and high-fidelity DNA polymerase Primestar HS/MAX (Takara, Japan) was used to clone the echinocandin biosynthetic gene cluster (BGC) genes (as shown in Table 3) by PCR (PCR operation program is shown in Table 2). The nonribosomal polypeptide synthetase (NRPS) gene *ecdA* of the echinocandin biosynthetic gene cluster (BGC) was divided into four fragments *ecdA* 1-4, each of which was approximately 6-7 kb, for PCR cloning, wherein the *ecdA* 1 fragment contained base pairs 1 to 5831, the *ecdA* 2 fragment contained base pairs 5648 to 11563, the *ecdA* 3 fragment contained base pairs 10591 to 16084, and the *ecdA* 4 fragment contained base pairs 14938 to 21910. All PCR primers used in the present invention are shown in Table 4.

**Table 2 PCR operation program:**

| | | | |
|---|---|---|---|
| Pre-denaturation | | 98° C | 1 min |
| 30× Cycle | Denaturation | 98° C | 10s |
| | annealing | 60° C | 30s |
| | extension | 72° C | 5s/kb |
| Thermal insulation | | 4° C | |

**Table 3 Genes of the echinocandin biosynthetic gene cluster**

| Gene | Length (bp) | Function | Accession Number |
|---|---|---|---|
| *ecdA* | 21910 | NRPS (TCATCATCATCATCATCATC)* | AFT91378.1 |
| *ecd*I | 1924 | Fatty acyl AMP ligase | AFT91380.1 |
| *ecdK* | 1119 | Dioxygenase, involved in the cyclization of L-leucine to 4R -Me-L-proline | AFT91382.1 |
| *htyE* | 1104 | Oxygenase, involved in the hydroxylation of L-proline C₄ and 4R -Me-L-proline C₃ | AFT91391.1 |
| *ecdG* | | Monooxygenase, responsible for the hydroxylation of L-homotyrosine at the C-3 position | AFT91379.1 |
| *HtyF* | | Monooxygenase, responsible for the hydroxylation of L-homotyrosine at the C-4 position | AFT91399.1 |
| *EcdH* | | Dioxygenase, responsible for the hydroxylation of L-ornithine at C-4 and C-5 positions | AFT91389.1 |

| | | | |
|---|---|---|---|
| *NRPS domains: T represents thiolation, C represents condensation, and A represents adenylylation. | | | |

**Table 4. PCR primers**

| Name | Sequence (5'-3') ^{a} | Purpose |
|---|---|---|
| ecdA1-F | ATGCACAGAACCAACGAGATGG | ecdA1 amplification |
| ecdA1-R | TCGCTGCAGGTGGAGTGAATG | |
| ecdA2-F | CGTTTGCGACCCTTACCTCTAC | ecdA2 amplification |
| ecdA2-R | TGGCCTGCTCCTCAACCATTTC | |
| pYX1-Ribo-yAdn-F | AACAATGTGTGCATGAAATGGTTGAGGAGCAGGCCAGCGGACT GAGTTATGGATGAC | Amplification of the Ribo and yA downstream fragments of pYX1 |
| pYX1-Ribo-yAdn-R | AGGAACCGAAATACATACATTGTCTTCCGTAAAGCCCCAGTCAC GACGTTGTAAAAC | |
| pYX1-AscI-F | GCTTTACGGAAGACAATG | Amplification of pYX1 plasmid |
| pYX1-AscI-R | CAATTCCACACAACATACGAGCCGGAAGCATAAAGAATTCGGG | |
| | TGCCTAATGAGTGAG | backbone |
| pYX1-YAup-F | TTTATGCTTCCGGCTCG | Amplification of the yA upstream fragment yA-up of pYX1 |
| pYX1-YAup-R | ATAAGTATACTCTATTGACCTATAGGACCTGAGTGATGCTCGGCT TGATGTTGTTCG | |
| pYX1-xylp-F | GCATCACTCAGGTCCTATAGG | Amplification of xylp of pYX1 |
| pYX1-xylp-R | CAGTTGGTTCTTCGAGTCGATG | |
| ecdA3-F | ACTCATTAGGCACCCAAATATGCGGCCGCTATAAAGAACAGTTC TTCGTATGGACG | ecdA3 amplification |
| ecdA3-R | CTTTCGTTGGATCTGGTATTC | |
| pYX5-pyrG-F | CGAGGTCGGTGAGGTTGAATACCAGATCCAACGAAAGAGCTTA TACGAACAGATGG | Amplification of AppyrG of pYX5 |
| pYX5-pyrG-R | CAAATAGAATTAAATGGTACTCGAGTCCACTGAAGGTGCCCAAC GCATTCTTCATG | |
| pYX5-gpdA-F | CACCTTCAGTGGACTCGAGTAC | Amplification of gpdAp of pYX5 |
| pYX5-gpdA-R | CCAGTTGGCTGCACCATGCTGGGGAAGTGAAGACCATTGTGAT GTCTGCTCAAGCG | |
| ecdI-F | ATGGTCTTCACTTCCCCAGCATG | ecdI amplification |
| ecdI-R | CCAAGGCGCATGTACTAGTCAAG | |
| pYX5-**yA-down-F** | GTATCATAACAATCTTGACTAGTACATGCGCCTTGGGGGTTGA TGTTTGGGATTC | Amplification of the yA downstream fragment yA-down of pYX5 |
| pYX5-**yA-down-R** | ACATTGTCTTCCGTAAAGCTTTATAGCGGCCGCATATTTCCTCGG TTCAGTCTTACTAG | |
| pYX5-AscI-F | *AAATATGCGGCCGCTATAAA*GCTTTACGGAAGACAATG | Amplification of pYX5 plasmid backbone |
| pYX5-AscI-R | *TTTATAUCUUCCUCATATTT*GGGTGCCTAATGAGTGAG | |
| ecdA4-F | GACCTTGCTGCCTCATTTGC | ecdA4 amplification |
| ecdA4-R | GGCTCCCTTTATGCTCTTCG | |
| pYX9-riboB-F | AGTCAGACAATGTTCCGAAGAGCATAAAGGAGCCGCGGACT GAGTTATGGATGAC | Amplification of AffiboB |
| pYX9-riboB-R | CAACAAGTGCCACTCAACGC | |
| pYX9-gpdA-F | TCACTGAGTCAATGGCGTTGAGTGGCACTTGTTGCACCTTCAG TGGACTCGAGTAC | gpdAp amplification |
| pYX9-gpdA-R | ATTGTCTTCCGTAAAGCTTTATAGCGGCCGCATATTTTGTGATGT CTGCTCAAGCG | |
| pYX9-Ascl-F | *AAATATGCGGCCGCTATAAA*GCTTTACGGAAGACAATG | Amplification of pYX9 plasmid backbone |
| pYX9-AscI-R | AGCAAATGAGGCAGCAAGGTCTTTATAGCGGCCGCATATTTGG[?] TGCCTAATGAGTGAG | |
| pYX14-yA-up -F | ACTCATTAGGCACCCAAATATGCGGCCGCTATAAATTTATGCTT CCGGCTCGTAT | Amplification of the yA upstream fragment yA-up of pYX14 |
| pYX14-yA-up -R | ATAAGTATACTCTATTGACCTATAGGACCTCGGCTTGATGTTGTT CG | |
| pYX14-xyl-F | ACGGAAGCGCGCAGTCGGCG | Amplification of xylp of pYX14 |
| pYX14-xyl-R | GAAAGGGAGTCATCCATAACTCAGTCCGCAGTTGGTTCTTCG[?] GTCGATG | |
| pYX14-riboB-F | GCGGACTGAGTTATGGATGAC | Amplification of AfriboB of pYX14 |
| pYX14-riboB-R | CAACAAGTGCCACTCAACGC | |
| pYX14-gpdA-F | GAGTCAATGGCGTTGAGTGGCACTTGTTGCACCTTCAGTGGA CTCGAGTAC | Amplification of gpdAp of pYX14 |
| pYX 14-gpdA-R | TGTGATGTCTGCTCAAGCG | |
| pYX14-yA-down-F | ACAGCTACCCCGCTTGAGCAGACATCACAGGGGTTGATGTTT[?] GGATTC | Amplification of the yA upstream fragment yA-down of pYX14 |
| pYX14-yA-down-R | TGTCTTCCGTAAAGCTTTATAGCGGCCGCATATTTCCTCGGTTC AGTCTTACTAG | |
| pYX14-Ascl-F | AAATATGCGGCCGCTATAAAGCTTTACGGAAGACAATGTA | Amplification of pYX14 plasmid backbone |
| pYX14-AscI-R | TTTATAGCGGCCGCATATTTGGGTGCCTAATGAGTGAGCT | |
| pYX10-amyB-F | CCATCATGGTGTTTTGATC | Amplification of amyBp of pYX10 |
| pYX10-amyB-R | AATTCGGAGCTTGCTGTGG | |
| pYX10-htyE-F | TTCTCTGAACAATAAACCCCACAGCAAGCTCCGAATTATGGCTA TCACTACGCTAG | Amplification of htyE |
| pYX10-htyE-R | TGTCAACTACGACTGTCATG | |
| pYX10-gpdA-F | CTCATATTCATATTCATGACAGTCGTAGTTGACACACCTTCAGT GGACTCGAGTAC | Amplification of gpdAp of pYX10 |
| pYX10-gpdA-R | TGTGATGTCTGCTCAAGCG | |
| pYX10-ecdK-F | CTTGACTAACAGCTACCCCGCTTGAGCAGACATCACAATGTCT GTTCTAACTCTCG | Amplification of ecdK |
| pYX10-ecdK-R | GGAATAGTCCTCTCGGGCCATCTGTTCGTATAAGCTGATAAACA GCAGGTGACATG | |
| pYX10-pyrG-F | AGCTTATACGAACAGATGG | Amplification of |
| pYX10-pyrG-R | ATACAAAAAATAAGCTGGCTTTCCCCGTCAAGCTCTAACCCAAC GCATTCTTCATG | AppyrG of pYX10 |
| pYX10-AMA1-F | TTAGAGCTTGACGGGGAAAGC | Amplification of AMA1 of pYX10 |
| pYX10-AMA1-R | CCAGGAACCGAAATACATACATTGTCTTCCGTAAAGCACTCTAG AGGATCCTGCAG | |
| pYX10-Ascl-F | GCTTTACGGAAGACAATG | Amplification of pYX10 plasmid backbone |
| pYX10-AscI-R | CCATATAAAAATTTAAAATGATCAAAACACCATGATGGGGGTGCC TAATGAGTGAG | |
| pYX12-gpdA-F | TCTGAACAATAAACCCCACAGCAAGCTCCGAATTCACCTTCAG TGGACTCGAGTAC | Amplification of gpdAp of pYX12 |
| pYX12-gpdA-R | CGGAATAGTCCTCTCGGGCCATCTGTTCGTATAAGCTTGTGATG TCTGCTCAAGCG | |
| pJQ10-pyrG/AMA1-F | AGCTTATACGAACAGATGG | Amplification of the pyrG-AMA1 fragment of |
| pJQ10-pyrG/AMA1-R | CCAGGAACCGAAATACATACATTGTCTTCCGTAAAGCACTCTAG AGGATCCTGCAG | |
| pJQ10-Ascl-F | GCTTTACGGAAGACAATG | Amplification of pJQ10 plasmid backbone |
| pJQ10-AscI-R | GGGTGCCTAATGAGTGAG | |
| pJQ10-amyBp/htyE-F | CGCAATTAATGTGAGTTAGCTCACTCATTAGGCACCCCCATCAT GGTGTTTTGATC | Amplification of the amyBp-htyE fragment of pJQ10 |
| pJQ10-amyBp/htyE-R | CGGAATAGTCCTCTCGGGCCATCTGTTCGTATAAGCTTGTCAAC TACGACTGTCATG | |
| pJQ5*-pyroA4*/*AMA1-*F | GCATCCACATGATCGACAGC | Amplification of fragments *pyroA4* and *AMA1* of pJQ5 |
| pJQ5*-pyroA4*/*AMA1-*R | CCAGGAACCGAAATACATACATTGTCTTCCGTAAAGCACTCTAG AGGATCCTGCAG | |
| pJQ5-Ascl-F | GCTTTACGGAAGACAATG | Amplification of pJQ5 plasmid backbone |
| pJQ5-AscI-R | GGGTGCCTAATGAGTGAG | |
| pJQ5*-amyBp*/*ecdG-*F | CGCAATTAATGTGAGTTAGCTCACTCATTAGGCACCCCCATCAT GGTGTTTTGATC | Amplification of fragments *amyBp* and *ecdG* of pJQ5 |
| pJQ5*-amyBp*/*ecdG-*R | CACTAACACTTGTTTGGCTGTCGATCATGTGGATGCGGATTTA CTGGATCTGTCA | |
| pJQ9*-pyroA4*/*AMA1-*F | GCATCCACATGATCGACAGC | Amplification of fragments *pyroA4* and *AMA1* of pJQ9 |
| pJQ9-*pyroA4*/*AMA1-*R | CCAGGAACCGAAATACATACATTGTCTTCCGTAAAGCACTCTAG AGGATCCTGCAG | |
| pJQ9-AscI-F | GCTTTACGGAAGACAATG | Amplification of pJQ9 plasmid backbone |
| pJQ9-AscI-R | GGGTGCCTAATGAGTGAG | |
| pJQ9*-amyBp*/*ecdG-*F | CGCAATTAATGTGAGTTAGCTCACTCATTAGGCACCCCCATCAT GGTGTTTTGATC | Amplification of fragments *amyBp* and *ecdG* of pJQ9 |
| pJQ9*-amyBp*/*ecdG-*R | GGATTTACTGGATCTGTCA | |
| pJQ9*-gpdAp*/*htyF-*F | TTCTTGCCTGAGATGAGCTGACAGATCCAGTAAATCCCACCTTC | Amplification of |
| | AGTGGACTCGAGTAC | fragments *gpdAp* and *htyF* of pJQ9 |
| *pJQ9-gpdAp*/*htyF-R* | CACTAACACTTGTTTTGGCTGTCGATCATGTGGATGCACGACC AAATAGCACACAGC | |
| RT-ecdA-F | TTCTCCGGGTCTTCTTGTC | |
| RT-ecdA-R | TTCTTCCCATTCTGGTGTC | |
| RT-ecdI-F | TCTTTGGCATTAACGACG | |
| RT-ecdI-R | CTGTGACCCTTGGGAATAC | |
| RT-ecdK-F | ATCTGCCTGTGCGGTTTG | Semiquantitative RT-PCR detection |
| RT-ecdK-R | ATCCTGGAACGATGGACG | |
| RT-htyE-F | TTTGTTCGGCGAATCATCG | |
| RT-htyE-R | GCAACGCAGTCAAATGGTC | |
| RT-H2B-F | CTGCCGAGAAGAAGCCTAGCAC | |
| RT-H2B-R | GAAGAGTAGGTCTCCTTCCTGGTC | |

| | | |
|---|---|---|
| ^{a} The bases in italics are the homologous region sequences used for yeast homologous recombination. | | |

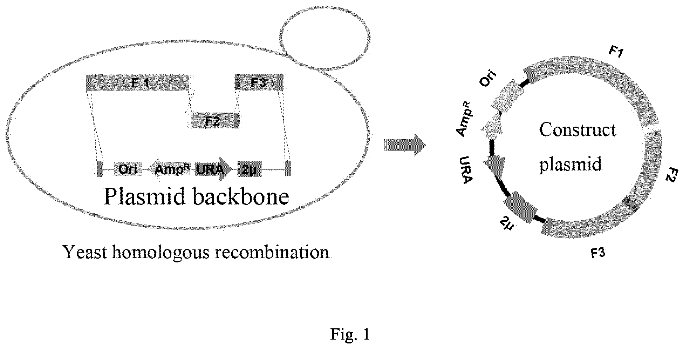

According to Yin et al. (Discovery of cryptic polyketide metabolites from dermatophytes using heterologous expression in Aspergillus nidulans, ACS Synth Biol (2013) 2, 629-634.), a plasmid was constructed in *Saccharomyces cerevisiae* BJ5464 by yeast homologous recombination (Fig. 1). The constructed plasmid can produce a gene expression cassette for *Aspergillus* transformation (as shown in Table 5). The plasmid backbone containing the *Escherichia coli* origin of replication (Ori) and ampicillin resistance (Amp^{R}) (GenBank: M77789), the yeast replication origin 2µ (2 micron/2 micron ori/2 mu) and the uracil auxotrophic selection marker URA (GenBank: X75459) was derived from the plasmid pYH-yA-riboA (Wang, PM et al. (2016). " TrpE feedback mutants reveal roadblocks and conduits toward increasing secondary metabolism in Aspergillus fumigatus. " Fungal genetics and biology 89: 102-113.).

**Table 5. Constructed plasmids**

| Name | Notes | Purpose |
|---|---|---|
| pYX1 | *xylp-ecdA*1*-*2*-AfriboB* | Contains the expression cassette for the first round of transformation |
| pYX5 | *ecdA3-AppyrG-gpdAp-ecdI* | Contains the expression cassette for the second round of transformation |
| pYX9 | *ecdA*4*-AfriboB-gpdAp* | Contains the expression cassette for the third round of transformation |
| pYX10 | *amyBp-htyE-gpdAp-ecdK-AppyrG, AMA1* | Heterologous expression of oxygenase genes *htyE* and *ecdK* |
| pYX14 | *xylp-AfriboB-gpdAp,* | Contains expression cassette for control transformant strains |
| pYX12 | *amyBp-gpdAp-AppyrG, AMA1* | pYX10 control |
| pJQ10 | *amyBp-htyE-AppyrG, AMA1* | Heterologous expression of oxygenase gene *htyE* |
| pJQ5 | *amyBp-ecdG-pyroA4, AMA1* | Heterologous expression of oxygenase gene *ecdG* |
| pJQ9 | *amyBp-ecdG-gpdAp-htyF-pyroA4, AMA1* | Heterologous expression of oxygenase genes *ecdG* and *htyF* |

### Example 2: Preparation of Aspergillus nidulans gene transformant strain LO8030-5.1

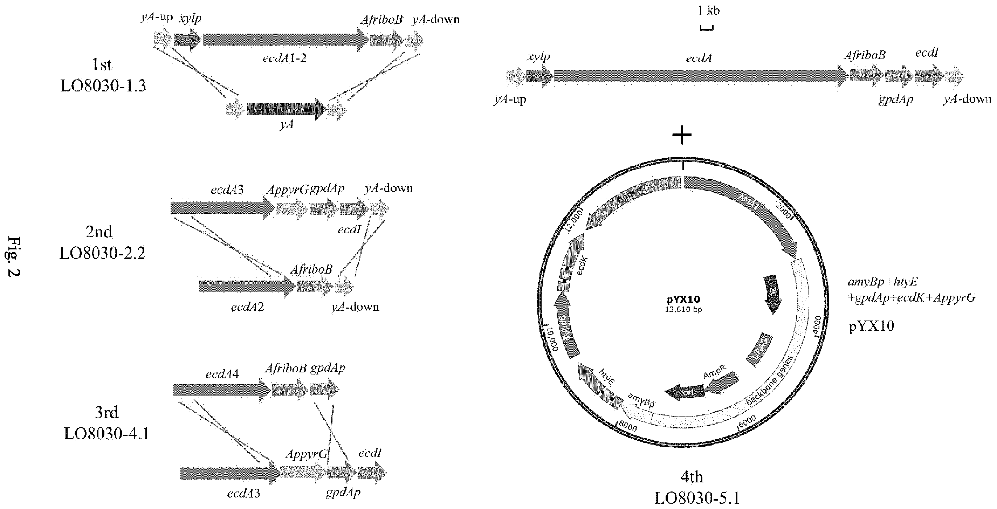

Some fragments of the plasmid were initially connected by double-joint PCR (DJ-PCR) (Yu et al. (2004) Double-joint PCR: a PCR-based molecular tool for gene manipulations in filamentous fungi, Fungal genetics and biology : FG & B 41, 973-981.), and then the plasmid was constructed by yeast homologous recombination to obtain the gene expression cassette. Through three rounds of transformation, the heterologous gene expression cassettes of *ecdA* and *ecdI* were integrated into the *yA* gene site of *Aspergillus nidulans* LO8030, as shown in Fig. 2.

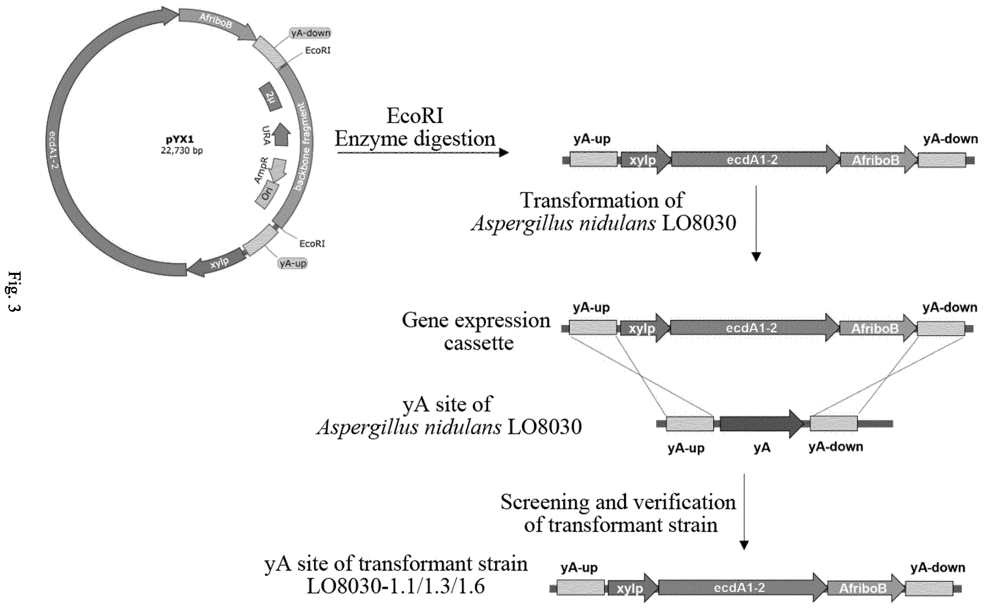

Specifically, the gene expression cassette used for the first round of transformation was from plasmid *pYX1* (Fig. 3), which contains the xylose-inducible promoter *xylp* (GenBank: HM640258) from pSK plasmid (Hartmann, T. et al. (2010). "Validation of a self-excising marker in the human pathogen Aspergillus fumigatus by employing the beta-rec/six site-specific recombination system." Appl Environ Microbiol 76 (18): 6313-6317.), the first and second fragments of *ecd*A*, ecd*A1-2*,* and the auxotrophic marker gene *ribo*B (*AfriboB*) from *Aspergillus fumigatus* (Nayak, T. et al. (2006). "A versatile and efficient gene-targeting system for Aspergillus nidulans." Genetics 172 (3): 1557-1566.). On both sides of the gene cassette are DNA fragments yA-up and yA-down, about 1 kb upstream and downstream of gene yA (O'Hara, EB and W. Timberlake (1989). "Molecular characterization of the Aspergillus nidulans yA locus." Genetics 121 (2): 249-254.). First, *ecd*A1 and *ecd*A2 fragments were connected by double-joint PCR to obtain *ecd*A1-2. The templates and primers for PCR cloning of each fragment are shown in Table 6, and the specific primer sequences used are shown in Table 4. After PCR amplification of each fragment (Table 6), after gel tapping and purification, each fragment was mixed and transformed into yeast BJ5464. After obtaining yeast transformants, yeast plasmids were extracted using a yeast plasmid extraction kit (OMEGA Bio-tek), and the plasmids were transformed into competent *Escherichia coli* for plasmid amplification and PCR detection. By detecting the interface of each fragment with primer pairs, the target fragment of the expected size was obtained, and the plasmid *pYX1* was successfully constructed (Fig. 3). Plasmid *pYX1* was digested with restriction endonuclease *Eco* RI and the 17.9 kb gene expression cassette was recovered using a DNA fragment sorting and purification kit (BioMag, China). Protoplast generation and transformation of *A. nidulans,* screening and verification of transformant strains of *A. nidulans* were performed according to Ma et al. ((2018) Rational design for heterologous production of aurovertin-type compounds in Aspergillus nidulans, Appl Microbiol Biotechnol 102, 297-304) and Bok et al. ((2004) LaeA, a regulator of secondary metabolism in Aspergillus spp, Eukaryotic cell 3, 527-535). Three transformant strains harboring *xylp-ecdA1-2-AfriboB* in the *yA* locus were obtained and named LO8030-1.1, 1.3, and 1.6, respectively. One of them, LO8030-1.3, was used as the parent strain for the next round of transformation (Fig. 3).

**Table 6 Templates and primers for constructing PCR clones of each fragment of pYX1 plasmid**

| Fragment No. | Fragment | Template | Primer pair | Length (kb) |
|---|---|---|---|---|
| ① | ecdA1 | *E.plicatilis* NRRL 11440 genomic DNA | ecdA1-F and ecdA1-R | 5.8 |
| ② | ecdA2 | *E.plicatilis* NRRL 11440 genomic DNA | ecdA2-F and ecdA2-R | 5.9 |
| ③ | AfriboB-yA-down | pYH-yA-riboA plasmid^{a} | pYX1-Ribo-yAdn-F and pYX1-Ribo-yAdn-R | 3.4 |
| ④ | Plasmid backbone fragment | pYH-yA-riboA plasmid^{a} | pYX1-AscI-F and pYX1-AscI-R | 4.8 |
| ⑤ | yA-up | pYH-yA-riboA plasmid^{a} | pYX1-YAup-F and pYX1-YAup-R | 1.1 |
| ⑥ | xylp | pSK485 plasmid^{b} | pYX1-xylp-F and pYX1-xylp-R | 1.8 |

| | | | | |
|---|---|---|---|---|
| a Wang, PM et al. (2016). " TrpE feedback mutants reveal roadblocks and conduits toward increasing secondary metabolism in Aspergillus fumigatus. " Fungal genetics and biology 89: 102-113.) b Hartmann, T. et al. (2010). "Validation of a self-excising marker in the human pathogen Aspergillus fumigatus by employing the beta-rec/six site-specific recombination system." Appl Environ Microbiol 76(18): 6313-6317 . | | | | |

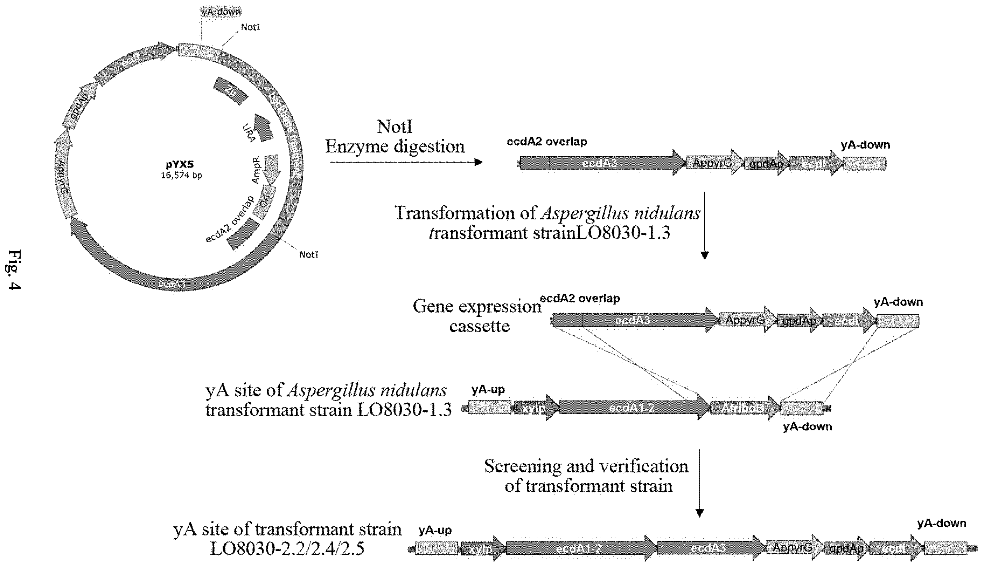

The gene expression cassette for the second round of transformation was from plasmid *pYX5* (Fig. 4). The gene expression cassette contains the third fragment of *ecd*A*, ecdA3,* the nutritional deficiency marker gene *pyrG* (*AppyrG*) from *Aspergillus parasiticus* (GenBank: EU817656), the promoter of the glyceraldehyde-3-phosphate dehydrogenase encoding gene *gpdAp* from *Aspergillus nidulans* (Lim, FY et al. (2012). "Genome-based cluster deletion reveals an endocrocin biosynthetic pathway in Aspergillus fumigatus ." Appl Environ Microbiol 78 (12): 4117-4125.), *ecd*I and a DNA fragment yA-down of about 1 kb downstream of the gene *yA,* wherein, ecdA3 and ecdA2 have a homologous sequence of about 1 kb, which is a homologous region fragment used for homologous recombination in *Aspergillus* transformation. After PCR amplification of each fragment (Table 7), after gel tapping, purification, and mixing, transformation of yeast BJ5464 was performed. After obtaining yeast transformants, yeast plasmids were extracted using a yeast plasmid extraction kit (OMEGA Bio-tek), and the plasmids were transformed into competent *Escherichia coli* for plasmid amplification and PCR detection. By detecting the interface of each fragment with primer pairs, the target fragment of the expected size was obtained, and the plasmid pYX5 was successfully constructed (Fig. 4). Plasmid pYX5 was digested with restriction endonuclease *Not*I and the 11.7 kb gene expression cassette was recovered using a DNA fragment sorting and purification kit (BioMag, China). Protoplast generation and transformation of *A. nidulans* LO8030-1.3, screening and verification of transformant strains of *A. nidulans* LO8030-1.3 were performed according to Ma et al. ((2018) Rational design for heterologous production of aurovertin-type compounds in Aspergillus nidulans, Appl Microbiol Biotechnol 102, 297-304) and Bok et al. ((2004) LaeA, a regulator of secondary metabolism in Aspergillus spp, Eukaryotic cell 3, 527-535). Three transformant strains with *xylp-ecdA1-3-AppyrG-*gpdAp-ecdI inserted into the *yA* locus were obtained and named LO8030-2.2, 2.4, and 2.5, respectively. One of them, LO8030-2.2, was used as the parent strain for the next round of transformation (Fig. 4).

**Table 7 Templates and primers for constructing PCR clones of each fragment of pYX5 plasmid**

| Fragment No. | Fragment | Template | Primer pair | Length (kb) |
|---|---|---|---|---|
| ① | ecdA3 | *E.plicatilis* NRRL 11440 genomic DNA | ecdA3-F and ecdA3-R | 5.5 |
| ② | AppyrG | pJMP9.1 plasmid^{a} | pYX5-pyrG-F and pYX5-pyrG-R | 2 |
| ③ | gpdAp | pJMP9.1 plasmid^{a} | pYX5-gpdA-F and pYX5-gpdA-R | 1.2 |
| ④ | ecdI | *E.plicatilis* NRRL 11440 genomic DNA | ecdI-F and ecdI-R | 2 |
| ⑤ | yA-down | pYH-yA-riboA plasmid | pYX5-yA-down-F and pYX5-yA-down-R | 1 |
| ⑥ | Plasmid backbone fragment | pYH-yA-riboA plasmid^{b} | pYX5-AscI-F2 and pYX5-AscI-R2 | 4.8 |

| | | | | |
|---|---|---|---|---|
| ^{a} Lim, FY et al. (2012). "Genome-based cluster deletion reveals an endocrocin biosynthetic pathway in Aspergillus fumigatus." Appl Environ Microbiol 78(12): 4117-4125. ^{b} Wang, PM et al. (2016). " TrpE feedback mutants reveal roadblocks and conduits toward increasing secondary metabolism in Aspergillus fumigatus. " Fungal genetics and biology 89: 102-113.) | | | | |

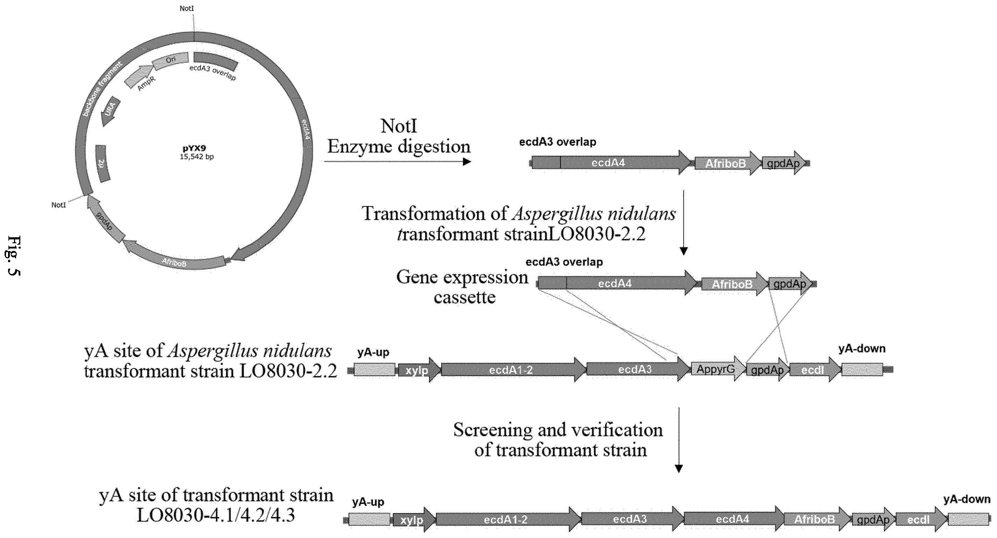

The gene expression cassette for the third round of transformation were from plasmid *pYX9* (Fig. 5). The gene expression cassette contains the fourth fragment (i.e., *ecdA4*) of *ecdA,* the nutritional deficiency marker gene *riboB* (*AfriboB*) derived from *Aspergillus fumigatus* (Nayak, T. et al. (2006). "A versatile and efficient gene-targeting system for Aspergillus nidulans." Genetics 172 (3): 1557-1566.), and the promoter of the gene encoding glyceraldehyde-3-phosphate dehydrogenase from *Aspergillus nidulans* (*GpdAp*) (Lim, FY et al. (2012). "Genome-based cluster deletion reveals an endocrocin biosynthetic pathway in Aspergillus fumigatus." Appl Environ Microbiol 78 (12): 4117-4125*.*)*,* wherein, *ecd*A4 and *ecd*A3 have a homologous sequence of about 1 kb, which is a homologous region fragment used for homologous recombination in Aspergillus transformation. After PCR amplification of each fragment (Table 8), after gel tapping, purification, and mixing, transformation of yeast BJ5464 was performed. After obtaining yeast transformants, yeast plasmids were extracted using a yeast plasmid extraction kit (OMEGA Bio-tek), and the plasmids were transformed into competent *Escherichia coli* for plasmid amplification and PCR detection. By detecting the interface of each fragment with primer pairs, the target fragment of the expected size was obtained, and the plasmid pYX9 was successfully constructed (Fig. 5). Plasmid pYX9 was digested with restriction endonuclease *Not*I and the 10.7 kb gene expression cassette was recovered using a DNA fragment sorting and purification kit (BioMag, China). Protoplast generation and transformation of *A. nidulans* LO8030-2.2, screening and verification of transformant strains of *A. nidulans* LO8030-2.2 were performed according to Ma et al. ((2018) Rational design for heterologous production of aurovertin-type compounds in Aspergillus nidulans, Appl Microbiol Biotechnol 102, 297-304) and Bok et al. ((2004) LaeA, a regulator of secondary metabolism in Aspergillus spp, Eukaryotic cell 3, 527-535). Three transformant strains with *xylp-ecdA-AfriboB-gpdAp-ecdI* inserted into the *yA* locus were obtained and named LO8030-4.1, 4.2, and 4.3, respectively. One of them, LO8030-4.1, was used as the parent strain for the next round of transformation (Fig. 5).

**Table 8 Templates and primers for constructing PCR clones of each fragment of pYX9 plasmid**

| Fragment No. | Fragment | Template | Primer pair | Length (kb) |
|---|---|---|---|---|
| ① | ecdA4 | *E.plicatilis* NRRL 11440 genomic DNA | ecdA4-F and ecdA4-R | 7.1 |
| ② | AfriboB | pYH-yA-riboA plasmid^{a} | pYX9-riboB-F and pYX9-riboB-R | 2.3 |
| ③ | gpdAp | pJMP9.1 plasmid^{b} | pYX9-gpdA-F and pYX9-gpdA-R | 1.2 |
| ④ | Plasmid backbone fragment | pYH-yA-riboA plasmid^{a} | pYX9-AscI-F and pYX9-AscI-R | 4.8 |

| | | | | |
|---|---|---|---|---|
| ^{a} Wang, PM et al. (2016). " TrpE feedback mutants reveal roadblocks and conduits toward increasing secondary metabolism in Aspergillus fumigatus. " Fungal genetics and biology 89: 102-113.) ^{b} Lim, FY et al. (2012). "Genome-based cluster deletion reveals an endocrocin biosynthetic pathway in Aspergillus fumigatus." Appl Environ Microbiol 78(12): 4117-4125. | | | | |

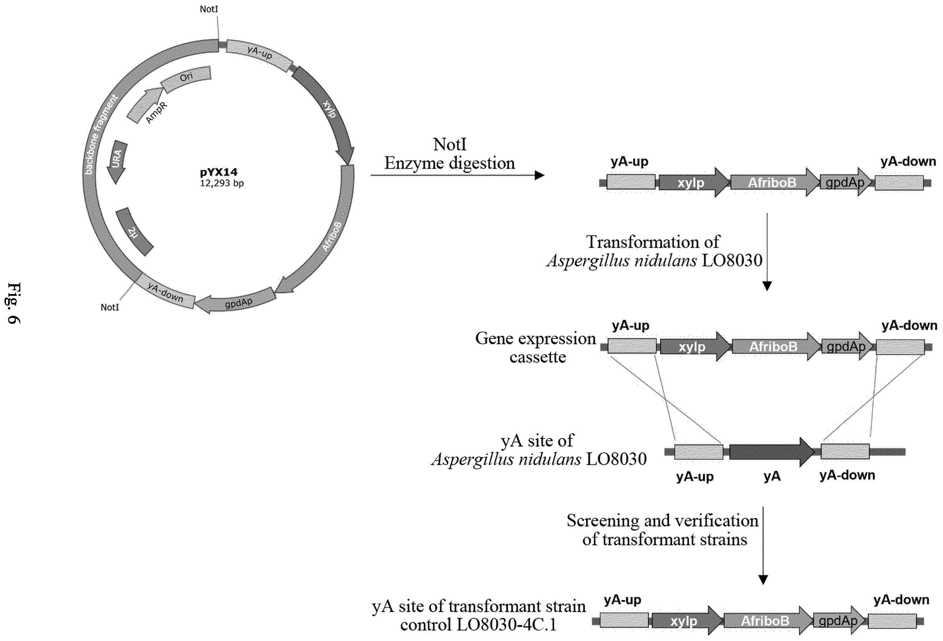

The control plasmid pYX14 was constructed to obtain the expression cassettes containing *xylp, AfriboB* and *gpdAp* (Fig. 6). After PCR amplification of each fragment (Table 9), after gel tapping, purification, and mixing, transformation of yeast BJ5464 was performed. After obtaining yeast transformants, yeast plasmids were extracted using a yeast plasmid extraction kit (OMEGA Bio-tek), and the plasmids were transformed into competent *Escherichia coli* for plasmid amplification and PCR detection. By detecting the interface of each fragment with primer pairs, the target fragment of the expected size was obtained, and the plasmid pYX14 was successfully constructed (Fig. 6). Plasmid pYX14 was digested with restriction endonuclease *Not*I and the 7.5 kb gene expression cassette was recovered using a DNA fragment sorting and purification kit (BioMag, China). Protoplast generation and transformation of *A. nidulans* LO8030, screening and verification of transformant strains of *A. nidulans* LO8030 were performed according to Ma et al. ((2018) Rational design for heterologous production of aurovertin-type compounds in Aspergillus nidulans, Appl Microbiol Biotechnol 102, 297-304) and Bok et al. ((2004) LaeA, a regulator of secondary metabolism in Aspergillus spp, Eukaryotic cell 3, 527-535). A control transformant strain with *xylp-gpdAp-AfriboB* inserted into the yA locus was obtained and named LO8030-4C.1 (Fig. 6).

**Table 9 Templates and primers of PCR clones of each fragment for constructing a control plasmid pYX14**

| Fragment No. | Fragment | Template | Primer pair | Length (kb) |
|---|---|---|---|---|
| ① | yA-up | pYX1 | pYX14-yA-up-F and pYX14-yA-up-R | 1.1 |
| ② | xylp | pYX1 | pYX14-xyl-F and pYX14-xyl-R | 1.8 |
| ③ | AfriboB | pYX9 | pYX14-riboB-F and pYX14-riboB-R | 2.3 |
| ④ | gpdAp | pYX9 | pYX14-gpdA-F and pYX14-gpdA-R | 1.2 |
| ⑤ | yA-down | pYX1 | pYX14-yA-down-F and pYX14-yA-down-R | 1 |
| ⑥ | Plasmid backbone fragment | pYX1 | pYX14-AscI-F and pYX14-AscI-R | 4.8 |

| | | | | |
|---|---|---|---|---|
| ^{a} Wang, PM et al. (2016). " TrpE feedback mutants reveal roadblocks and conduits toward increasing secondary metabolism in Aspergillus fumigatus. " Fungal genetics and biology 89: 102-113.) ^{b} Lim, FY et al. (2012). "Genome-based cluster deletion reveals an endocrocin biosynthetic pathway in Aspergillus fumigatus." Appl Environ Microbiol 78(12): 4117-4125. | | | | |

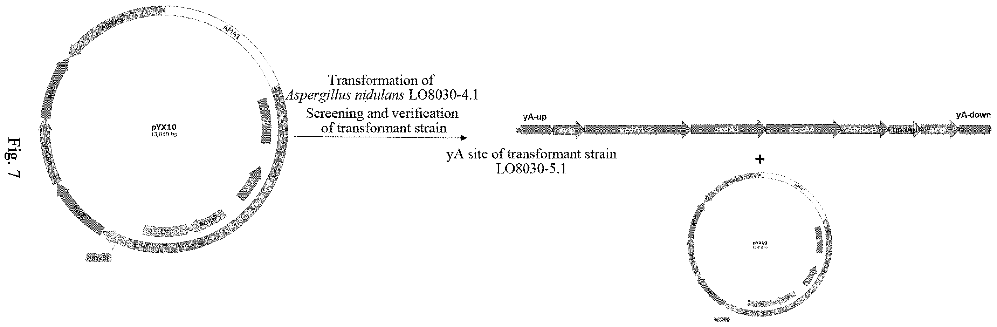

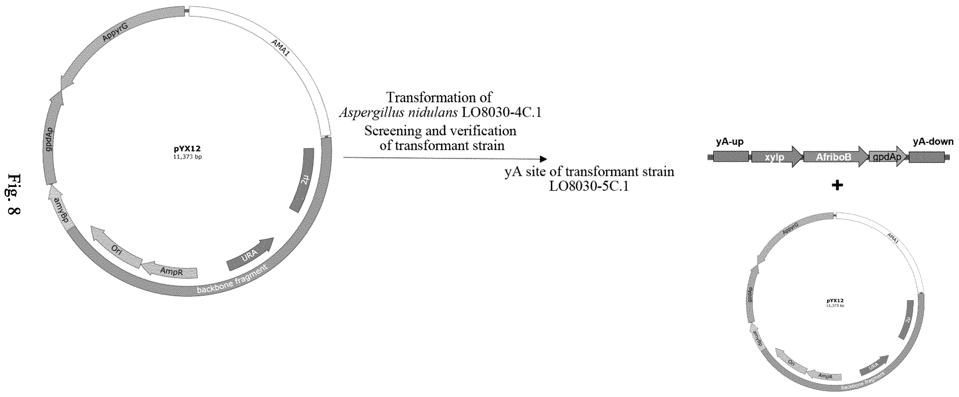

The *"E. coli-yeast-Aspergillus"* shuttle plasmid pYX10 (Fig. 7) was constructed to express the oxygenase genes *ecdK* and *htyE* in the transformant strain LO8030-4.1. In addition to the plasmid backbone containing the *Escherichia coli* origin of replication *(Ori)* and ampicillin resistance (*Amp^{R}*) (GenBank: M77789), the yeast replication origin *2µ* (2 micron/2 micron ori/2 mu) and the uracil auxotrophic screening marker *URA* (GenBank: X75459), the plasmid also has the *htyE* gene (*amyBp-htyE*) regulated by the promoter *amyBp,* the *ecdK* gene (gpdAp-ecdK) regulated by the promoter *gpdAp* (Lim, FY et al. (2012). "Genome-based cluster deletion reveals an endocrocin biosynthetic pathway in Aspergillus fumigatus." Appl Environ Microbiol 78(12): 4117-4125.), and the nutritional deficiency marker gene *pyrG (AppyrG)* derived from *A. parasiticus)* (GenBank: EU817656), and the *Aspergillus* autonomous replicating sequence *AMA1* derived from *Aspergillus nidulans* (Storms, R. et al. (2005). "Plasmid vectors for protein production, gene expression and molecular manipulations in Aspergillus niger." Plasmid 53(3): 191-204.). After PCR amplification of each fragment (Table 10), after gel tapping, purification, and mixing, transformation of yeast BJ5464 was performed. After obtaining yeast transformants, yeast plasmids were extracted using a yeast plasmid extraction kit (OMEGA Bio-tek), and the plasmids were transformed into competent *Escherichia coli* for plasmid amplification and PCR detection. By detecting the interface of each fragment with primer pairs, the target fragment of the expected size was obtained, and the plasmid pYX10 was successfully constructed (Fig. 7). Protoplast generation and transformation of *A. nidulans* LO8030-4.1, screening and verification of transformant strains of *A. nidulans* LO8030-4.1 were performed according to Ma et al. ((2018) Rational design for heterologous production of aurovertin-type compounds in Aspergillus nidulans, Appl Microbiol Biotechnol 102, 297-304) and Bok et al. ((2004) LaeA, a regulator of secondary metabolism in Aspergillus spp, Eukaryotic cell 3, 527-535). A series of transformant strains with plasmid pYX10 in the cells were obtained and named LO8030-5.1 to 5.6, respectively. One of them, LO8030-5.1, was used for subsequent research. At the same time, a control plasmid pYX12 was constructed. The difference between this plasmid and pYX10 is that it does not have the *ecdK* and *htyE* genes. After PCR amplification of each fragment according to Table 11, the transformation of yeast BJ5464, the protoplast generation and transformation of *Aspergillus nidulans* LO8030-4.1, screening and verification of transformant strains of *Aspergillus nidulans* LO8030-4.1 were carried out in the same manner as above. A transformant strain with the control plasmid pYX12 was obtained and named LO8030-5C.1 (Fig. 8).

**Table 10 Templates and primers of PCR clones of each fragment for constructing plasmid pYX10**

| Fragment No. | Fragment | Template | Primer pair | Length (kb) |
|---|---|---|---|---|
| ① | amyBp | pTAex3 plasmid^{a} | pYX10-amyB-F and pYX10-amyB-R | 0.6 |
| ② | htyE | *E.plicatilis* NRRL 11440 | pYX10-htyE-F and | 1.2 |
| | | genomic DNA | pYX14-xyl-R | |
| ③ | gpdAp | pYX5 plasmid | pYX10-gpdA-F and pYX10-gpdA-R | 1.2 |
| ④ | ecdK | *E.plicatilis* NRRL 11440 genomic DNA | pYX10-ecdK-F and pYX10-ecdK-R | 1.2 |
| ⑤ | AppyrG | pYX5 plasmid | pYX10-pyrG-F and pYX10-pyrG-R | 2.0 |
| ⑥ | AMA1 | ANEp2^{b} | pYX10-AMA1-F and pYX10-AMA1-R | 2.6 |
| ⑦ | Plasmid backbone fragment | pYX5 | pYX10-AscI-F and pYX10-AscI-R | 4.8 |

| | | | | |
|---|---|---|---|---|
| ^{a} Awakawa, T. et al. (2012). " A heptaketide naphthaldehyde produced by a polyketide synthase from Nectria haematococca. " Bioorg Med Chem Lett 22(13): 4338-4340.) ^{b} Storms, R. et al. (2005). "Plasmid vectors for protein production, gene expression and molecular manipulations in Aspergillus niger." Plasmid 53(3): 191-204. | | | | |

**Table 11 Templates and primers of PCR clones of each fragment for constructing control plasmid pYX12**

| Fragment No. | Fragment | Template | Primer pair | Length (kb) |
|---|---|---|---|---|
| ① | AppyrG-AMA1-backbone fragment-amyBp | pYX10 plasmid | pYX10-pyrG-F and pYX10-amyB-R | 10 |
| ② | gpdAp | pYX10 plasmid | pYX12-gpdA-F and pYX12-gpdA-R | 1.2 |

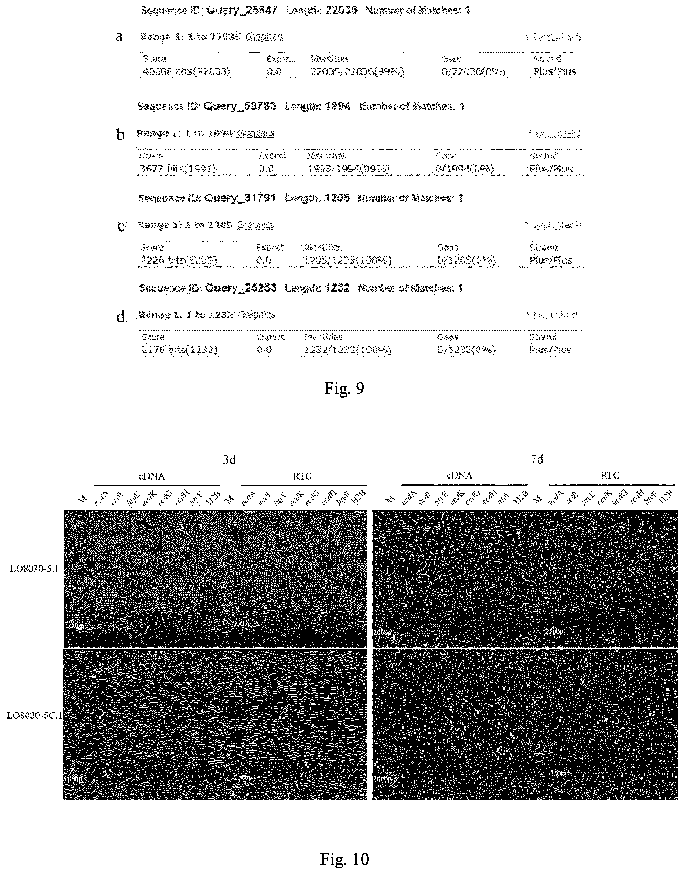

Whole genome sequencing of the gene transformant strain LO8030-5.1 was performed by Illumina HiSeq 2000, and the results showed that no mutations occurred in the four gene cluster genes (*ecdA, ecd*I*, ecdK,* and *htyE*) expressed in the transformant strain LO8030-5.1 (as shown in Fig. 9) and each gene cassette was not randomly inserted into chromosome. This indicates that high-fidelity DNA polymerase can effectively prevent gene mutations that are prone to occur in PCR cloning of large fragments, and also proves the accuracy and reliability of homologous recombination integration of gene expression cassettes in the chassis strain *Aspergillus nidulans* LO8030.

### Example 3. RNA preparation and semi-quantitative RT-PCR

The *Aspergillus nidulans* gene transformant strain LO8030-5.1 and its control strain LO8030-5C.1 were cultured in XSMM medium under the same conditions as those for the fermentation production of echinocandin, and the strains were collected in duplicate. XSMM medium is derived from liquid GMM medium, except that the carbon source glucose (10 g/L) in GMM is replaced by xylose (20 g/L) and starch (20 g/L). Total RNA was extracted using Trizol^{®} reagent (Ambion) and DNase I (Promaga). cDNA was synthesized using the PrimeScript RT reagent kit (TaKaRa) and 100 ng of DNase I-treated RNA. Semi-quantitative RT-PCR was performed in a 25 µL reaction mixture containing 1 µL of diluted cDNA (diluted 10-fold) as a template and 12.5 µL of 2×Es Taq MasterMix (Cwbio, China). The internal control was the histone 2B gene (*H2B*), and the primers used for each indicated gene are shown in Table 4.

The results showed that the four transformed genes (*ecdA, ecd*I*, htyE,* and *ecdK*) could be transcribed to varying degrees. Furthermore, the expression of *ecdG, ecdH* and *htyF* was not detected, indicating that the above genes were not transformed into LO8030-5.1. The results of RT-PCR are shown in Fig. 10.

### Example 4. Fermentation and HPLC/MS analysis

The *Aspergillus nidulans* gene transformant strains LO8030-4.1 and LO8030-5.1 and their controls LO8030-4C.1 and LO8030-5C.1 were cultured in XSMM fermentation medium. XSMM medium is derived from liquid GMM medium, except that the carbon source glucose (10 g/L) in GMM is replaced by xylose (20 g/L) and starch (20 g/L). 1 mg/L pyridoxine hydrochloride was added to the culture medium of LO8030-5.1 and LO8030-5C.1, and 1.26 g/L uracil, 1.26 g/L uridine and 100 mg/L 4R-Me-L-proline were added to the culture medium of LO8030-4.1 and LO8030-4C.1. Since the BGC biosynthetic gene cluster transformed in the present invention does not contain *htyA-D* genes, L-homotyrosine (100 mg/L) is added to the culture medium of the present invention for the synthesis of deoxyechinocandins. In addition, linoleic acid (300 mg/L) was added to the XSMM fermentation medium to promote the production of echinocandins.

The above 100 mL XSMM medium was added to a 500 mL flask and fermented at 25°C and 180 rpm for 12 days in triplicate.

Then, the fungal cells were separated from the culture solution by filtration. The cells were soaked in methanol and the metabolites were extracted by ultrasound for 3 times, 30 min each time, and the culture medium was extracted for 3 times with an equal amount of ethyl acetate. The organic phases were combined, and the solvent was removed by evaporation in vacuo. The extract was dissolved in 1 mL of methanol, filtered through a 0.22 µm filter membrane, and used for HPLC and LC-MS analysis.

20 µL of the filtrate was subj ected to reverse phase high performance liquid chromatography (RP-HPLC) (Agilent 1260). A COSMOCIL C₁₈ -MS-II column (4.6×250 mm, 5 µm) was used with a flow rate of 1 mL/min, a column temperature of 35°C, an eluent of 10-100% MeOH-H₂O (v/v) (linear gradient), and a time of 60 min. The chromatographic peaks were detected at a wavelength of 222 nm.

LC-MS analysis was performed with an Agilent UPLC-Triple-TOF 5600⁺ LC/MS system using positive mode electrospray ionization. A Waters ACQUITY UPLC HSS SB-C₁₈ column (100×2.1 mm, 1.7µm) was used, the eluent was 5-100% methanol in water + 0.1% formic acid (linear gradient), and the flow rate was 0.4 mL/min.

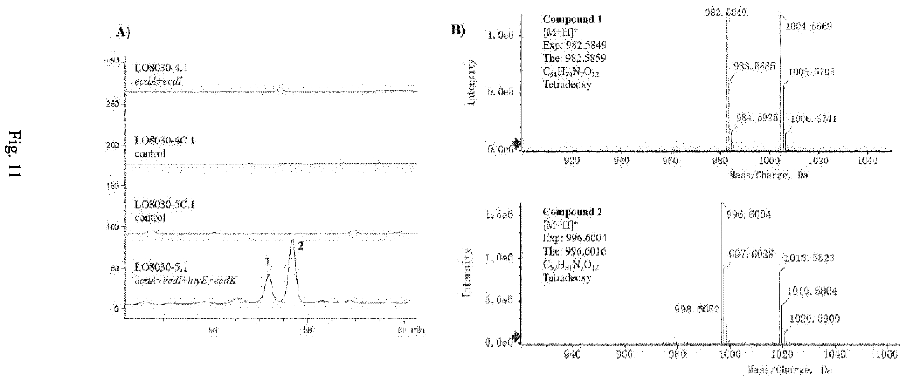

HPLC analysis showed that compared with the control strain LO8030-4C.1, LO8030-4.1 did not have any new obvious peaks (as shown in Fig. 11). Compared with the control strain LO8030-5C.1, two new peaks appeared at retention times of 57.3 minutes and 57.8 minutes in the HPLC spectrum of LO8030-5.1 (as shown in Fig. 11). Subsequently, the molecular weights of these two peaks were determined by LC-MS. Compound 1: TOF-ESIMS, *m*/*z* 982.5841 [M+H]⁺, *m*/*z* 1004.5668 [M+Na]⁺; Compound 2: TOF-ESIMS, *m*/*z* 996.6004 [M+H]⁺, *m*/*z* 1018.5823 [M+Na]⁺ (Fig. 11).

### Example 5. Nuclear Magnetic Resonance Analysis of Compound 2 (Echinocandin E) and Compound 1 (Echinocandin F)

After the fermentation of the transformant strain LO8030-5.1 in 10 L XSMM medium was completed, the fermentation liquid and the thallus were extracted three times with equal volumes of ethyl acetate and methanol, respectively. The organic phases were combined and evaporated under reduced pressure to obtain a crude extract (2.20 g). The extract was separated using a silica gel column with a mobile phase of CH₂Cl₂/MeOH (50:1, 20:1, 10:1, 5:1, 2:1, 1:1). The echinocandins were preliminarily identified by thin layer chromatography (TLC) and high performance liquid chromatography (HPLC). Then, it was further purified by semi-preparative HPLC with a COSMOCIL C18-MS-II column (10 × 250 mm, 5 µm) and a mobile phase of MeOH/H₂O (85:15) at a flow rate of 3.0 mL/min.

The separated compounds 1 and 2 were dissolved in CD₃OD for NMR analysis. ¹H, ¹³C and 2D (¹H-¹³C HSQC, ¹H-¹³C HMBC and ¹H-¹HCOSY) NMR spectra were recorded on a JNM-ECZ600R/S1 spectrometer. Table 12 shows the ¹H NMR and ¹³C NMR data.

**Table 12. ¹³C NMR and ¹H NMR of Echinocandin E and Echinocandin F**

| Position | 1 (Echinocandin F) | 2 (Echinocandin E) | Position | 1 (Echinocandin F) | 2 (Echinocandin E) |
|---|---|---|---|---|---|
| | ¹³C NMR | | | ¹H NMR | |

| 1^{st} L-Omithine | | | | | |
|---|---|---|---|---|---|
| C-1 | 175.1 | 175.0 | | | |
| C-2 | 53.5 | 53.4 | 2-H | 4.39 | 4.38 |
| C-3 | 28.4 | 28.4 | 3-H₂ | 1.54, 2.10 | 1.54, 2.06 |
| C-4 | 25.1 | 25.1 | 4-H₂ | 1.69 | 1.69 |
| C-5 | 38.5 | 38.5 | 5-H₂ | 2.98, 3.47 | 3.02, 3.41 |

| 2^{st} L-Threonine | | | | | |
|---|---|---|---|---|---|
| C-1 | 172.9 | 172.8 | | | |
| C-2 | 58.9 | 58.9 | 2-H | 4.83 | 4.81 |
| C-3 | 68.7 | 68.5 | 3-H | 4.42 | 4.41 |
| C-4 | 19.9 | 19.9 | 4-H₃ | 1.26 | 1.26 |

| 5^{st} L-Threonine | | | | | |
|---|---|---|---|---|---|
| C-1 | 170.6 | 170.8 | | | |
| C-2 | 56.6 | 56.7 | 2-H | 4.89 | 4.90 |
| C-3 | 69.5 | 69.4 | 3-H | 4.23 | 4.27 |
| C-4 | 20.6 | 20.6 | 4-H₃ | 1.18 | 1.18 |

| 3^{rd} 4R-OH-L-proline | | | | | |
|---|---|---|---|---|---|
| C-1 | 173.8 | 173.7 | | | |
| C-2 | 62.3 | 62.2 | 2-H | 4.54 | 4.55 |
| C-3 | 38.7 | 38.7 | 3-H₂ | 1.81, 2.32 | 1.82, 2.32 |
| C-4 | 71.4 | 71.4 | 4-H | 4.48 | 4.49 |
| C-5 | 57.2 | 57.2 | 5-H₂ | 3.75 | 3.76 |

| 4^{th} L-Homotyrosine | | | | | |
|---|---|---|---|---|---|
| C-1 | 174.0 | 174.0 | | | |
| C-2 | 55.4 | 55.4 | 2-H | 4.26 | 4.23 |
| C-3 | 34.5 | 34.4 | 3-H₂ | 2.11, 2.24 | 2.14, 2.24 |
| C-4 | 33.0 | 33.0 | 4-H₂ | 2.58 | 2.58 |
| C-1' | 133.2 | 133.2 | | | |
| C-2'/C-6' | 130.6 | 130.6 | 2'/6'-H | 7.01 | 7.01 |
| C-3'/C-5' | 116.4 | 116.4 | 3'/5'-H | 6.70 | 6.70 |
| C-4' | 156.9 | 156.9 | | | |

| 6^{th} 3*S*-OH-L-proline/3*S*-OH-4*S*-Me-L-proline | | | | | |
|---|---|---|---|---|---|
| C-1 | 172.6 | 172.5 | | | |
| C-2 | 69.9 | 70.1 | 2-H | 4.22 | 4.34 |
| C-3 | 74.3 | 76.0 | 3-H | 4.35 | 4.18 |
| C-4 | 34.5 | 39.1 | 4-H₂/H | 1.97, 2.24 | 2.45 |
| C-5 | 46.8 | 52.9 | 5-H₂ | 3.81 | 3.39, 3.88 |
| 4-CH₃ | | 11.5 | 4-CH₃ | | 1.06 |

| Fatty acid side chain | | | | | |
|---|---|---|---|---|---|
| C-1 | 176.7 | 176.7 | | | |
| C-2 | 37.0 | 36.9 | 2-H | 2.24 (m, 2H) | 2.23(m, 2H) |
| C-3 | 27.2 | 27.2 | 3-H | 1.61(m, 2H) | 1.60(m, 2H) |
| C-8/C-14 | 28.3, 28.3 | 28.3, 28.3 | 8-H /14-H | 2.06 (m, 4H) | 2.06 (m, 4H) |
| C-9 /C-10/ C-12/C-13 | 131.1, 131.1, 129.2, 129.2 | 131.1, 131.1, 129.2, 129.2 | 9-H /10-H / 12-H/13-H | 5.29-5.39 (m, 4H) | 5.29-5.39 (m, 4H) |
| C-11 | 26.7 | 26.7 | 11-H | 2.78 (*t, J =* 6.8 Hz, 2H) | 2.77 *(t, J=* 6.8 Hz, 2H) |
| C-4/C-5 / C-6/C-7 / C-15/C-16 / C-17 | 32.8, 30.9, 30.6, 30.5, 30.4, 30.4, 23.8 | 32.8, 30.9, 30.6, 30.5, 30.4, 30.4, 23.8 | 4-H /5-H / 6-H /7-H / 15-H /16-H / 17-H | 1.28-1.39 (m, 14H) | 1.28-1.39 (m, 14H) |
| C-18 | 14.6 | 14.6 | 18-H | 0.91 (t, *J* = 7.2 Hz, 3H) | 0.91 (t, *J* = 7.3 Hz, 3H) |

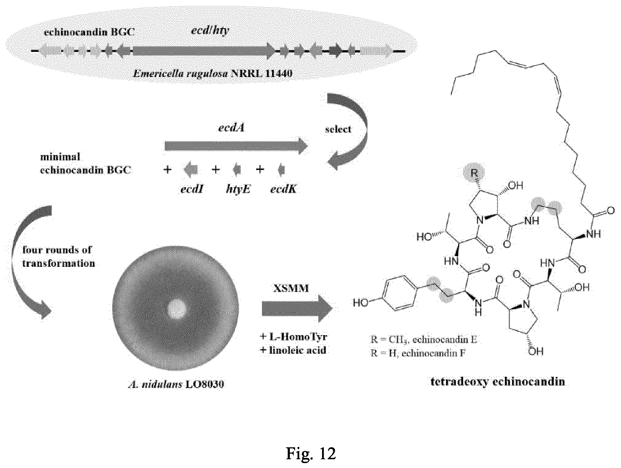

Compound 1 (echinocandin F) has the same structural features as compound 2 (echinocandin E), except for the loss of the methyl on the 3S-OH-4S-Me-L-proline residue, including the loss of the hydroxyl at C₃/C₄ of L-homotyrosine and the loss of the hydroxyl at C₄/C₅ of L-ornithine (as shown in Fig. 12). In the gene cluster (*ecdA, ecd*I*, ecdK, htyE*), *ecdK* is responsible for the formation of 3S-OH-4S -Me-L-proline. Therefore, we speculate that the reason that the transformant strain LO8030-5.1 produces echinocandin F may be that *ecdK* does not play a role in the biosynthesis of this compound, that is, the biosynthetic gene cluster of echinocandin F is (*ecdA, ecd*I*, htyE*). This inference is verified in Examples 6 and 7.

### Example 6: Construction of plasmid and preparation of Aspergillus nidulans gene transformant strain LO8030-4.2.1

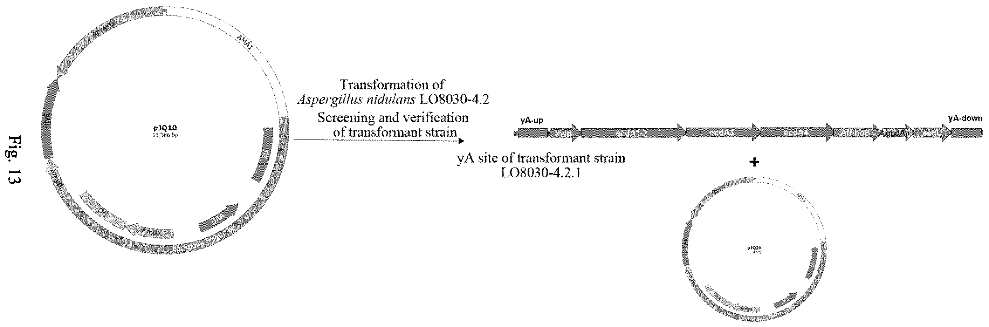

The *"E. coli-yeast-Aspergillus"* shuttle plasmid pJQ10 (Fig. 13) was constructed to express the oxygenase gene *htyE* in the transformant strain LO8030-4.2. In addition to the plasmid backbone containing the *Escherichia coli* origin of replication (*Ori*) and ampicillin resistance (*Amp^{R}*) (GenBank: M77789), the yeast replication origin *2µ* (2 micron/2 micron ori/2 mu) and the uracil auxotrophic screening marker *URA* (GenBank: X75459), the plasmid also has the *htyE* gene (*amyBp-htyE*) regulated by the promoter *amyBp,* the nutritional deficiency marker gene pyrG (AppyrG) derived from *A. parasiticus)* (GenBank: EU817656), and the *Aspergillus* autonomous replicating sequence *AMA1* derived from *Aspergillus nidulans* (Storms, R. et al. (2005). "Plasmid vectors for protein production, gene expression and molecular manipulations in Aspergillus niger." Plasmid 53(3): 191-204.). After PCR amplification of each fragment (Table 13), after gel tapping, purification, and mixing, transformation of yeast BJ5464 was performed. After obtaining yeast transformants, yeast plasmids were extracted using a yeast plasmid extraction kit (OMEGA Bio-tek), and the plasmids were transformed into competent *Escherichia coli* for plasmid amplification and PCR detection. By detecting the interface of each fragment with primer pairs, the target fragment of the expected size was obtained, and the plasmid pJQ10 was successfully constructed (Fig. 13). Protoplast generation and transformation of *A. nidulans* LO8030-4.2, screening and verification of transformant strains of *A. nidulans* LO8030-4.2 were performed according to Ma et al. ((2018) Rational design for heterologous production of aurovertin-type compounds in Aspergillus nidulans, Appl Microbiol Biotechnol 102, 297-304) and Bok et al. ((2004) LaeA, a regulator of secondary metabolism in Aspergillus spp, Eukaryotic cell 3, 527-535). A series of transformant strains were obtained that contained plasmid pJQ10 in the cells, and one of them was named LO8030-4.2.1 for subsequent research.

**Table 13 Templates and primers for constructing PCR clones of each fragment of pJQ10 plasmid^{b}**

| Fragment No. | Fragment | Template | Primer pair | Length (kb) |
|---|---|---|---|---|
| ① | pyrG-AMA1 | pYX10 plasmid | pJQ10-pyrG/AMA1-F | 4.7 |
| | | | and pJQ10-pyrG/AMA1-R | |
| ② | Plasmid backbone fragment | pYX10 plasmid | pJQ10-AscI-F and pJQ10-AscI-R | 4.8 |
| ③ | amyBp-htyE | pYX10 plasmid | pJQ 10-PamyB/htyE-F and pJQ10-PamyB/htyE-R | 1.9 |

### Example 7. Fermentation and analysis

*The Aspergillus nidulans* gene transformant strain LO8030-4.2.1 was cultured in XSMM medium, and 1 mg/L pyridoxine hydrochloride was added to the medium. In addition, L-homotyrosine (100 mg/L) and linoleic acid (300 mg/L) were added to the XSMM fermentation medium. After the transformant strain LO8030-4.2.1 was fermented in 10 L XSMM medium for 12 days, the fermentation liquid and the fungal cells were extracted three times with equal volumes of ethyl acetate and methanol, respectively. The organic phases were combined and evaporated under reduced pressure to obtain a crude extract. According to the analysis method in Example 4, it was confirmed that the transformant strain LO8030-4.2.1 was able to produce echinocandin F.

### Example 8. Quantitative analysis of echinocandin E and echinocandin F in fermentation products

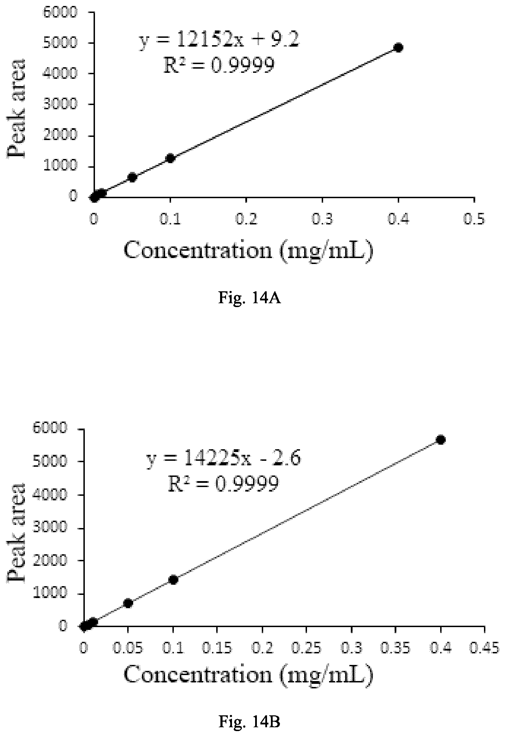

The transformant strains LO8030-5.1 and LO8030-4.2.1 were fermented in parallel in three bottles (200 mL in each bottle) according to the preceding fermentation method. Ten volumes of methanol were added to each bottle of fermentation product (including fungal cells and fermentation liquid). After precipitation, the samples were centrifuged and filtered through a membrane and used as samples for HPLC quantitative analysis. The ODS-HPLC analysis conditions were: isocratic elution with 80% methanol/water, a flow rate of 1 mL/min, and a detection wavelength of 220 nm. The quantitative control substances are the echinocandin E and echinocandin F separated above. The control substances were prepared into solutions with different concentrations, and the HPLC peak areas of the solutions at each concentration under the condition of constant injection volume were measured to draw a standard curve (Figs. 14A and 14B). According to the standard curve, the yield of echinocandin E and echinocandin F in the fermentation product of LO8030-5.1 was 10 mg/L and 6 mg/L, respectively, and the yield of echinocandin F in the fermentation product of LO8030-4.2.1 was 7 mg/L.

### Example 9: Construction of plasmid and preparation of Aspergillus nidulans gene transformant strain LO8030-4.2.1.5

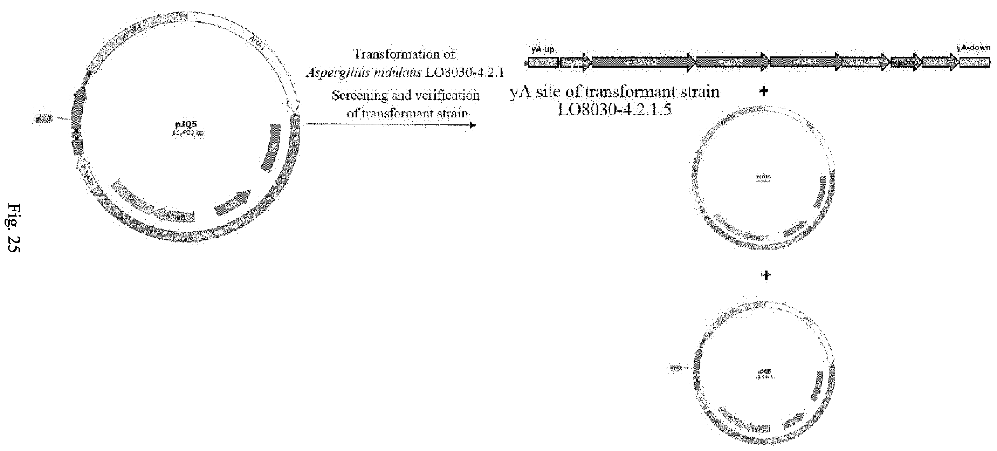

The "E. coli-yeast-Aspergillus" shuttle plasmid pJQ5 (Fig. 25) was constructed to express the oxygenase gene *ecdG* in the transformant strain LO8030-4.2.1. In addition to the plasmid backbone containing the *Escherichia coli* origin of replication (Ori) and ampicillin resistance screening marker (AmpR) (GenBank: M77789), the yeast origin of replication 2µ (2 micron/2 micron ori/2 mu) and the uracil auxotrophic screening marker URA (GenBank: X75459), the plasmid also has the *ecdG* gene (*amyBp-ecdG*) regulated by the promoter amyBp, the nutritional deficiency marker gene *pyroA4* derived from *Aspergillus fumigatus*) (GenBank: CP097567.1), and the *Aspergillus* autonomous replicating sequence *AMA1* derived from *Aspergillus nidulans* (Storms, R. et al. (2005). "Plasmid vectors for protein production, gene expression and molecular manipulations in Aspergillus niger." Plasmid 53(3): 191-204.). After PCR amplification of each fragment (Table 14), after gel tapping, purification, and mixing, transformation of yeast BJ5464 was performed. After obtaining yeast transformants, yeast plasmids were extracted using a yeast plasmid extraction kit (OMEGA Bio-tek), and the plasmids were transformed into competent *Escherichia coli* for plasmid amplification and PCR detection. By detecting the interface of each fragment with primer pairs, the target fragment of the expected size was obtained, and the plasmid pJQ5 was successfully constructed (Fig. 25). Protoplast generation and transformation of *A. nidulans* LO8030-4.2.1, screening and verification of transformant strains of *A. nidulans* LO8030-4.2.1 were performed according to Ma et al. ((2018) Rational design for heterologous production of aurovertin-type compounds in Aspergillus nidulans, Appl Microbiol Biotechnol 102, 297-304) and Bok et al. ((2004) LaeA, a regulator of secondary metabolism in Aspergillus spp, Eukaryotic cell 3, 527-535). A series of transformant strains were obtained that contained plasmid pJQ5 in the cells, and one of them was named LO8030-4.2.1.5 for subsequent research.

**Table 14 Templates and primers for constructing PCR clones of each fragment of pJQ5 plasmid^{b}**

| Fragment No. | Fragment | Template | Primer pair | Length (kb) |
|---|---|---|---|---|
| ① | *pyro-AMA1* | pYH11 plasmid | pJQ5-pyro/AMA1-F and pJQ5-pyro/AMA1-R | 4.5 |
| ② | Plasmid backbone fragment | pYH11 plasmid | pJQ5-AscI-F and pJQ5-AscI-R | 4.8 |
| ③ | *amyBp-ecdG* | pYH11 plasmid | pJQ5-PamyB/ecdG-F and pJQ5-PamyB/ecdG-R | 1.8 |

### Example 10. Fermentation and analysis

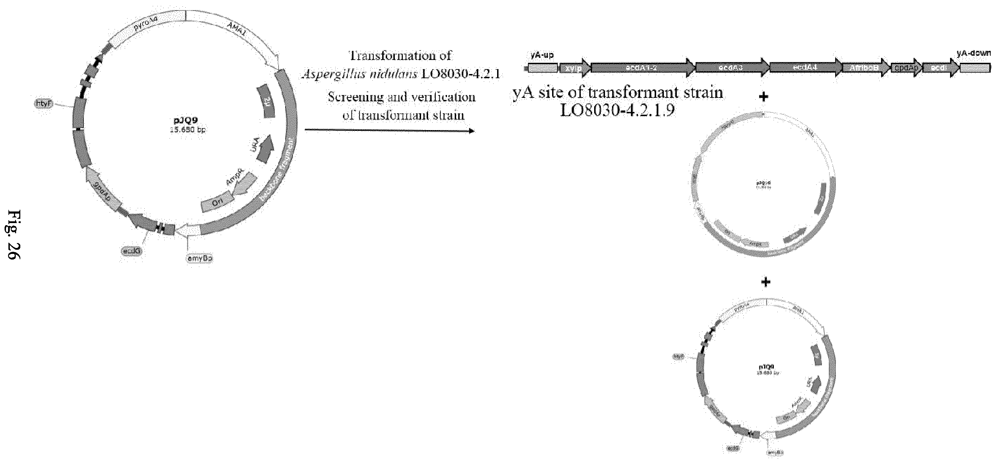

The *Aspergillus nidulans* gene transformant strain LO8030-4.2.1.5 was cultured in XSMM medium, and L-homotyrosine (100 mg/L) and linoleic acid (300 mg/L) were added to the medium. After the transformant strain LO8030-4.2.1 was fermented in 10 L XSMM medium for 12 days, the fermentation liquid and the fungal cells were extracted for three times with equal volumes of ethyl acetate and methanol, respectively. The organic phases were combined and evaporated under reduced pressure to obtain a crude extract. The extract was separated using a silica gel column with a mobile phase of CH₂Cl₂/MeOH (50:1, 20:1, 10:1, 5:1, 2:1, 1:1). The echinocandins were preliminarily identified by thin layer chromatography (TLC) and high-performance liquid chromatography (HPLC). Then, it was further purified by semi-preparative HPLC with a COSMOCIL C18-MS-II column (10 × 250 mm, 5 µm) and a mobile phase of MeOH/H₂O (85:15) at a flow rate of 3.0 mL/min. Compound 3 was isolated, which is a novel tetradeoxyechinocandin and named as echinocandin G (ECG). TOF-ESIMS *m*/*z* 998.5800 [M+H] *⁺, m*/*z* 1020.5626 [M+Na] ⁺ (Fig. 26).

The isolated compound 3 was dissolved in CD₃OD for NMR analysis. ¹H, ¹³C and 2D (¹H-¹³C HSQC, ¹H -¹³C HMBC and ¹H-¹HCOSY) NMR spectra were recorded on a JNM-ECZ600R/S1 spectrometer. Table 15 shows the ¹H NMR and ¹³C NMR data.

**Table 15 ¹H and ¹³C NMR data of compound Echinocandin G (¹H 600 MHz, ¹³C 150 MHz, CD₃OD)**

| Position | ¹³C NMR | Position | ¹H NMR |
|---|---|---|---|
| 1^{st} L-Ornithine | | | |
| C-1 | 175.2 | | |
| C-2 | 53.3 | 2-H | 4.40 |
| C-3 | 28.1 | 3-H₂ | 1.56, 2.16 |
| C-4 | 24.9 | 4-H₂ | 1.73, 1.73 |
| C-5 | 38.2 | 5-H₂ | 2.94, 3.53 |

| 2^{st} L-Threonine | | | |
|---|---|---|---|
| C-1 | 173.1 | | |
| C-2 | 58.9 | 2-H | 4.93 |
| C-3 | 68.4 | 3-H | 4.49 |
| C-4 | 20.4 | 4-H | 1.23 |

| 5^{st} L-Threonine | | | |
|---|---|---|---|
| C-1 | 170.5 | | |
| C-2 | 57.0 | 2-H | 4.91 |
| C-3 | 69.5 | 3-H | 4.22 |
| C-4 | 19.8 | 4-H | 1.19 |

| 3^{rd} 4*R*-methyl-L-proline | | | |
|---|---|---|---|
| C-1 | 174.4 | | |
| C-2 | 62.6 | 2-H | 4.64 |
| C-3 | 39.1 | 3-H₂ | 2.47, 2.07 |
| C-4 | 71.4 | 4-H | 4.58 |
| C-5 | 57.4 | 5-H₂ | 4.02, 3.82 |

| 4^{th} L-Homotyrosine | | | |
|---|---|---|---|
| C-1 | 172.8 | | |
| C-2 | 57.9 | 2-H | 4.45 |
| C-3 | 74.4 | 3-H | 4.39 |
| C-4 | 41.1 | 4-H₂ | 2.57, 2.66 |
| C-1' | 129.9 | | |
| C-2'/ C-6' | 131.6 | 2'/ 6'- H | 7.02 |
| C-3'/ C-5' | 116.4 | 3'/ 5'- H | 6.71 |
| C-4' | 157.3 | | |

| 6^{rd} 3S-Hydroxy-L-proline | | | |
|---|---|---|---|
| C-1 | 172.8 | | |
| C-2 | 69.9 | 2-H | 4.19 |
| C-3 | 74.4 | 3-H | 4.33 |
| C-4 | 34.6 | 4-H₂ | 1.96, 2.25 |
| C-5 | 46.9 | 5-H₂ | 3.79, 3.79 |

| Fatty acid side chains | | | |
|---|---|---|---|
| C-1 | 176.6 | | |
| C-2 | 37.0 | 2-H | 2.25 (m, 2H) |
| C-3 | 27.2 | 3-H | 1.61(m, 2H) |
| C-8 / C-14 | 28.3, 28.3 | 8-H / 14-H | 2.07 (m, 3H) |
| C-11 | 26.7 | 11-H | 2.78 *(t, J* = 6.7 Hz, 1H) |
| C-9 / C-10 / C-12 / C-13 | 131.1, 131.1, 129.2, 129.2 | 9-H / 10-H / 12-H / 13-H | 5.30-5.40 (m, 2H) |
| C-4 / C-5 / C-6 / C-7 / C-15 / C-16 / C-17 | 32.8, 30.9, 30.6, 30.5, 30.4, 30.4, 23.8 | 4-H / 5-H / 6-H / 7-H / 15-H / 16-H / 17-H | 1.28-1.40 (m, 14H) |
| C-18 | 14.6 | 18-H | 0.91 (t, *J* = 7.3 Hz, 3H) |

### Example 11: Construction of plasmid and preparation of Aspergillus nidulans gene transformant strain LO8030-4.2.1.9

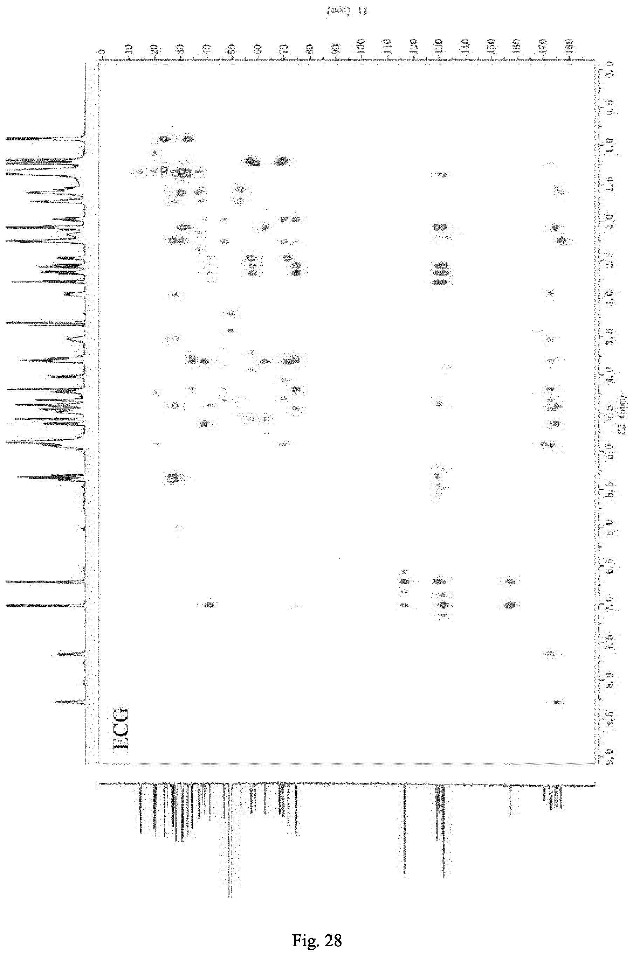

The "*E. coli*-yeast-Aspergillus" shuttle plasmid pJQ9 (Fig. 28) was constructed to express the oxygenase genes *ecdG* and *htyF* in the transformant strain LO8030-4.2.1. In addition to the plasmid backbone of the *Escherichia coli* origin of replication (Ori) and ampicillin resistance screening marker (AmpR) (GenBank: M77789), the yeast origin of replication 2µ (2 micron/2 micron ori/2 mu) and the uracil auxotrophic screening marker URA (GenBank: X75459), the plasmid also has the *ecdG* gene (*amyBp-ecdG*) regulated by the promoter amyBp, the *htyF* gene *(gpdAp-htyF)* regulated by the promoter *gpdAp,* the nutritional deficiency marker gene *pyroA4* derived from *Aspergillus fumigatus*) (GenBank: CP097567.1), and the *Aspergillus* autonomous replicating sequence *AMA1* derived from *Aspergillus nidulans* (Storms, R. et al. (2005). "Plasmid vectors for protein production, gene expression and molecular manipulations in Aspergillus niger." Plasmid 53(3): 191-204.). After PCR amplification of each fragment (Table 16), after gel tapping, purification, and mixing, transformation of yeast BJ5464 was performed. After obtaining yeast transformants, yeast plasmids were extracted using a yeast plasmid extraction kit (OMEGA Bio-tek), and the plasmids were transformed into competent *Escherichia coli* for plasmid amplification and PCR detection. By detecting the interface of each fragment with primer pairs, the target fragment of the expected size was obtained, and the plasmid pJQ9 was successfully constructed (Fig. 28). Protoplast generation and transformation of *A. nidulans* LO8030-4.2.1, screening and verification of transformant strains of *A. nidulans* LO8030-4.2.1 were performed according to Ma et al. ((2018) Rational design for heterologous production of aurovertin-type compounds in Aspergillus nidulans, Appl Microbiol Biotechnol 102, 297-304) and Bok et al. ((2004) LaeA, a regulator of secondary metabolism in Aspergillus spp, Eukaryotic cell 3, 527-535). A series of transformant strains were obtained that contained plasmid pJQ9 in the cells, and one of them was named LO8030-4.2.1 for subsequent research.

**Table 16 Templates and primers for constructing PCR clones of each fragment of pJQ9 plasmid**

| Fragment No. | Fragment | Template | Primer pair | Length (kb) |
|---|---|---|---|---|
| ① | *pyro-AMA1* | pYH11 plasmid | pJQ9-pyro/AMA1-F and pJQ9-pyro/AMA1-R | 4.5 |
| ② | Plasmid backbone fragment | pYH11 plasmid | pJQ9-AscI-F and pJQ9-AscI-R | 4.8 |
| ③ | *amyBp-ecdG* | pYH11 plasmid | pJQ9-PamyB/ecdG-F and pJQ9-PamyB/ecdG-R | 1.8 |
| ④ | *gpdAp-htyF* | pYH11 plasmid | pJQ9-PgpdA/htyF-F and pJQ9-PgpdA/htyF-R | 4.0 |

### Example 12. Fermentation and analysis

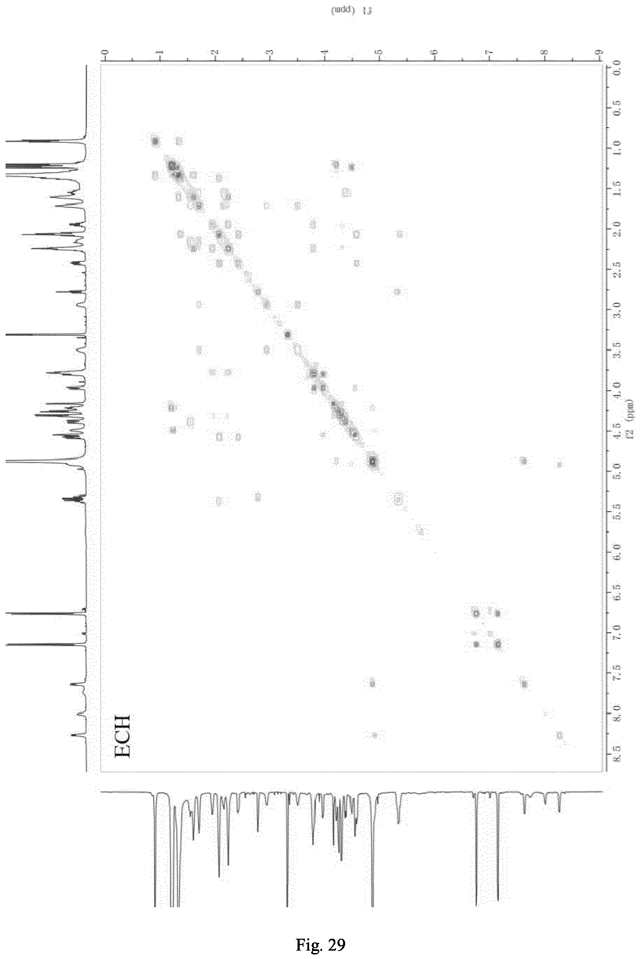

The *Aspergillus nidulans* gene transformant strain LO8030-4.2.1.9 was cultured in XSMM medium, and L-homotyrosine (100 mg/L) and linoleic acid (300 mg/L) were added to the medium. After the transformant strain LO8030-4.2.1.9 was fermented in 10 L XSMM medium for 12 days, the fermentation liquid and the fungal cells were extracted for three times with equal volumes of ethyl acetate and methanol, respectively. The organic phases were combined and evaporated under reduced pressure to obtain a crude extract. The extract was separated using a silica gel column with a mobile phase of CH₂Cl₂/MeOH (50:1, 20:1, 10:1, 5:1, 2:1, 1:1). The echinocandins were preliminarily identified by thin layer chromatography (TLC) and high performance liquid chromatography (HPLC). Then, it was further purified by semi-preparative HPLC with a COSMOCIL C18-MS-II column (10 × 250 mm, 5 µm) and a mobile phase of MeOH/H₂O (85:15) at a flow rate of 3.0 mL/min. Compound 4 was isolated, which is a novel tetradeoxyechinocandin and named as echinocandin H (ECH). TOF-ESIMS C₅₁H₇₉N₇O₁₄ *m*/*z* 1014.5770 [M+H]⁺, *m*/*z* 1036.5590 [M+Na]⁺ (Fig. 29).

The isolated compound 4 was dissolved in CD₃OD for NMR analysis. ¹H, ¹³C and 2D (¹H-¹³C HSQC, ¹H -¹³C HMBC and ¹H-¹HCOSY) NMR spectra were recorded on a JNM-ECZ600R/S1 spectrometer. Table 17 shows the ¹H NMR and ¹³C NMR data.

**Table 17 ¹H and ¹³C NMR data of compound ECH (¹H 600 MHz, ¹³C 150 MHz, CD₃OD)**

| Position | ¹³C NMR | Position | ¹H NMR |
|---|---|---|---|
| 1^{st} L-Ornithine | | | |
| C-1 | 175.2 | | |
| C-2 | 53.5 | 2-H | 4.39 |
| C-3 | 28.2 | 3-H₂ | 1.55, 2.15 |
| C-4 | 25.0 | 4-H₂ | 1.71, 1.71 |
| C-5 | 38.5 | 5-H₂ | 2.94, 3.50 |

| 2^{st} L-Threonine | | | |
|---|---|---|---|
| C-1 | 172.6 | | |
| C-2 | 58.7 | 2-H | 4.90 |
| C-3 | 68.4 | 3-H | 4.49 |
| C-4 | 19.9 | 4-H | 1.23 |

| 5^{st} L-Threonine | | | |
|---|---|---|---|
| C-1 | 170.4 | | |
| C-2 | 57.2 | 2-H | 4.88 |
| C-3 | 69.7 | 3-H | 4.22 |
| C-4 | 20.3 | 4-H | 1.21 |

| 3^{rd} 4*R*-methyl-L-proline | | | |
|---|---|---|---|
| C-1 | 173.9 | | |
| C-2 | 62.7 | 2-H | 4.58 |
| C-3 | 38.8 | 3-H₂ | 2.42, 2.07 |
| C-4 | 71.4 | 4-H | 4.55 |
| C-5 | 57.4 | 5-H₂ | 3.97, 3.80 |

| 4^{th} L-Homotyrosine | | | |
|---|---|---|---|
| C-1 | 173.1 | | |
| C-2 | 56.8 | 2-H | 4.26 |
| C-3 | 77.0 | 3-H | 4.26 |
| C-4 | 76.0 | 4-H | 4.31 |
| C-1' | 133.2 | | |
| C-2'/ C-6' | 129.7 | 2'/ 6'- H | 7.14 |
| C-3'/ C-5' | 116.4 | 3'/ 5'- H | 6.76 |
| C-4' | 158.7 | | |

| 6^{rd} 3S-Hydroxy-L-proline | | | |
|---|---|---|---|
| C-1 | 172.8 | | |
| C-2 | 69.9 | 2-H | 4.16 |
| C-3 | 74.3 | 3-H | 4.32 |
| C-4 | 34.6 | 4-H₂ | 1.94, 2.24 |
| C-5 | 46.9 | 5-H₂ | 3.79, 3.79 |

| Fatty acid side chains | | | |
|---|---|---|---|
| C-1 | 176.6 | | |
| C-2 | 37.0 | 2-H | 2.24 (m, 2H) |
| C-3 | 27.2 | 3-H | 1.61(m, 2H) |
| C-8 / C-14 | 28.3, 28.3 | 8-H / 14-H | 2.07 (m, 2H) |
| C-11 | 26.7 | 11-H | 2.78 (t, *J* = 7.3Hz, 1H) |
| | | | 1.36 (m, 1H) |
| C-9 / C-10 / C-12 / C-13 | 131.1, 131.1, 129.2, 129.2 | 9-H / 10-H / 12-H / 13-H | 5.30-5.39 (m, 2H) |
| C-4 / C-5 / C-6 / C-7 / C-15 / C-16 / C-17 | 32.8, 30.9, 30.6, 30.5, 30.4, 30.4, 23.8 | 4-H / 5-H / 6-H / 7-H / 15-H / 16-H / 17-H | 1.28-1.39 (m, 14H) |
| C-18 | 14.6 | 18-H | 0.91 (t, *J* = 7.3 Hz, 3H) |

### Example 13. Biological activity test

The minimum inhibitory concentration (MIC) values of echinocandin E and echinocandin F were determined by broth microdilution method, and the test strain was *Candida* albicans ATCC 10231. The MIC value was defined as the lowest antibiotic concentration that inhibited the growth of more than 90% of the strains. Amphotericin B was used as a positive control and echinocandin B as a reference, and each sample was subjected to three parallel experiments.

*Candida albicans* was cultured in Mueller-Hinton (MH) broth (50 mL 20× Nitrate Salts, 1 mL Trace Elements, and 10.0 g glucose, dissolved in 800 mL ddH₂O, adjusted to pH 6.5, fixed to 1 L, added with 16.0 g agar powder, and sterilized at 121°C for 20 min) (purchased from Hangzhou Basebio Biotechnology Co., Ltd. (Basebio)) and cultured at 37°C and 180 rpm for 12-16 hours. The fungal solution concentration was controlled to 10⁶ CFU/mL by gradient dilution with MH broth.

The sample solution was prepared by dissolving the test samples (amphotericin B, echinocandin B, echinocandin E, echinocandin F) in DMSO to prepare a stock solution with a concentration of 40 µg/ml. An appropriate amount of sterile MH broth medium and the test compounds were added to a 96-well plate, and *Candida albicans* was quantitatively inoculated so that the total liquid volume in each well was 100 µL. The final concentrations of the compounds were 2, 1.5, 1, 0.8, 0.6, 0.4, 0.2, 0.1, 0.05 and 0 µg/ml, respectively, and DMSO (volume concentration of 5%) was used as a negative control.

After the 96-well plate was placed in a 37°C constant temperature incubator for 12 hours, the growth of pathogens in the wells was observed, and the concentration at which the pathogens did not grow in the wells was used as the judgment standard. The fungal concentration was measured at OD₆₀₀ using an ELISA reader to determine the MIC value of each sample. The results are shown in Table 18 below.

**Table 18 MIC values of the compounds of the present invention**

| Compound | MIC value µg/mL |
|---|---|
| Amphotericin B | 0.2 |
| Echinocandin B | 0.4 |
| Echinocandin F | 1.0 |
| Echinocandin E | 0.6 |
| Echinocandin G | 0.8 |
| Echinocandin H | 0.8 |

Experiments have shown that echinocandins E, F, G and H exhibit good activity against *Candida albicans.* It can also be further known that compounds of the present invention which are appropriately modified on this basis will also have good *anti-Candida albicans* activity.

Although the foregoing invention has been described in some detail by way of illustration and examples for the purposes of clarity of understanding, it will be readily apparent to one of ordinary skill in the art in light of the teaching of this invention that certain changes and modification may be made thereto without departing from the spirit or scope of the invention as defined in the claims.

## Claims

1. An echinocandin compound represented by the general formula (I), 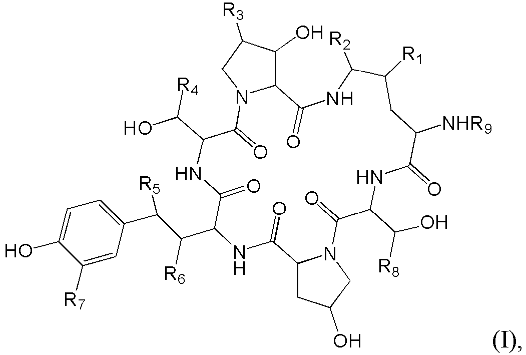
or an ester, a stereoisomer, a prodrug, a solvate and a pharmaceutically acceptable salt thereof, wherein,
R₁, R₅, and R₆ are identical with or different from each other, and are each independently selected from H or OH;
R₂ is selected from the group consisting of H, OH, -X₁-(alkylene-X₂)ₘ-alkylene-X₃, -X₁-(alkylene-X₂)ₙ-CH[CH₂(X₄-alkylene)ₚ-X₅]₂, wherein X₁, X₂, X₄ are selected from -O-, -NH-, and -S-; X₃, X₅ are selected from ORₐ, NRₐR_{b}, NRₐR_{b}Rₑ, wherein Rₐ, R_{b}, R_{c} are each independently selected from the group consisting of H, alkyl, arylalkyl, heteroarylalkyl, heterocyclylalkyl, and cycloalkylalkyl, said aryl, heteroaryl, heterocyclyl, and cycloalkyl are optionally substituted by a group selected from: hydroxyl, alkoxyl, amino, alkyl, alkenyl, alkynyl, halogen, cyano, nitro, thiol, -S-alkyl, -S(O)-alkyl, -SO₂-alkyl, NR_{c}R_{d}, COORₑ, and CONR_{c}R_{d}, wherein Rₑ, R_{d}, and Rₑ are each independently selected from H and alkyl; or any two of Rₐ, R_{b}, and R_{c} are connected to form a heterocycle; or any one of Rₐ, R_{b}, and R_{c} is connected to a carbon atom of the alkylene to which NRₐR_{b}R_{c} is connected to form a heterocycle; m, n, and p are integers of 0-5;
R₃ is selected from H, alkyl;
R₄ and R₈ are identical with or different from each other, and are each independently selected from the group consisting of H, alkyl, alkenyl, alkynyl, aryl, heteroaryl, cycloalkyl, and heterocyclyl, said alkyl, alkenyl, alkynyl, aryl, heteroaryl, cycloalkyl, and heterocyclyl are optionally substituted by halogen, cyano, nitro, thiol, -S-alkyl, -S(O)-alkyl, -SO₂-alkyl, NR_{c}R_{d}, COORₑ, and CONR_{c}'R_{d}', wherein R_{c}, R_{d}, Rₑ, R_{c}', and R_{d}' are each independently selected from H and alkyl;
R₇ is selected from H, OH, halogen, -OSO₃H, or -alkylene-N_{f}R_{g}, wherein R_{f} and R_{g} are each independently selected from H, alkyl;
R₉ is H or fatty acyl;
provided that:
at least one of R₁, R₂, R₅, and R₆ is H;
R_{c}' and R₄' are not both H;
when R₁, R₂, and R₅ are H, and R₆ is OH, R₃ is not methyl;
when R₁ and R₆ are OH, R₂ and R₅ are H, R₃ is not methyl;
when R₁, R₂, and R₆ are OH, and R₅ is H, R₃ is not methyl;
when R₁, R₅, and R₆ are OH, and R₂ is H, R₃ is not methyl.

2. The compound or an ester, a stereoisomer, a prodrug, a solvate and a pharmaceutically acceptable salt thereof according to claim 1, wherein
R₂ is selected from the group consisting of H, OH, -O-(C₁₋₃ alkylene-O)ₘ-C₁₋₃alkylene-X₃, -NH-(C₁₋₃alkylene-O)ₘ-C₁₋₃alkylene-X₃, -O-(C₁₋₃alkylene-NH)ₘ-C₁₋₃alkylene-X₃, -NH-(C₁₋₃alkylene-NH)ₘ-C₁₋₃alkylene-X₃, -O-(C₁₋₃alkylene-O)ₙ-CH[CH₂(O-C₁₋₃alkylene)ₚ-X₅]₂, -NH-(C₁₋₃alkylene-O)ₙ-CH[CH₂(O-C₁₋₃alkylene)ₚ-X₅]₂, -O-(C₁₋₃alkylene-O)ₙ-CH[CH₂(O-C₁₋₃alkylene)ₚ-X₅]₂, -O-(C₁₋₃alkylene-NH)ₙ-CH[CH₂(NH-C₁₋₃alkylene)ₚ-X₅]₂, and -NH-(C₁₋₃alkylene-NH)ₙ-CH[CH₂(NH-C₁₋₃alkylene)ₚ-X₅]₂; X₃ and X₅ are selected from ORₐ, NRₐR_{b}, NRₐR_{b}R_{c}, wherein, Rₐ, R_{b}, R_{c} are each independently selected from H, alkyl, arylalkyl, heteroarylalkyl, heterocyclylalkyl, and cycloalkylalkyl, said aryl, heteroaryl, heterocyclyl, cycloalkyl are optionally substituted by a group selected from: hydroxyl, alkoxyl, amino, alkyl, alkenyl, alkynyl, halogen, cyano, nitro, thiol, -S-alkyl, -S(O)-alkyl, -SO₂-alkyl, NR_{c}R_{d}, COORₑ, and CONR_{c}R_{d}, wherein R_{c}, R_{d}, and Rₑ are each independently selected from H and alkyl; or any two of Rₐ, R_{b}, and R_{c} are connected to form a heterocycle; or any one of Rₐ, R_{b}, and R_{c} is connected to a carbon atom of the alkylene to which NRₐR_{b}R_{c} is connected to form a heterocycle; m, n, and p are integers of 0-5.

3. The compound or an ester, a stereoisomer, a prodrug, a solvate and a pharmaceutically acceptable salt thereof according to any one of the preceding claims, wherein
R₂ is selected from H, OH, -O-(C₁₋₃alkylene-O)ₘ-C₁₋₃alkylene-X₃, -NH-(C₁₋₃alkylene-O)ₘ-C₁-₃alkylene-X₃, -O-(C₁₋₃alkylene-NH)ₘ-C₁₋₃alkylene-X₃, -NH-(C₁₋₃alkylene-NH)ₘ-C₁₋₃alkylene-X₃, -O-(C₁₋₃alkylene-O)ₙ-CH[CH₂(O-C₁₋₃alkylene)ₚ-X₅]₂, -NH-(C₁₋₃alkylene-O)ₙ-CH[CH₂(O-C₁₋₃alkylene)ₚ-X₅]₂, -O-(C₁₋₃alkylene-O)ₙ-CH[CH₂(O-C₁₋₃alkylene)ₚ-X₅]₂, -O-(C₁₋₃alkylene-NH)ₙ-CH[CH₂(NH-C₁₋₃alkylene)ₚ-X₅]₂, and -NH-(C₁₋₃alkylene-NH)ₙ-CH[CH₂(NH-C₁₋₃alkylene)ₚ-X₅]₂; X₃, X₅ are selected from ORₐ, NRaRb, NRaRbRc, wherein Ra, Rb, Rc are each independently selected from the group consisting of H, C₁₋₆ alkyl, C₇₋₂₀ arylalkyl, C₇₋₂₀ heteroarylalkyl, C₇₋₂₀ heterocyclylalkyl, and C₇₋₂₀ cycloalkylalkyl, said aryl, heteroaryl, heterocyclyl, and cycloalkyl are optionally substituted by a group selected from: hydroxyl, C₁₋₆ alkoxyl, amino, C₁₋₆ alkyl, C₁₋₆ alkenyl, C₁₋₆ alkynyl, halogen, cyano, nitro, thiol, -S-C₁₋₆alkyl, - S(O)-C₁₋₆alkyl, -SO₂-C₁₋₆alkyl, NR_{c}R_{d}, COORₑ, and CONR_{c}R_{d}, wherein R_{c}, R_{d}, and Rₑ are each independently selected from H, C₁₋₆ alkyl; or any two of Rₐ, R_{b} and R_{c} are connected to form a heterocycle; or any one of Rₐ, R_{b} and R_{c} is connected to a carbon atom of the alkylene to which NRₐR_{b}R_{c} is connected to form a heterocycle; m, n and p are integers of 0-5.

4. The compound or an ester, a stereoisomer, a prodrug, a solvate and a pharmaceutically acceptable salt thereof according to any one of the preceding claims, wherein
R₂ is selected from H, OH,

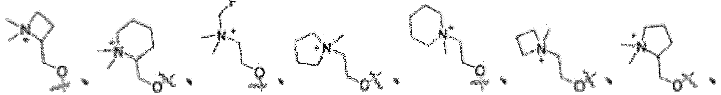

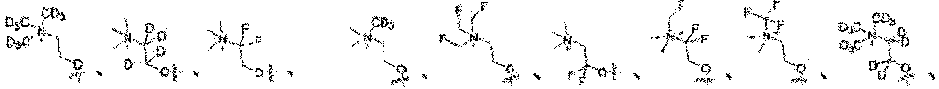

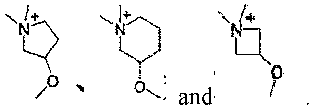

5. The compound or an ester, a stereoisomer, a prodrug, a solvate and a pharmaceutically acceptable salt thereof according to any one of the preceding claims, wherein
R₄ and R₈ are identical with or different from each other and are each independently selected from H, C₁₋₆ alkyl, C₁₋₆ alkenyl, C₁₋₆ alkynyl, C₆₋₁₄ aryl, C₆₋₁₄ heteroaryl, C₆₋₁₄ cycloalkyl, C₆₋₁₄ heterocyclyl, wherein the C₁₋₆ alkyl, C₁₋₆ alkenyl, C₁₋₆ alkynyl, C₆₋₁₄ aryl, C₆₋₁₄ heteroaryl, C₆₋₁₄ cycloalkyl, C₆₋₁₄ heterocyclyl are optionally substituted by halogen, cyano, nitro, thiol, -S-C₁₋₆alkyl, -S(O)-C₁₋₆alkyl, -SO₂ -C₁₋₆alkyl, NR_{c}R_{d}, COORₑ, and CONR_{c}'R_{d}', wherein R_{c}, R_{d}, Rₑ, R_{c}', and R_{d}' are each independently selected from H, C₁₋₆ alkyl.

6. The compound or an ester, a stereoisomer, a prodrug, a solvate and a pharmaceutically acceptable salt thereof according to any one of the preceding claims, wherein, R₄ and R₈ are identical with or different from each other, and are each independently selected from H, C₁₋₆ alkyl, said C₁₋₆ alkyl is optionally substituted by halogen, cyano, nitro, thiol, -S-C₁₋₆alkyl, -S(O)-C₁₋₆alkyl, -SO₂-C₁₋₆alkyl, NR_{c}R_{d}, COORₑ, CONR_{c}'R_{d}', wherein R_{c}, R_{d}, Rₑ, R_{c}', and R_{d}' are each independently selected from H, C ₁₋₆ alkyl.

7. The compound or an ester, a stereoisomer, a prodrug, a solvate and a pharmaceutically acceptable salt thereof according to any one of the preceding claims, wherein
R ₉ is C₆₋₅₀ fatty acyl, which optionally contains an unsaturated alkenyl, cycloalkyl, aryl, heteroaryl, or heterocyclic.

8. The compound or an ester, a stereoisomer, a prodrug, a solvate and a pharmaceutically acceptable salt thereof according to any one of the preceding claims, wherein
R₉ is

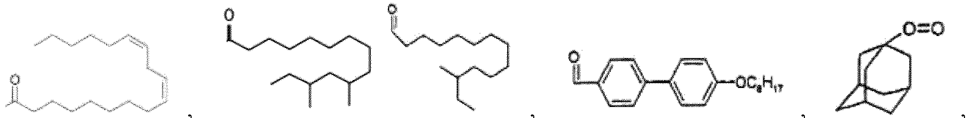

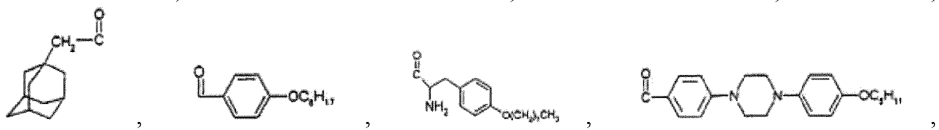

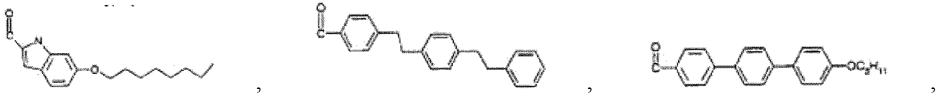

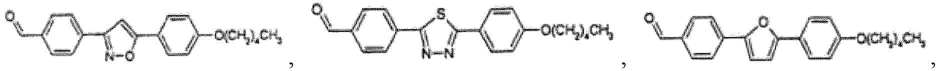

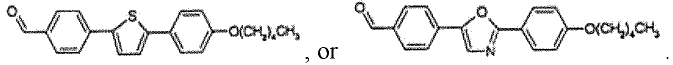

9. The compound or an ester, a stereoisomer, a prodrug, a solvate and a pharmaceutically acceptable salt thereof according to any one of the preceding claims, wherein
provided that:
at least two of R₁, R₂, R₅, and R₆ are H;
R_{c}' and R₄' are not both H;
when R₁, R₂, and R₅ are H, and R₆ is OH, R₃ is not methyl;
when R₁ and R₆ are OH, R₂ and R₅ are H, R₃ is not methyl;
when R₁, R₂, and R₆ are OH, and R₅ is H, R₃ is not methyl;
when R₁, R₅, and R₆ are OH, and R₂ is H, R₃ is not methyl.

10. The compound or an ester, a stereoisomer, a prodrug, a solvate and a pharmaceutically acceptable salt thereof according to any one of the preceding claims, wherein
provided that:
at least three of R₁, R₂, R₅, and R₆ are H;
R_{c}' and R₄' are not both H;
when R₁, R₂, and R₅ are H, and R₆ is OH, R₃ is not methyl;
when R₁ and R₆ are OH, R₂ and R₅ are H, R₃ is not methyl;
when R₁, R₂, and R₆ are OH, and R₅ is H, R₃ is not methyl;
when R₁, R₅, and R₆ are OH, and R₂ is H, R₃ is not methyl.

11. The compound or an ester, a stereoisomer, a prodrug, a solvate and a pharmaceutically acceptable salt thereof according to any one of the preceding claims, wherein
R₁, R₅, and R₆ are H;
R₂ is selected from H, -X₁-(alkylene-X₂)ₘ-alkylene-X₃, or -X₁-(alkylene-X₂)n -CH[CH₂(X₄-alkylene)ₚ-X₅ ]₂, wherein X₁, X₂, X₄ are selected from -O-, -NH-, and -S-; X3, X5 are selected from ORa, NRaRb, NRaRbRc, wherein Ra, Rb, Rc are each independently selected from H, alkyl, arylalkyl, heteroarylalkyl, heterocyclylalkyl, and cycloalkylalkyl, said aryl, heteroaryl, heterocyclyl, cycloalkyl are optionally substituted by a group selected from the: hydroxyl, alkoxyl, amino, alkyl, alkenyl, alkynyl, halogen, cyano, nitro, thiol, -S-alkyl, -S(O)-alkyl, -SO₂-alkyl, NR_{c}R_{d}, COORₑ, and CONR_{c}R_{d}, wherein Rc, Rd, and Re are each independently selected from H and alkyl; or any two of Ra, Rb, and Rc are connected to form a heterocycle; or any one of Ra, Rb, and Rc is connected to a carbon atom of the alkylene to which NRaRbRc is connected to form a heterocycle; m, n, and p are integers of 0-5.

12. The compound or an ester, a stereoisomer, a prodrug, a solvate and a pharmaceutically acceptable salt thereof according to any one of the preceding claims, wherein
R₁, R₂, R₅, and R₆ are H.

13. The compound or an ester, a stereoisomer, a prodrug, a solvate and a pharmaceutically acceptable salt thereof according to any one of the preceding claims, wherein
R₁, R₂, R₅, and R₆ are H;
R₃, R₄, and R₈ are each independently H or methyl;
R₇ is H, OH, or -OSO₃H;
R₉ is

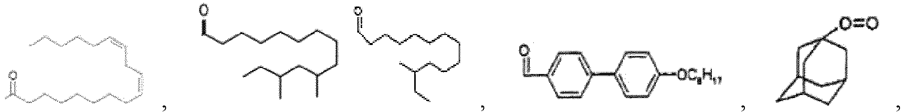

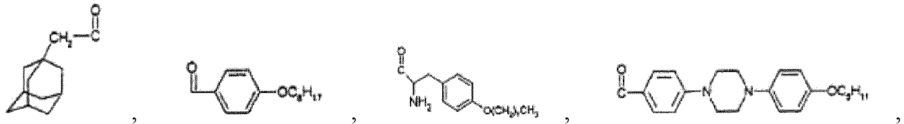

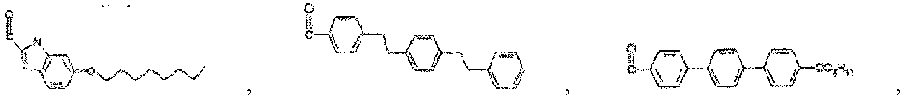

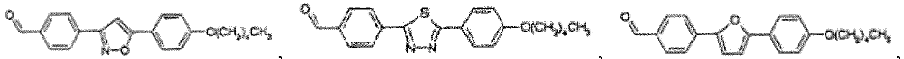

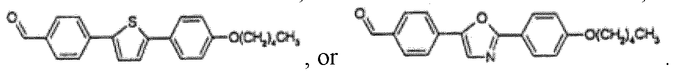

14. The compound according to any one of the preceding claims, wherein the compound is 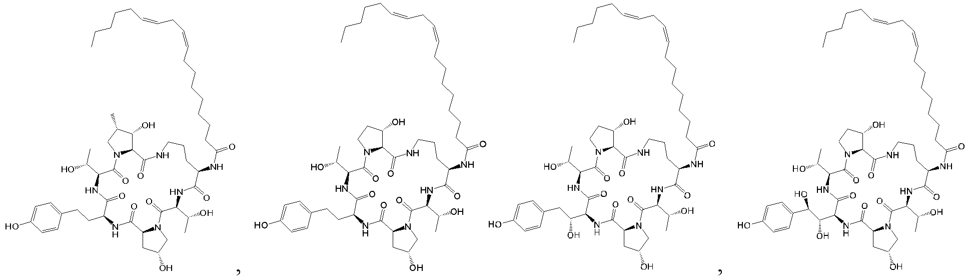 or an ester, a stereoisomer, a prodrug, a solvate and a pharmaceutically acceptable salt thereof.

15. A pharmaceutical composition comprising the compound, or an ester, stereoisomer, prodrug, solvate and pharmaceutically acceptable salt thereof according to any one of the preceding claims.

16. The pharmaceutical composition according to any one of the preceding claims, further comprises an additional antifungal agent.

17. An echinocandin biosynthetic gene cluster comprising two or more of genes *ecdA, ecd*I*, ecdK* and *htyE.*

18. The echinocandin biosynthetic gene cluster according to any one of the preceding claims, which comprises genes *ecdA, ecd*I and *htyE.*

19. The echinocandin biosynthetic gene cluster according to any one of the preceding claims, which comprises genes *ecdA, ecd*I*, ecdK* and *htyE.*

20. The echinocandin biosynthetic gene cluster according to any one of the preceding claims, which consists of genes *ecdA, ecd*I and *htyE.*

21. The echinocandin biosynthetic gene cluster according to any one of the preceding claims, which consists of genes *ecdA, ecd*I*, ecdK* and *htyE.*

22. The echinocandin biosynthetic gene cluster according to any one of the preceding claims, which further comprises one or more of genes *ecdG, ecdH* or *htyF.*

23. The echinocandin biosynthetic gene cluster according to any one of the preceding claims, which consists of genes *ecdA, ecd*I*, ecdG* and *htyE.*

24. The echinocandin biosynthetic gene cluster according to any one of the preceding claims, which consists of genes *ecdA, ecd*I*, ecdG, htyE,* and *htyF.*

25. A genetically engineered transformant strain comprising the echinocandin biosynthetic gene cluster according to any one of the preceding claims.

26. The genetically engineered transformant strain according to any one of the preceding claims, which is the transformant strain LO8030-5.1 with a deposit accession number of CCTCC M 20211360, or the transformant strain LO8030-4.2.1 with a deposit accession number of CCTCC M 2022168, or the transformant strain LO8030-4.2.1.5 with a deposit accession number of CCTCC M 2023155, or the transformant strain LO8030-4.2.1.9 with a deposit accession number of CCTCC M 2023156.

27. A method for preparing the compound according to any one of the preceding claims, which comprises: culturing the genetically engineered transformnt strains according to any one of the preceding claims for heterologous expression, and extracting and separating the fermentation broth to obtain the compound.

28. Use of the compound according to any one of claims 1 to 14 in the preparation of a medicament for treating or preventing fungal infection-associated diseases.

29. The use according to any one of the preceding claims, wherein the fungus is selected from any one or more of *Aspergillus* (such as *Aspergillus flavus, Aspergillus fumigatus, Aspergillus glaucus, Aspergillus nidulans, Aspergillus niger* and *Aspergillus terreus), Blastomyces dermatitidis, Candida* (such as *Candida albicans, Candida glabrata, Candida tropicalis, Candida parapsilosis, Candida krusei* and *Candida guilliermondii), Coccidioides immitis, Cryptococcus* (such as *Cryptococcus neoformans, Cryptococcus albicans* and *Cryptococcus laurentii*), *Histoplasma capsulatum var.capsulatum, Histoplasma capsulatum* duboisii, *Paracoccidioides brasiliensis, Sporothrix schenckii, Absidia corymbifera, Rhizomucor pusillus* and *Rhizopus arrhizus.*

30. The use according to any one of the preceding claims, the fungal infection-associated disease is selected from one or more of scalp tinea, tinea corporis, tinea pedis, onychomycosis, perionychomycosis, tinea versicolor, thrush, vaginal candidiasis, respiratory tract candidiasis, biliary tract candidiasis, esophageal candidiasis, urethral candidiasis, systemic candidiasis, mucosal and skin candidiasis, aspergillosis, mucormycosis, paracoccidioidomycosis, North American blastomycosis, histoplasmosis, coccidioidomycosis, sporotrichosis, fungal sinusitis and chronic sinusitis.

## Amended claims

### Amended claims under Art. 19.1 PCT

1. An echinocandin compound represented by the general formula (I), 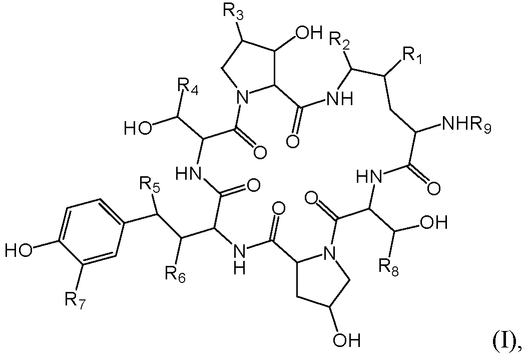
or an ester, a stereoisomer, a prodrug, a solvate and a pharmaceutically acceptable salt thereof, wherein,
R₁ is selected from H;
R₂ is selected from H;
R₃ is selected from H and alkyl;
R₅, and R₆ are identical with or different from each other, and are each independently selected from H or OH;
R₄ and R₈ are identical with or different from each other, and are each independently selected from the group consisting of H, alkyl, alkenyl, alkynyl, aryl, heteroaryl, cycloalkyl, and heterocyclyl, said alkyl, alkenyl, alkynyl, aryl, heteroaryl, cycloalkyl, and heterocyclyl are optionally substituted by halogen, cyano, nitro, thiol, -S-alkyl, -S(O)-alkyl, -SO₂-alkyl, NR_{c}R_{d}, COORₑ, and CONR_{c}'R_{d}', wherein R_{c}, R_{d}, Rₑ, R_{c}', and R_{d}' are each independently selected from H and alkyl;
R₇ is selected from H, OH, halogen, -OSO₃H, or -alkylene-N_{f}R_{g}, wherein R_{f} and R_{g} are each independently selected from H, alkyl;
R₉ is H or fatty acyl;
provided that:
R_{c}' and R_{d}' are not both H;
when R₁, R₂, and R₅ are H, and R₆ is OH, R₃ is not methyl.

2. The compound or an ester, a stereoisomer, a prodrug, a solvate and a pharmaceutically acceptable salt thereof according to claim 1, wherein
R₄ and R₈ are identical with or different from each other and are each independently selected from H, C₁₋₆ alkyl, C₁₋₆ alkenyl, C₁₋₆ alkynyl, C₆₋₁₄ aryl, C₆₋₁₄ heteroaryl, C₆₋₁₄ cycloalkyl, C₆₋₁₄ heterocyclyl, wherein the C₁₋₆ alkyl, C₁₋₆ alkenyl, C₁₋₆ alkynyl, C₆₋₁₄ aryl, C₆₋₁₄ heteroaryl, C₆₋₁₄ cycloalkyl, C₆₋₁₄ heterocyclyl are optionally substituted by halogen, cyano, nitro, thiol, -S-C₁₋₆alkyl, -S(O)-C₁₋₆alkyl, -SO₂ -C₁₋₆alkyl, NR_{c}R_{d}, COORₑ, and CONR_{c}'R_{d}', wherein R_{c}, R_{d}, Rₑ, R_{c}', and R_{d}' are each independently selected from H, C₁₋₆ alkyl.

3. The compound or an ester, a stereoisomer, a prodrug, a solvate and a pharmaceutically acceptable salt thereof according to claim 1, wherein, R₄ and R₈ are identical with or different from each other, and are each independently selected from H, C₁₋₆ alkyl, said C₁₋₆ alkyl is optionally substituted by halogen, cyano, nitro, thiol, -S-C₁₋₆alkyl, -S(O)-C₁₋₆alkyl, -SO₂-C₁₋₆alkyl, NR_{c}R_{d}, COORₑ, CONR_{c}'R_{d}', wherein R_{c}, R_{d}, Rₑ, R_{c}', and R_{d}' are each independently selected from H, C ₁₋₆ alkyl.

4. The compound or an ester, a stereoisomer, a prodrug, a solvate and a pharmaceutically acceptable salt thereof according to claim 1, wherein
R ₉ is C₆₋₅₀ fatty acyl, which optionally contains an unsaturated alkenyl, cycloalkyl, aryl, heteroaryl, or heterocyclic.

5. The compound or an ester, a stereoisomer, a prodrug, a solvate and a pharmaceutically acceptable salt thereof according to any one of claims 1 to 4, wherein
R₉ is

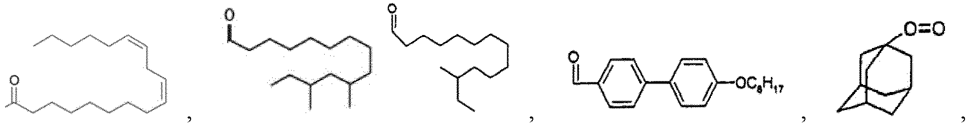

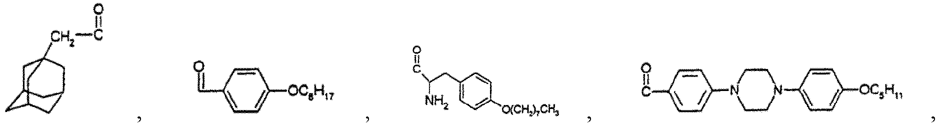

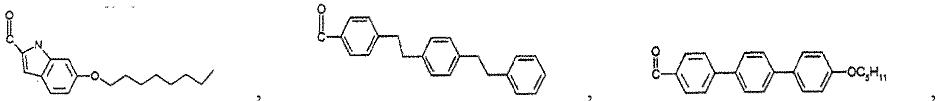

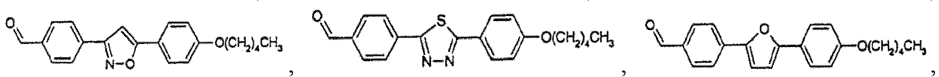

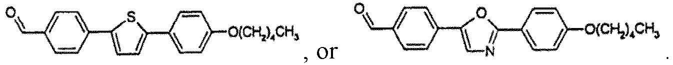

6. The compound or an ester, a stereoisomer, a prodrug, a solvate and a pharmaceutically acceptable salt thereof according to claim 1, wherein
provided that:
at least one of R₅, and R₆ is H;
R_{c}' and R_{d}' are not both H;
when R₁, R₂, and R₅ are H, and R₆ is OH, R₃ is not methyl.

7. The compound or an ester, a stereoisomer, a prodrug, a solvate and a pharmaceutically acceptable salt thereof according to claim 1, wherein
R₁, R₂, R₅, and R₆ are H.

8. The compound or an ester, a stereoisomer, a prodrug, a solvate and a pharmaceutically acceptable salt thereof according to claim 1, wherein
R₁, R₂, R₅, and R₆ are H;
R₃, R₄, and R₈ are each independently H or methyl;
R₇ is H, OH, or -OSO₃H;
R₉ is

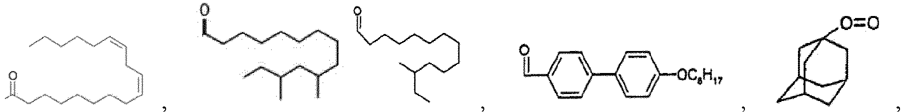

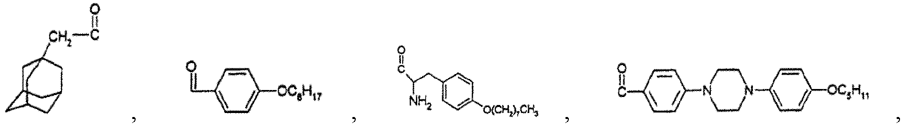

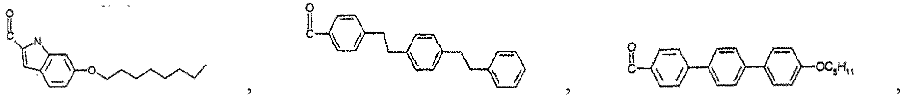

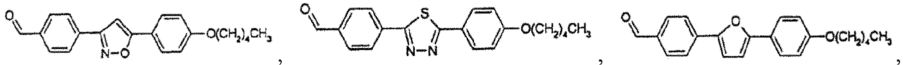

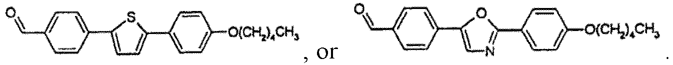

9. The compound according to claim 1, which is 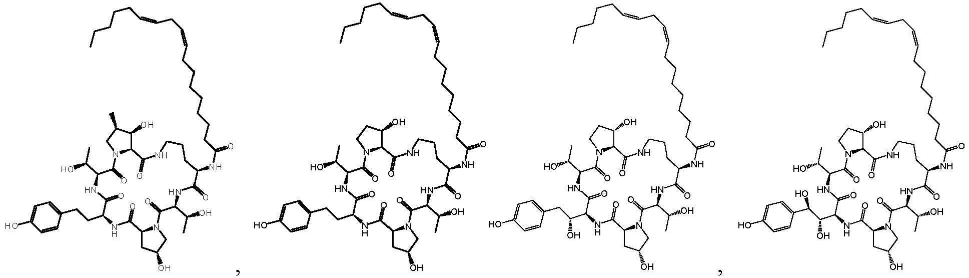 or an ester, a stereoisomer, a prodrug, a solvate and a pharmaceutically acceptable salt thereof.

10. A pharmaceutical composition comprising the compound, or an ester, stereoisomer, prodrug, solvate and pharmaceutically acceptable salt thereof according to any one of claims 1 to 9.

11. The pharmaceutical composition according to claim 10, which further comprises an additional antifungal agent.

12. An echinocandin biosynthetic gene cluster , which consists of genes *ecdA, ecd*I and *htyE,* and optional *ecdG* and/or *htyF;* or which consists of genes *ecdA, ecd*I*, ecdK* and *htyE,* and optional *ecdG* and/or *htyF.*

13. The echinocandin biosynthetic gene cluster according to claim 12, which consists of genes *ecdA, ecd*I and *htyE;* or consists of genes *ecdA, ecd*I*, ecdK* and *htyE;* or consists of genes *ecdA, ecd*I*, ecdG* and *htyE;* or consists of genes *ecdA, ecd*I*, ecdG, htyE* and *htyF.*

14. A genetically engineered transformant strain comprising the echinocandin biosynthetic gene cluster according to claim 13.

15. The genetically engineered transformant strain according to claim 14, which is the transformant strain LO8030-5.1 with a deposit accession number of CCTCC M 20211360, or the transformant strain LO8030-4.2.1 with a deposit accession number of CCTCC M 2022168, or the transformant strain LO8030-4.2.1.5 with a deposit accession number of CCTCC M 2023155, or the transformant strain LO8030-4.2.1.9 with a deposit accession number of CCTCC M 2023156.

16. Use of the compound according to claims 1 to 9 in the preparation of a medicament for treating or preventing fungal infection-associated diseases.

17. The use according to claim 16, wherein the fungus is selected from any one or more of *Aspergillus* (such as *Aspergillus flavus, Aspergillus fumigatus, Aspergillus glaucus, Aspergillus nidulans, Aspergillus niger* and *Aspergillus terreus), Blastomyces dermatitidis, Candida* (such as *Candida albicans, Candida glabrata, Candida tropicalis, Candida parapsilosis, Candida krusei* and *Candida guilliermondii), Coccidioides immitis, Cryptococcus* (such as *Cryptococcus neoformans, Cryptococcus albicans* and *Cryptococcus laurentii*), *Histoplasma capsulatum var.capsulatum, Histoplasma capsulatum* duboisii, *Paracoccidioides brasiliensis, Sporothrix schenckii, Absidia corymbifera, Rhizomucor pusillus* and *Rhizopus arrhizus.*

18. The use according to claim 16, the fungal infection-associated disease is selected from one or more of scalp tinea, tinea corporis, tinea pedis, onychomycosis, perionychomycosis, tinea versicolor, thrush, vaginal candidiasis, respiratory tract candidiasis, biliary tract candidiasis, esophageal candidiasis, urethral candidiasis, systemic candidiasis, mucosal and skin candidiasis, aspergillosis, mucormycosis, paracoccidioidomycosis, North American blastomycosis, histoplasmosis, coccidioidomycosis, sporotrichosis, fungal sinusitis and chronic sinusitis.
